# EUROPEAN PATENT SPECIFICATION

(11) **EP 1 664 053 B1**
(45) Date of publication and mention of the grant of the patent: **24.01.2007**
(21) Application number: 04783482.5
(22) Date of filing: 23.08.2004
(51) Int. Cl.: C07D 487/14, A61K 31/519

(54) **SUBSTITUTED HETEROCYCLIC COMPOUNDS AND METHODS OF USE**
SUBSTITUIERTE HETEROCYCLISCHE VERBINDUNGEN UND ANWENDUNGSVERFAHREN
COMPOSES HETEROCYCLIQUES SUBSTITUES ET LEURS METHODES D'UTILISATION

(30) Priority: 27.08.2003 US 498404 P; 20.08.2004 US 923523
(43) Date of publication of application: 07.06.2006
(73) Proprietor: AMGEN INC., Thousand Oaks, CA 91320-1799 (US)
(72) Inventor: NUNES, Joseph J., Andover, Massachusetts 01810 (US); ZHU, Xiaotian, Newton, Massachusetts 02465 (US); ERMANN, Monika, Abingdon, Oxon Oxfordshire OX14 4XB (GB); GHIRON, Chiara, I-53014 Isola d'Arbia - Asciano (Siena) (IT); JOHNSTON, David N., Abingdon Oxfordshire OX14 4SD (GB); SALUSTE, Carl-Gustaf Pierre, E-28012 Madrid (ES)
(74) Representative: Grünecker, Kinkeldey, Stockmair & Schwanhäusser Anwaltssozietät
(86) International application number: PCT/US2004/029246
(87) International publication number: WO 2005/021551

(56) References cited:
- WO-A-03/082871
- PING CHEN, EDWIN J. IWANOWICZ, DEREK NORRIS, HENRY H. GU, JAMES LIN ET AL.: "Synthesis and SAR of Novel Imidazoquinoxaline-Based Lck Inhibitors: Improvement of Cell Potency" BIOORGANIC MEDICINAL CHEMISTRY LETTERS, vol. 12, 2002, pages 3153-3156, XP002307152
- ANDREW F. BURCHAT, DAVID J. CALDERWOOD, MICHAEL M. FRIEDMAN, GAVIN C. HIRST ET AL.: "Pyrazolo[3,4-d]pyrimidines Containing an Extended 3-Substituent as Potent Inhibitors of Lck - a Selectivity Insight" BIOORGANIC MEDICINAL CHEMISTRY LETTERS, vol. 12, 2002, pages 1687-1690, XP002307153

## Description

This application claims the benefit of U.S. Provisional Application No. 60/498,404 filed August 27, 2003, which disclosure is hereby incorporated by reference in its entirety.

### BACKGROUND OF THE INVENTION

T cells play a pivotal role in the regulation of immune responses and are important for establishing immunity to pathogens. In addition, T cells are often activated during inflammatory autoimmune diseases, such as rheumatoid arthritis, inflammatory bowel disease, type I diabetes, multiple sclerosis, Sjogren's disease, myasthenia gravis, psoriasis, and lupus. T cell activation is also an important component of transplant rejection, allergic reactions, and asthma.

T cells are activated by specific antigens through the T cell receptor (TCR) which is expressed on the cell surface. This activation triggers a series of intracellular signaling cascades mediated by enzymes expressed within the cell (Kane, LP et al. Current Opinion in Immunol. 200, 12, 242). These cascades lead to gene regulation events that result in the production of cytokines, like interleukin-2 (IL-2). IL-2 is a critical cytokine in T cell activation, leading to proliferation and amplification of specific immune responses.

One class of enzymes shown to be important in signal transduction is the kinase class of enzymes, which include tyrosine kinases. Members of the Src-family of tyrosine kinases include, for example: Lck, Fyn(B), Fyn(T), Lyn, Src, Yes, Hck, Fgr and Blk (for review see: Bolen, JB, and Brugge, JS Annu. Rev. Immunol 1997, 15, 371). Gene disruption studies suggest that inhibition of some members of the Src family of kinases would potentially lead to therapeutic benefit. Src(-/-) mice have abnormalities in bone remodeling or osteopetrosis (Soriano, P. Cell 1991, 64, 693), suggesting that inhibition of this kinase might be useful in diseases of bone resorption, such as osteoporosis. Lck(-/-) mice have defects in T cell maturation and activation (Anderson, SJ et al. Adv. Immunol. 1994, 56, 151), suggesting that inhibition of this kinase might be useful in diseases of T cell mediated inflammation. In addition, human patients have been identified with mutations effecting Lck kinase activity (Goldman, FD et al. J. Clin. Invest.1998, 102,421). These patients suffer from a severe combined immunodeficiency disorder (SCID).

Without wishing to imply that the compounds disclosed in the present invention possess pharmacological activity only by virtue of an effect on a single biological process, it is believed that the compounds modulate T cell activation by way of inhibition of one or more of the multiple protein tyrosine kinases involved in early signal transduction steps leading to T cell activation, for example by way of inhibition of Lck kinase.

Src-family kinases are also important for signaling downstream of other immune cell receptors. Fyn, like Lck, is involved in TCR signaling in T cells (Appleby, MW et al. Cell 1992, 70, 751). Hck and Fgr are involved in Fcγ receptor signaling leading to neutrophil activation (Vicentini, L. et al. J. Immunol. 2002, 168, 6446). Lyn and Src also participate in Fcγ receptor signaling leading to release of histamine and other allergic mediators (Turner, H. and Kinet, J-P Nature 1999, 402, B24). These findings suggest that Src family kinase inhibitors may be useful in treating allergic diseases and asthma.

Src kinases have also been found to be activated in tumors including sarcoma, melanoma, breast, and colon cancers suggesting that Src kinase inhibitors may be useful anti-cancer agents (Abram, CL and Courtneidge, SA Exp. Cell Res. 2000, 254, 1).

Src kinase inhibitors have also been reported to be effective in an animal model of cerebral ischemia (R. Paul et al. Nature Medicine 2001, 7, 222), suggesting that Src kinase inhibitors may be effective at limiting brain damage following stroke.

Several groups have published on inhibitors of Src family kinase and the activities of these inhibitors in various in vitro and in vivo biological systems. These include the 2-phenylamino-imidazo [4,5-h]isoquinolin-9-ones (Snow, RJ et al. J. Med. Chem. 2002, 45, 3394), the pyrazolo [3,4-d]pyrimidines (Burchat, AF et al. Bioorganic and Med. Chem. Letters 2002, 12, 1687. Hanke, JH et al. J. Biol. Chem. 1996, 271, 695), the pyrrolo [2,3-d]pyrimidines (Altmann, E et al. Bioorganic and Med. Chem. Letters 2001, 11, 853), the anilinoquinazolines (Wang, YD et al. Bioorganic and Med. Chem. Letters 2000, 10, 2477), and the imidazoquinoxalines (Chen, P. et al. Bioorganic and Med. Chem. Letters 2002, 12, 3153).

### BRIEF DESCRIPTION OF THE INVENTION

In one embodiment, the present invention provides compounds, which are capable of modulating protein tyrosine kinases, especially Src-family kinases such as Lck, Fyn(B), Fyn(T), Lyn, Src, Yes, Hck, Fgr and Blk, and are thus useful in the treatment, including prevention and therapy, of protein tyrosine kinase-associated disorders such as immunologic disorders. "Protein tyrosine kinase-associated disorders" are those disorders which result from aberrant tyrosine kinase activity, and/or which are alleviated by the inhibition of one or more of these enzymes. For example, Lck inhibitors are of value in the treatment of a number of such disorders (for example, the treatment of autoimmune diseases), as Lck inhibition blocks T cell activation. The treatment of T cell mediated diseases, including inhibition of T cell activation and proliferation, is a preferred embodiment of the present invention. In one embodiment of the invention, there are provided compounds which selectively block T cell activation and proliferation. In another embodiment, there are provided compounds which may block the activation of endothelial cell protein tyrosine kinase by oxidative stress, thereby limiting surface expression of adhesion molecules that induce neutrophil binding, and compounds which can inhibit protein tyrosine kinase necessary for neutrophil activation would be useful, for example, in the treatment of ischemia and reperfusion injury.

In another embodiment, the present invention provides methods for the treatment of protein tyrosine kinase-associated disorders, the method comprising the step of administering to a subject at least one compound of the present invention in an amount effective to treat the disorder. Other therapeutic agents such as those described herein may be administered in combination with the inventive compounds in these treatment methods. For example, In these methods, such other therapeutic agent(s) may be administered prior to, simultaneously with or following the administration of the compound(s) of the present invention.

Use of the compound(s) of the present invention in treating protein tyrosine kinase-associated disorders is exemplified by, but is not limited to, treating a range of disorders such as: arthritis (such as rheumatoid arthritis, psoriatic arthritis or osteoarthritis); transplant (such as organ transplant, acute transplant or heterograft or homograft (such as is employed in burn treatment)) rejection; protection from ischemic or reperfusion injury such as ischemic or reperfusion injury incurred during organ transplantation, myocardial infarction, stroke or other causes; transplantation tolerance induction; multiple sclerosis; inflammatory bowel disease, including ulcerative colitis and Crohn's disease; lupus (systemic lupus erythematosis); graft vs. host diseases; T -cell mediated hypersensitivity diseases, including contact hypersensitivity, delayed-type hypersensitivity, and gluten-sensitive enteropathy (Celiac disease); Type 1 diabetes; psoriasis; contact dermatitis (including that due to poison ivy); Hashimoto's thyroiditis; Sjogren's syndrome; Autoimmune Hyperthyroidism, such as Graves' Disease; Addison's disease (autoimmune disease of the adrenal glands); Autoimmune polyglandular disease (also known as autoimmune polyglandular syndrome); autoimmune alopecia; pernicious anemia; vitiligo; autoimmune hypopituatarism; Guillain-Barre syndrome; other autoimmune diseases; cancers where Lck or other Src-family kinases such as Src are activated or overexpressed, such as colon carcinoma and thymoma, or cancers where Src-family kinase activity facilitates tumor growth or survival; glomerulonephritis, serum sickness; uticaria; allergic diseases such as respiratory allergies (asthma, hayfever, allergic rhinitis) or skin allergies; scleracielma; mycosis fungoides; acute inflammatory responses (such as acute respiratory distress syndrome and ishchemia/reperfusion injury); dermatomyositis; alopecia areata; chronic actinic dermatitis; eczema; Behcet's disease; Pustulosis palmoplanteris; Pyoderma gangrenum; Sezary's syndrome; atopic dermatitis; systemic schlerosis; and morphea. The present invention also provides for a method for treating the aforementioned disorders such as atopic dermatitis by administration of a therapeutically effective amount of a compound of the present invention, which is an inhibitor of protein tyrosine kinase, to a patient in need of such treatment.

Src-family kinases other than Lck, such as Hck and Fgr , are important in the Fcγ receptor induced respiratory burst of neutrophils as well as the Fcγ receptor responses of monocytes and macrophages. The compounds of the present invention may inhibit the Fcγ induced respiratory burst response in neutrophils, and may also inhibit the Fcγ dependent production of TNFα. The ability to inhibit Fcγ receptor dependent neutrophil, monocyte and macrophage responses would result in additional anti-inflammatory activity for the present compounds in addition to their effects on T cells. This activity would be especially of value, for example, in the treatment of inflammatory diseases, such as arthritis or inflammatory bowel disease. The present compounds may also be of value for the treatment of autoimmune glomerulonephritis and other instances of glomerulonephritis induced by deposition of immune complexes in the kidney that trigger Fcγ receptor responses and which can lead to kidney damage.

In addition, certain Src family kinases, such as Lyn and Fyn(B), may be important in the Fcε receptor induced degranulation of mast cells and basophils that plays an important role in asthma, allergic rhinitis, and other allergic disease. Fcε receptors are stimulated by IgE-antigen complexes. The compounds of the present invention may inhibit the Fcε induced degranulation responses. The ability to inhibit Fcε receptor dependent mast cell and basophil responses may result in additional anti-inflammatory activity for the present compounds beyond their effect on T cells.

The combined activity of the present compounds towards monocytes, macrophages, T cells, etc. may prove to be a valuable tool in the treatment of any of the aforementioned disorders.

In a particular embodiment, the compounds of the present invention are useful for the treatment of the aforementioned exemplary disorders irrespective of their etiology, for example, for the treatment of rheumatoid arthritis, transplant rejection, multiple sclerosis, inflammatory bowel disease, lupus, graft v. host disease, T cell mediated hypersensitivity disease, psoriasis, Hashimoto's thyroiditis, Guillain-Barre syndrome, cancer, contact dermatitis, allergic disease such as allergic rhinitis, asthma, ischemic or reperfusion injury, or atopic dermatitis whether or not associated with PTK.

The compounds of the present invention are represented by the general structure Formula I: 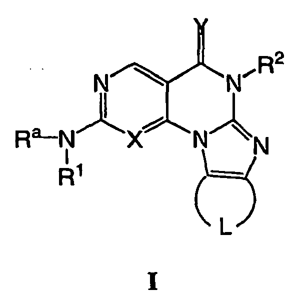 wherein X, Y, R^{a}, R¹, R² and L are defined herein below.

The foregoing merely summarizes certain aspects of the invention and is not intended, nor should it be construed, as limiting the invention in any way.

### DETAILED DESCRIPTION OF THE INVENTION

In accordance with the present invention, one embodiment of the invention provides a compound of Formula I: 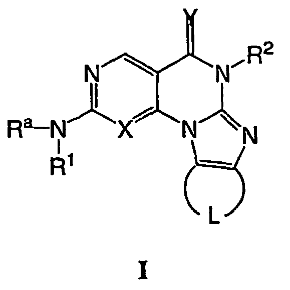 or a pharmaceutically-acceptable salt or derivative thereof, wherein
X is N or CH;
Y is NH, N(CN), O or S;
L is a 4-atom chain made up of C and N atoms, wherein the chain is substituted by 0 or 1 R³ groups and the chain is additionally substituted by 0, 1, 2 or 3 substituents independently selected from R^{c};
R¹ is selected from -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ and -R¹¹-R¹⁴-R¹³-R¹⁴, any of which is substituted by 0, 1, 2, 3, 4 or 5 substituents independently selected from R^{c};
R² is selected from -R²¹, -R²¹-R²², -R²¹-R²⁴, -R²²-R²⁴, -R²¹-R²²-R²⁴, -R²¹-R²³-R²⁴,-R²²-R²³-R²⁴, -R²¹-R²³-R²²-R²⁴ and -R²¹-R²²-R²³-R²⁴, any of which is substituted by 0, 1, 2, 3, 4 or 5 substituents independently selected from R^{c};
R³ is selected from -R³², -R³⁴, -R³²-R³⁴, -R³²-R³⁴, -R³³-R³⁴, -R³²-R³³-R³⁴, -R³³-R³²-R³⁴ and -R³²-R³³-R³⁴, any of which is substituted by 0, 1, 2, 3, 4 or 5 substituents independently selected from R^{c};
R¹¹ is independently at each instance a saturated or unsaturated 5-, 6- or 7-membered monocyclic or 6-, 7-, 8-, 9-, 10- or 11-membered bicyclic ring containing 0,1,2,3 or 4 atoms selected from N, O and S, so long as the combination of O and S atoms is not greater than 2, wherein the carbon atoms of the ring are substituted by 0, 1 or 2 oxo groups;
R¹² is independently at each instance C₁₋₈alkyl;
R¹³ is independently at each instance -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -OC₂₋₆alkylN(R^{a})-, -OC₂₋₆alkyl-, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)2N(R^{a})-, -NR^{a}C₂₋₆alkylN(R^{a})- or -NR^{a}C₂₋₆alkylO-;
R¹⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R²¹ is independently at each instance a saturated or unsaturated 5-, 6- or 7-membered monocyclic or 6-, 7-, 8-, 9-, 10- or 1 1-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R²² is independently at each instance C₁₋₈alkyl;
R²³ is independently at each instance -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -OC₂₋₆alkylN(R^{a})-, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a})₂-, -N(R^{a})S(=O)₂-, -N(R^{a})S(=O)₂N(R^{a}) -, -NR^{a}C₂₋₆alkylN(R^{a})- or -NR^{a}C₂₋₆alkylO-;
R²⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R³² is independently at each instance C₁₋₈alkyl;
R³³ is independently at each instance -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)_{2-,} -OC₂₋₆alkylN(R^{a}) -, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a}) -, -NR^{a}C₂₋₆alkylN(R^{a})- or -NR^{a}C₂₋₆alkylO-;
R³⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R^{a} is independently at each instance H or R^{b};
R^{b} is independently at each instance C₁₋₈alkyl, CN, phenyl or benzyl; and
R^{c} is independently at each instance C₁₋₈alkyl, C₁₋₄haloalkyl, halo, cyano, nitro, -C₂₋₆alkylOR^{a}, -C₂₋₆alkyl C(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a},
-OC(=O)N(R^{a})S(=O)₂R^{b}, -OC₂₋₆alkylNR^{a}R^{a}, -OC₂₋₆alkylC(=O)NR^{a}R^{a}, -OC₂₋₆alkylOR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkylNR^{a}R^{a} or -NR^{a}C₂₋₆alkylOR^{a}.

In another embodiment, there are provided compounds of Formula II 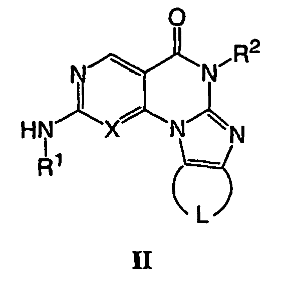 or a pharmaceutically-acceptable salt thereof, wherein
X is N or CH;
L is a 3- or 4-atom chain made up of C and N atoms, wherein the chain is substituted by 0 or 1 R³ groups and the chain is additionally substituted by 0,1,2 or 3 substituents independently selected from R^{c};
R¹ is selected from -R¹¹, -R¹¹-R¹², -R¹¹-R¹⁴, -R¹²-R¹⁴, -R¹¹-R¹²-R¹⁴, -R¹¹-R¹³-R¹⁴, -R¹²-R¹³-R¹⁴, -R¹¹-R¹³-R¹²-R¹⁴ and -R¹¹-R¹²-R¹³-R¹⁴, any of which is substituted by 0, 1, 2, 3 or 4 substituents independently selected from R^{c};
R² is selected from -R²¹, -R²¹-R²², -R²¹-R²⁴, -R²²-^{R24}, -R²²-R²²-R²⁴, -R²¹-R²³-R²⁴, -R²²-R²³-R²⁴, -R²¹-R²³-R²²-R²⁴ and -R²¹-R²²-R²³-R²⁴, any of which is substituted by 0, 1, 2, 3 or 4 substituents independently selected from R^{c};
R³ is selected from -R³², -R³⁴, -R³²-R³⁴, -R³²-R³⁴, -R³³-R³⁴, -R³²-R³³-R³⁴, -R³³-R³²-R³⁴ and -R³²-R³³-R³⁴, any of which is substituted by 0, 1, 2, 3 or 4 substituents independently selected from R^{c};
R¹¹ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R¹² is independently at each instance C₁₋₈alkyl;
R¹³ is independently at each instance -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(-O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-;
R¹⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of 0 and S atoms is not greater than 2, wherein the carbon atoms of the ring are substituted by 0, 1 or 2 oxo groups;
R²¹ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R²² is independently at each instance C₁₋₈alkyl;
R²³ is independently at each instance -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-;
R²⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R³² is independently at each instance C₁₋₈alkyl;
R³³ is independently at each instance -C(=O)-, -C(=O)O-, -C(=O)NR^{a}-, -C(=NR^{a})NR^{a}-, -O-, -OC(=O)-, -C(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-,-N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-;
R³⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R^{a} is independently at each instance H or R^{b};
R^{b} is independently at each instance C₁₋₈alkyl, phenyl or benzyl; and
R^{c} is independently at each instance C₁₋₈alkyl, C₁₋₄haloalkyl, halo, cyano, nitro, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -OC₂₋₆alkylNR^{a}R^{a}, -OC₂₋₆alkylOR^{a}, -SR^{a}, -S(=O)R^{b}, -S(=-O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkylNR^{a}R^{a} or -NR^{a}C₂₋₆alkylOR^{a}.

Included herein are a multitude of other embodiments of the present invention, many of which are exemplified below.

In one embodiment, X is N or CH.

In another embodiment, in conjunction with any of the above or below embodiments, X is N.

In another embodiment, in conjunction with any of the above or below embodiments, X is CH.

In another embodiment, in conjunction with any of the above or below embodiments, Y is NH, N(CN), O or S.

In another embodiment, in conjunction with any of the above or below embodiments, Y is NH.

In another embodiment, in conjunction with any of the above or below embodiments, Y is O.

In another embodiment, in conjunction with any of the above or below embodiments, Y is S.

In another embodiment, in conjunction with any of the above or below embodiments, X is N and Y is O.

Embodiment A: In another embodiment, in conjunction with any of the above or below embodiments, R¹ is selected from -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ and -R¹¹-R¹⁴-R¹³-R¹⁴, any of which is substituted by 0, 1, 2, 3, 4 or 5 substituents independently selected from R^{c}.

In another embodiment, in conjunction with any of the above or below embodiments, R¹ is selected from -R¹¹, -R¹¹-R¹⁴, -R¹¹-R¹², -R¹¹-R¹²-R¹⁴, -R¹¹-R¹³-R¹⁴, -R¹¹-R¹³-R¹²-R¹⁴ and -R¹¹-R¹²-R¹³-R¹⁴, any of which is substituted by 0, 1, 2, 3, 4 or 5 substituents independently selected from R^{c}.

In another embodiment, in conjunction with any of the above or below embodiments, R¹ is R¹¹, which is substituted by 0, 1, 2, 3, 4 or 5 substituents independently selected from R^{c}.

Embodiment B: In another embodiment, in conjunction with any of the above or below embodiments, R¹ is -R¹¹-R¹⁴, which is substituted by 0, 1, 2, 3, 4 or 5 substituents independently selected from R^{c}.

Embodiment C: In another embodiment, in conjunction with any of the above or below embodiments, R¹ is -R¹¹-R¹², which is substituted by 0, 1, 2, 3, 4 or 5 substituents independently selected from R^{c}.

In another embodiment, in conjunction with any of the above or below embodiments, R¹ is -R¹¹-R¹²-R¹⁴*,* which is substituted by 0, 1, 2, 3, 4 or 5 substituents independently selected from R^{c}.

In another embodiment, in conjunction with any of the above or below embodiments, R¹ is -R¹¹-R¹³-R¹⁴, which is substituted by 0, 1, 2, 3, 4 or 5substituents independently selected from R^{c}.

In another embodiment, in conjunction with any of the above or below embodiments, R¹ is -R¹¹-R¹³-R¹², which is substituted by 0, 1, 2, 3, 4 or 5 substituents independently selected from R^{c}.

Embodiment D: In another embodiment, in conjunction with any of the above or below embodiments, R¹ is -R¹¹-R¹³-R¹²-R¹⁴, which is substituted by 0, 1, 2, 3, 4 or 5 substituents independently selected from R^{c}.

In another embodiment, in conjunction with any of the above or below embodiments, R¹ is -R¹¹-R¹²-R¹³-R¹⁴, which is substituted by 0, 1, 2, 3, 4 or 5 substituents independently selected from R^{c}.

In another embodiment, in conjunction with any of the above or below embodiments, R¹ is -R¹¹-R¹⁴-R¹²-R¹³, which is substituted by 0, 1, 2, 3, 4 or 5 substituents independently selected from R^{c}.

In another embodiment, in conjunction with any of the above or below embodiments, R¹ is -R¹¹-R¹⁴-R¹³-R¹², which is substituted by 0, 1, 2, 3, 4 or 5 substituents independently selected from R^{c}.

In another embodiment, in conjunction with any of the above or below embodiments, R¹ is -R¹¹-R¹⁴-R¹²-R¹⁴, which is substituted by 0, 1, 2, 3, 4 or 5 substituents independently selected from R^{c}.

In another embodiment, in conjunction with any of the above or below embodiments, R¹ is -R¹¹-R¹⁴-R¹³-R¹⁴, any of which is substituted by 0, 1, 2, 3, 4 or 5 substituents independently selected from R^{c}.

In another embodiment, in conjunction with any of the above or below embodiments, R² is selected from -R²¹, -R²¹-R²², -R²¹-R²⁴, -R²²-R²⁴, -R²¹-R²²-R²⁴, -R²¹-R²³-R²⁴, -R²²-R²³-R²⁴, -R²¹-R²³⁻R²²-R²⁴ and -R²¹-R²²-R²³-R²⁴, any of which is substituted by 0, 1, 2, 3, 4 or 5 substituents independently selected from R^{c}.

Embodiment E: In another embodiment, in conjunction with any of the above or below embodiments, R² is selected from -R²¹, -R²¹-R²⁴, -R²¹-R²², -R²¹-R²²-R²⁴, -R²¹-R²³-R²⁴, -R²¹-R²³-R²²-R²⁴ and -R²¹-R²²-R²³-R²⁴, any of which is substituted by 0, 1, 2, 3, 4 or 5 substituents independently selected from R^{c}.

Embodiment F: In another embodiment, in conjunction with any of the above or below embodiments, R² is R²¹, which is substituted by 0, 1, 2, 3, 4 or 5 substituents independently selected from R^{c}.

In another embodiment, in conjunction with any of the above or below embodiments, R² is -R²¹-R²⁴, which is substituted by 0, 1, 2, 3, 4 or 5 substituents independently selected from R^{c}.

In another embodiment, in conjunction with any of the above or below embodiments, R² is -R²¹-R²², which is substituted by 0, 1, 2, 3, 4 or 5 substituents independently selected from R^{c}.

In another embodiment, in conjunction with any of the above or below embodiments, R² is -R²¹-R²²-R²⁴, which is substituted by 0, 1, 2, 3, 4 or 5 substituents independently selected from R^{c}.

In another embodiment, in conjunction with any of the above or below embodiments, R² is -R²¹-R²³-R²⁴, which is substituted by 0, 1, 2, 3, 4 or 5 substituents independently selected from R^{c}.

Embodiment G: In another embodiment, in conjunction with any of the above or below embodiments, R² is -R²¹-R²³-R²²-R²⁴, which is substituted by 0, 1, 2, 3, 4 or 5 substituents independently selected from R^{c}.

In another embodiment, in conjunction with any of the above or below embodiments, R² is -R²¹-R²²-R²³-R²⁴, which is substituted by 0, 1, 2, 3, 4 or 5 substituents independently selected from R^{c}.

In another embodiment, in conjunction with any of the above or below embodiments, R² is phenyl substituted by 1, 2, 3, 4 or 5 substituents independently selected from R^{c}.

In another embodiment, in conjunction with any of the above or below embodiments, R² is a 2,5-disubstituted phenyl, wherein the two substituents are independently selected from R^{c}.

In another embodiment, in conjunction with any of the above or below embodiments, R² is a 2,5-disubstituted phenyl, wherein the two substituents are independently selected from C₁₋₂alkyl, halo and C₁₋₂haloalkyl.

In another embodiment, in conjunction with any of the above or below embodiments, R² is a 2,5-disubstituted phenyl, wherein the two substituents are independently selected from CH₃ and Cl.

In another embodiment, in conjunction with any of the above or below embodiments, R² is 2,5-dichlorophenyl.

In another embodiment, in conjunction with any of the above or below embodiments, R² is 2,5-dimethylphenyl.

Embodiment H: In another embodiment, in conjunction with any of the above or below embodiments, R³ is selected from -R³⁴, -R³², -R³²-R³⁴, -R³³-R³⁴, -R³³-R³²-R³⁴ and -R³²-R³³-R³⁴, any of which is substituted by 0,1,2,3,4 or 5 substituents independently selected from R^{c}.

Embodiment I: In another embodiment, in conjunction with any of the above or below embodiments, R³ is selected from -R³⁴, -R³², -R³²-R³⁴, -R³²-R³⁴, -R³³-R³⁴, R³²-R³³-R³⁴, R³³-R³²-R³⁴ and -R³²-R³³-R³⁴, any of which is substituted by 0,1,2,3,4 or 5 substituents independently selected from R^{c}.

Embodiment J: In another embodiment, in conjunction with any of the above or below embodiments, R³ is -R³⁴, which is substituted by 0,1,2,3,4 or 5 substituents independently selected from R^{c}.

Embodiment K: In another embodiment, in conjunction with any of the above or below embodiments, R³ is -R³², which is substituted by 0, 1, 2, 3, 4 or 5 substituents independently selected from R^{c}.

In another embodiment, in conjunction with any of the above or below embodiments, R³ is -R³²-R³⁴, which is substituted by 0, 1, 2, 3, 4 or 5 substituents independently selected from R^{c}.

In another embodiment, in conjunction with any of the above or below embodiments, R³ is -R³³-R³⁴, which is substituted by 0,1,2,3,4 or 5 substituents independently selected from R^{c}.

Embodiment L: In another embodiment, in conjunction with any of the above or below embodiments, R³ is -R³³-R³²-R³⁴, which is substituted by 0, 1, 2, 3, 4 or 5 substituents independently selected from R^{c}.

In another embodiment, in conjunction with any of the above or below embodiments, R³ is -R³²-R³³-R³⁴, which is substituted by 0, 1, 2, 3, 4 or 5 substituents independently selected from R^{c}.

In another embodiment, in conjunction with any of the above or below embodiments, R¹¹ is independently at each instance a saturated or unsaturated 5-, 6-or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹¹ is independently at each instance an unsaturated 5- or 6-membered monocyclic or 9- or 10-membered bicyclic ring containing 0,1,2,3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹¹ is independently at each instance an unsaturated 9- or 10-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹¹ is independently at each instance an unsaturated 5- or 6-membered monocyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹¹ is independently at each instance an unsaturated 5-membered monocyclic ring containing 1 atom selected from N, O and S.

In another embodiment, in conjunction with any of the above or below embodiments, R¹¹ is independently at each instance an unsaturated 6-membered monocyclic ring containing 0, 1 or 2 N atoms.

Embodiment M: In another embodiment, in conjunction with any of the above or below embodiments, R¹¹ is phenyl.

Embodiment N: In another embodiment, in conjunction with any of the above or below embodiments, R¹¹ is independently at each instance a saturated or unsaturated 5-, 6- or 7-membered monocyclic or 6-, 7-, 8-, 9-, 10- or 11-membered bicyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹¹ is independently at each instance an unsaturated 6-membered monocyclic ring containing 1 or 2 N atoms.

In another embodiment, in conjunction with any of the above or below embodiments, R¹¹ is phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine or morpholine.

In another embodiment, in conjunction with any of the above or below embodiments, R¹¹ is pyridinyl, pyrimidinyl or pyridazinyl.

In another embodiment, in conjunction with any of the above or below embodiments, R¹² is independently at each instance C₁₋₈alkyl.

In another embodiment, in conjunction with any of the above or below embodiments, R¹² is independently at each instance C₁₋₄alkyl.

In another embodiment, in conjunction with any of the above or below embodiments, R¹² is independently at each instance C₂₋₄alkyl.

Embodiment O: In another embodiment, in conjunction with any of the above or below embodiments, R¹³ is independently at each instance -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-.

In another embodiment, in conjunction with any of the above or below embodiments, R¹³ is independently at each instance -C(=O)-, -C(=O)O-, -C(=O)NR^{a}-, -C(=NR^{a})NR^{a}-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-.

In another embodiment, in conjunction with any of the above or below embodiments, R¹³ is independently at each instance -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-.

In another embodiment, in conjunction with any of the above or below embodiments, R¹³ is -O-.

In another embodiment, in conjunction with any of the above or below embodiments, R¹³ is -N(R^{a})-.

In another embodiment, in conjunction with any of the above or below embodiments, R¹³ is -N(R^{a})C(=O)-, -C(=O)NR^{a}-, -C(=O)O- or -OC(=O)-.

In another embodiment, in conjunction with any of the above or below embodiments, R¹³ is -O-, -N(R^{a})-, -N(R^{a})C(=O)-, -C(=O)NR^{a}-, -C(=O)O- or -OC(=O)-.

Embodiment P: In another embodiment, in conjunction with any of the above or below embodiments, R¹⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹⁴ is phenyl.

In another embodiment, in conjunction with any of the above or below embodiments, R¹⁴ is naphthyl.

In another embodiment, in conjunction with any of the above or below embodiments, R¹⁴ is independently at each instance a saturated or unsaturated 5-, 6-or 7-membered monocyclic or 6-, 7-, 8-, 9-, 10- or 11-membered bicyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹⁴ is independently at each instance a saturated or unsaturated 5-, 6-or 7-membered monocyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹⁴ is independently at each instance a saturated 5- or 6-membered monocyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R¹⁴ is independently at each instance a saturated 5- or 6-membered monocyclic ring containing 1 or 2 N atoms, wherein the carbon atoms of the ring are substituted by 0 or 1 oxo groups.

In another embodiment, in conjunction with any of the above or below embodiments, R¹⁴ is piperidinyl, piperazinyl or pyrrolidinyl.

In another embodiment, in conjunction with any of the above or below embodiments, R²¹ is independently at each instance a saturated or unsaturated 5-, 6-or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R²¹ is independently at each instance an unsaturated 5- or 6-membered monocyclic or 9- or 10-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R²¹ is independently at each instance an unsaturated 9- or 10-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R²¹ is independently at each instance an unsaturated 5- or 6-membered monocyclic ring containing 0,1,2,3 or 4 atoms selected from N, O and S, so long as the combination of O and S atoms is not greater than 2, wherein the carbon atoms of the ring are substituted by 0,1 or 2 oxo groups.

In another embodiment, in conjunction with any of the above or below embodiments, R²¹ is independently at each instance an unsaturated 5-membered monocyclic ring containing 1 atom selected from N, O and S.

In another embodiment, in conjunction with any of the above or below embodiments, R²¹ is independently at each instance an unsaturated 6-membered monocyclic ring containing 0, 1 or 2 N atoms.

Embodiment Q: In another embodiment, in conjunction with any of the above or below embodiments, R²¹ is phenyl.

Embodiment R: In another embodiment, in conjunction with any of the above or below embodiments, R²¹ is independently at each instance a saturated or unsaturated 5-, 6- or 7-membered monocyclic or 6-, 7-, 8-, 9-, 10- or 11-membered bicyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R²¹ is independently at each instance an unsaturated 6-membered monocyclic ring containing 1 or 2 N atoms.

In another embodiment, in conjunction with any of the above or below embodiments, R²¹ is phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine or morpholine.

In another embodiment, in conjunction with any of the above or below embodiments, R²¹ is pyridinyl, pyrimidinyl or pyridazinyl.

In another embodiment, in conjunction with any of the above or below embodiments, R²² is independently at each instance C₁₋₈alkyl.

In another embodiment, in conjunction with any of the above or below embodiments, R²² is independently at each instance C₁₋₄alkyl.

In another embodiment, in conjunction with any of the above or below embodiments, R²² is independently at each instance C₂₋₄alkyl.

Embodiment S: In another embodiment, in conjunction with any of the above or below embodiments, R²³ is independently at each instance -C(=O)-, -C(=O)O-, -C(=O)NR^{a}, -C(=NR^{a})NR^{a}-, -O-, -OC(=O)-, -OC(=O)NR^{a}-, -OC(=O)N(R^{a})S(=O)₂, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-.

In another embodiment, in conjunction with any of the above or below embodiments, R²³ is independently at each instance -C(=O)-, -C(=O)O-, -C(=O)NR^{a}-, -C(=NR^{a})NR^{a}-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-,

In another embodiment, in conjunction with any of the above or below embodiments, R²³ is independently at each instance -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-.

In another embodiment, in conjunction with any of the above or below embodiments, R²³ is -O-.

In another embodiment, in conjunction with any of the above or below embodiments, R²³ is -N(R^{a})-.

In another embodiment, in conjunction with any of the above or below embodiments, R²³ is -N(R^{a})C(=O)-, -C(=O)NR^{a}-, -C(=O)O- or -OC(=O)-.

In another embodiment, in conjunction with any of the above or below embodiments, R²³ is -O-, -N(R^{a})-, -N(R^{a})C(=O)-, -C(=O)NR^{a}-, -C(=O)O- or -OC(=O)-.

Embodiment T: In another embodiment, in conjunction with any of the above or below embodiments; R²⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R²⁴ is phenyl.

In another embodiment, in conjunction with any of the above or below embodiments, R²⁴ is naphthyl.

In another embodiment, in conjunction with any of the above or below embodiments, R²⁴ is independently at each instance a saturated or unsaturated 5-, 6-or 7-membered monocyclic or 6-, 7-, 8-, 9-, 10- or 11-membered bicyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R²⁴ is independently at each instance a saturated or unsaturated 5-, 6-or 7-membered monocyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R²⁴ is independently at each instance a saturated 5- or 6-membered monocyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R²⁴ is independently at each instance a saturated 5- or 6-membered monocyclic ring containing 1 or 2 N atoms, wherein the carbon atoms of the ring are substituted by 0 or 1 oxo groups.

In another embodiment, in conjunction with any of the above or below embodiments, R²⁴ is piperidinyl, piperazinyl or pyrrolidinyl.

In another embodiment, in conjunction with any of the above or below embodiments, L is a 3- or 4-atom chain made up of C and N atoms, wherein the chain is substituted by 0 or 1 R³ groups and the chain is additionally substituted by 0, 1, 2 or 3 substituents independently selected from R^{c}.

In another embodiment, in conjunction with any of the above or below embodiments, L is a 3-atom chain made up of C and N atoms, wherein the chain is substituted by 0 or 1 R³ groups and the chain is additionally substituted by 0, 1, 2 or 3 substituents independently selected from R^{c}.

In another embodiment, in conjunction with any of the above or below embodiments, L is a 4-atom chain formed up of C and N atoms, wherein the chain is substituted by 0 or 1 R³ groups and the chain is additionally substituted by 0, 1, 2 or 3 substituents independently selected from R^{c}.

Embodiment V: In another embodiment, in conjunction with any of the above or below embodiments, L is a 4-atom chain made up of C and N atoms, wherein the chain is substituted by 0 or 1 R³ groups and the chain is additionally substituted by 0, 1, 2 or 3 substituents independently selected from R^{c}.

In another embodiment, in conjunction with any of the above or below embodiments, L is a 4-atom chain made up of C and N atoms, wherein the chain is substituted by one R³ groups and the chain is additionally substituted by 0, 1, 2 or 3 substituents independently selected from R^{c}.

In another embodiment, in conjunction with any of the above or below embodiments, L is a 4-atom chain made up of C and N atoms, wherein the chain is substituted by 0,1,2 or 3 substituents independently selected from R^{c}.

In another embodiment, in conjunction with any of the above or below embodiments, L is -C=C-C=C- substituted by 0 or 1 R³ groups and the chain is additionally substituted by 0, 1, 2 or 3 substituents independently selected from R^{c}.

Embodiment V: In another embodiment, in conjunction with any of the above or below embodiments, L is -C=C-C=C- substituted by 0,1,2 or 3 substituents independently selected from R^{c}.

In another embodiment, in conjunction with any of the above or below embodiments, L is -CH=CH-CH=CH-.

In another embodiment, in conjunction with any of the above or below embodiments, L is a 4-atom chain containing C atoms and at least one N atom, wherein the chain is substituted by 0 or 1 R³ groups and the chain is additionally substituted by 0, 1, 2 or 3 substituents independently selected from R^{c}.

In another embodiment, in conjunction with any of the above or below embodiments, L is a 4-atom chain containing C atoms and 1 or 2 N atoms, wherein the chain is substituted by 0 or 1 R³ groups and the chain is additionally substituted by 0,1, 2 or 3 substituents independently selected from R^{c}.

In another embodiment, in conjunction with any of the above or below embodiments, L is a chain selected from -N=C-C=C-, -C=N-C=C-, -C=C-N=C- and -C=C-C=N-, wherein the chain is substituted by 0 or 1 R³ groups and the chain is additionally substituted by 0, 1, 2 or 3 substituents independently selected from R^{c}.

In another embodiment, in conjunction with any of the above or below embodiments, R³² is independently at each instance C₁₋₈alkyl.

In another embodiment, in conjunction with any of the above or below embodiments, R³² is independently at each instance C₁₋₄alkyl.

In another embodiment, in conjunction with any of the above or below embodiments, R³² is independently at each instance C₂₋₄alkyl.

Embodiment W: In another embodiment, in conjunction with any of the above or below embodiments, R³³ is independently at each instance -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-.

In another embodiment, in conjunction with any of the above or below embodiments, R³³ is independently at each instance -C(=O)-, -C(=O)O-, -C(=O)NR^{a}-, -C(=NR^{a})NR^{a}-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-.

In another embodiment, in conjunction with any of the above or below embodiments, R³³ is independently at each instance -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-.

In another embodiment, in conjunction with any of the above or below embodiments, R³³ is -O-.

In another embodiment, in conjunction with any of the above or below embodiments, R³³ is -N(R^{a})-.

In another embodiment, in conjunction with any of the above or below embodiments, R³³ is -N(R^{a})C(=O)-, -C(=O)NR^{a}-, -C(=O)O- or -OC(=O)-.

In another embodiment, in conjunction with any of the above or below embodiments, R³³ is -O-, -N(R^{a})-, -N(R^{a})C(=O)-, -C(=O)NR^{a}-, -C(=O)O- or -OC(=O)-.

Embodiment X: In another embodiment, in conjunction with any of the above or below embodiments, R³⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R³⁴ is phenyl.

In another embodiment, in conjunction with any of the above or below embodiments, R³⁴ is naphthyl.

In another embodiment, in conjunction with any of the above or below embodiments, R³⁴ is independently at each instance a saturated or unsaturated 5-, 6-or 7-membered monocyclic or 6-, 7-, 8-, 9-, 10- or 11-membered bicyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R³⁴ is independently at each instance a saturated or unsaturated 5-, 6-or 7-membered monocyclic ring containing 1, 2, 3 or 4 atoms selected from N, O and S, so long as the combination of O and S atoms is hot greater than 2, wherein the carbon atoms of the ring are substituted by 0, 1 or 2 oxo groups.

In another embodiment, in conjunction with any of the above or below embodiments, R³⁴ is independently at each instance a saturated 5- or 6-membered monocyclic ring containing 1, 2, 3 or 4 atoms selected from N, O and S, so long as the combination of O and S atoms is not greater than 2, wherein the carbon atoms of the ring are substituted by 0, 1 or 2 oxo groups.

In another embodiment, in conjunction with any of the above or below embodiments, R³⁴ is independently at each instance a saturated 5- or 6-membered monocyclic ring containing 1 or 2 N atoms, wherein the carbon atoms of the ring are substituted by 0 or 1 oxo groups.

In another embodiment, in conjunction with any of the above or below embodiments, R³⁴ is piperidinyl, piperazinyl or pyrrolidinyl.

In another embodiment of the invention, a compound of Formula I is provided, wherein
X is N;
Y is O;
L is a 4-atom chain formed of C atoms, wherein the chain is substituted by 0 or 1 R³ groups and the chain is additionally substituted by 0, 1, 2 or 3 substituents independently selected from R^{c};
R¹ is selected from -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ and -R¹¹-R¹⁴-R¹³-R¹⁴, any of which is substituted by 0, 1, 2, 3, 4 or 5 substituents independently selected from R^{c};
R² is selected from -R²¹, -R²¹-R²², -R²¹-R²⁴, -R²²-R²⁴, -R²¹-R²²-R²⁴, -R²¹-R²³-R²⁴ and -R²²-R²³-R²⁴, any of which is substituted by 0, 1, 2, 3, 4 or 5 substituents independently selected from R^{c};
R³ is selected from -R³², -R³⁴, -R³²-R³⁴, -R³²-R³⁴, -R³³-R³⁴, -R³²-R³³-R³⁴, -R³³-R³²-R³⁴ and -R³²-R³³-R³⁴ any of which is substituted by 0, 1, 2, 3, 4 or 5 substituents independently selected from R^{c};
R¹¹ is independently at each instance a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine or morpholine;
R¹² is independently at each instance C₁₋₈alkyl;
R¹³ is independently at each instance -C(=O)-, -C(=O)O-, -C(=O)N(R^{a})-, -C(=NR^{a})N(R^{a}) -, -O-, -OC(=O)N(R^{a})₂₋, -OC₂₋₆alkylN(R^{a})-, -OC₂₋₆alkyl-, -OC₂₋₆akylO-, -S-, -S(=O)-, -S(=O)₂-, -S(=O)₂N(R^{a}) -, -S(=O)₂N(R^{a})C(=O)-, -N(R^{a})₂-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})-, or -NR^{a}C₂₋₆alkylO-;
R¹⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R²¹ is independently at each instance a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine or morpholine;
R²² is independently at each instance C₁₋₈alkyl;
R²³ is independently at each instance -C(=O)-, -C(=O)O-, -C(=O)N(R^{a}) -, -C(=NR^{a})N(R^{a})-, -O-, -OC(=O)-, -OC(=O)N(R^{a})-, -OC(=O)N(R^{a})S(=O)₂-, -OC₂₋₆alkylN(R^{a})-, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})₂-, -N(R^{a})-,-N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a}) -, -NR^{a}C₂₋₆alkylN(R^{a})- or -NR^{a}C₂₋₆alkylO-;
R²⁴ is independently at each instance a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine, morpholine, pyran, dioxane, cyclopropane, cyclobutane, cyclopentane or cyclohexane;
R³² is independently at each instance C₁₋₈alkyl;
R³³ is independently at each instance -C(=O)-, -C(=O)O-, -C(=O)N(R^{a})-, -C(=NR^{a})N(R^{a}) -, -O-, -OC₂₋₆alkylN(R^{a}) -, -OC₂₋₆alkylO-, -S-, -S(=O)-, -S(=O)₂-, -S(=O)₂N(R^{a}) -, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})-or -NR^{a}C₂₋₆alkylO-;
R³⁴ is independently at each instance a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine, morpholine, pyran, dioxane, cyclopropane, cyclobutane, cyclopentane or cyclohexane;
R^{a} is independently at each instance H or R^{b};
R^{b} is independently at each instance C₁₋₈alkyl, CN, phenyl or benzyl; and
R^{c} is independently at each instance C₁₋₈alkyl, C₁₋₄haloalkyl, halo, cyano, nitro, -C₂₋₆alkylOR^{a}, -C₂₋₆alkyl C(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -OC₂₋₆alkylNR^{a}R^{a}, -OC₂₋₆alkylC(=O)NR^{a}R^{a}, -OC₂₋₆alkylOR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkylNR^{a}R^{a} or -NR^{a}C₂₋₆alkylOR^{a}.

In another embodiment, a compound of Formula I is provided, wherein
L is an unsubstituted 4-atom chain formed of C atoms;
R¹¹ is a phenyl, naphthylene, pyridine, pyrazine, triazine, quinoline, isoquinoline or thiophene;
R¹⁴ is a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine, morpholine, pyran, dioxane, cyclopropane, cyclobutane, cyclopentane or cyclohexane; and
R²¹ is phenyl, pyridine, pyrazine, triazine or thiophene.

In another embodiment, a compound of Formula I is provided, wherein
R^{c} is independently at each instance methyl, ethyl, propyl, isopropyl, n-butyl, sec-butyl, tert-butyl, pentyl, C₁₋₄haloalkyl, F, Cl, Br, cyano, nitro, -C₂₋₆alkylOR^{a}, -C₂₋₆alkyl C(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC₂₋₆alkylNR^{a}R^{a}, -OC₂₋₆alkylC(=O)NR^{a}R^{a}, -OC₂₋₆alkylOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkylNR^{a}R^{a} or -NR^{a}C₂₋₆alkylOR^{a}.

In another embodiment, a compound of Formula II 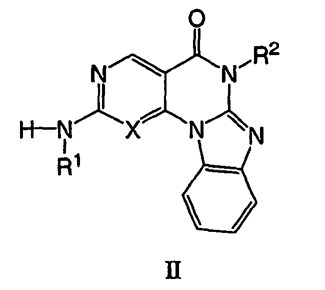 or a pharmaceutically-acceptable salt or derivative thereof is provided, wherein
R¹ is selected from -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ and -R¹¹-R¹⁴-R¹³-R¹⁴, any of which is substituted by 0, 1, 2, 3, 4 or 5 substituents independently selected from R^{c};
R² is selected from -R²¹, -R²¹-R²², -R²¹-R²⁴ and -R²²-R²⁴, any of which is substituted by 0, 1, 2, 3, 4 or 5 substituents independently selected from R^{c};
R¹¹ is independently at each instance a phenyl, pyridine or pyrazine;
R¹² is independently at each instance C₁₋₈alkyl;
R¹³ is independently at each instance -C(=O)-, -C(=O)O-, -C(=O)N(R^{a})-, -C(=NR^{a})N(R^{a})-, -O-, -OC(=O)N(R^{a})₂-, -OC₂₋₆alkylN(Ra)-, -OC₂₋₆alkyl-, -OC₂₋₆alkylO-, -S-, -S(=O)-, -S(=O)₂-, -S(=O)₂N(R^{a})-, -S(=O)₂N(R^{a})C(=O)-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})-, or -NR^{a}C₂₋₆alkylO-;
R¹⁴ is phenyl, pyridine, pyrazine, thiophene, pyrrole, pyrrolidine, furan, tetrahyrofuran, piperidine, piperazine, morpholine, cyclopropane, cyclopentane or cyclohexane;
R²¹ is phenyl, pyridine or pyrazine;
R²² is independently at each instance C₁₋₈alkyl;
R²³ is independently at each instance -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -OC₂₋₆alkylN(R^{a})-, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})₂-, -N(R^{a})-,-N(R^{a})C(-O)-, -N(R^{a})C(=O)O-, -N(R^{a})C(=O)N(R^{a}) -, -N(R^{a})C(=NR^{a})N(R^{a})-, -N(R^{a})S(=O)₂-, -N(R^{a})S(=O)₂N(R^{a}) -, -NR^{a}C₂₋₆alkylN(R^{a}) - or -NR^{a}C₂₋₆alkylO-;
R²⁴ is phenyl, pyridine, pyrazine, thiophene, pyrrole, pyrrolidine, furan, tetrahyrofuran, piperidine, piperazine, morpholine, cyclopropane, cyclopentane or cyclohexane;
R^{a} is independently at each instance H or R^{b};
R^{b} is independently at each instance C₁₋₈alkyl, CN, phenyl or benzyl; and
R^{c} is independently at each instance methyl, ethyl, propyl, isopropyl, n-butyl, sec-butyl, tert-butyl, pentyl, C₁₋₄haloalkyl, F, Cl, Br, cyano, nitro, -C₂₋₆alkyl OR^{a}, -C₂₋₆alkylC(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC₂₋₆alkylNR^{a}R^{a}, -OC₂₋₆alkylC(=O)NR^{a}R^{a}, -OC₂₋₆alkylOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkylNR^{a}R^{a} or -NRC₂₋₆alkylOR^{a}.

As stated above, the above embodiments may be used inconjuction with other embodiments listed. The following table is a non-exclusive, non-limiting list of some of the combinations of embodiments. For the structure 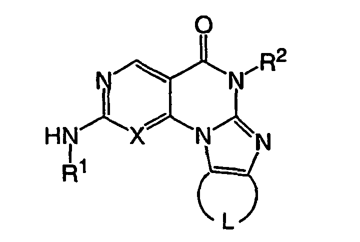 wherein
R¹², R²² and R³² are independently selected from C₁₋₄alkyl; R¹³, R²³ and R³³ are independently selected from Embodiments O, S and W, respectively; and R¹⁴, R²⁴ and R³⁴ are independently selected from Embodiments P, T and X, respectively.

| **No.** | **R¹** | **R¹¹** | **R²** | **R²¹** | **L** |
|---|---|---|---|---|---|
| 1001 | A | M | E | Q | U |
| 1002 | A | M | E | Q | V |
| 1003 | A | M | E | Q | U |
| 1004 | A | M | E | Q | V |
| 1005 | A | M | E | Q | U |
| 1006 | A | M | E | Q | V |
| 1007 | A | M | E | Q | U |
| 1008 | A | M | E | Q | V |
| 1009 | A | M | E | Q | U |
| 1010 | A | M | E | Q | V |
| 1011 | A | M | E | R | U |
| 1012 | A | M | E | R | V |
| 1013 | A | M | E | R | U |
| 1014 | A | M | E | R | V |
| 1015 | A | M | E | R | U |
| 1016 | A | M | E | R | V |
| 1017 | A | M | E | R | U |
| 1018 | A | M | E | R | V |
| 1019 | A | M | E | R | U |
| 1020 | A | M | E | R | V |
| 1021 | A | M | F | Q | U |
| 1022 | A | M | F | Q | V |
| 1023 | A | M | F | Q | U |
| 1024 | A | M | F | Q | V |
| 1025 | A | M | F | Q | U |
| 1026 | A | M | F | Q | V |
| 1027 | A | M | F | Q | U |
| 1028 | A | M | F | Q | V |
| 1029 | A | M | F | Q | U |
| 1030 | A | M | F | Q | V |
| 1031 | A | M | F | R | U |
| 1032 | A | M | F | R | V |
| 1033 | A | M | F | R | U |
| 1034 | A | M | F | R | V |
| 1035 | A | M | F | R | U |
| 1036 | A | M | F | R | V |
| 1037 | A | M | F | R | U |
| 1038 | A | M | F | R | V |
| 1039 | A | M | F | R | U |
| 1040 | A | M | F | R | V |
| 1041 | A | M | G | Q | U |
| 1042 | A | M | G | Q | V |
| 1043 | A | M | G | Q | U |
| 1044 | A | M | G | Q | V |
| 1045 | A | M | G | Q | U |
| 1046 | A | M | G | Q | V |
| 1047 | A | M | G | Q | U |
| 1048 | A | M | G | Q | V |
| 1049 | A | M | G | Q | U |
| 1050 | A | M | G | Q | V |
| 1051 | A | M | G | R | U |
| 1052 | A | M | G | R | V |
| 1053 | A | M | G | R | U |
| 1054 | A | M | G | R | V |
| 1055 | A | M | G | R | U |
| 1056 | A | M | G | R | V |
| 1057 | A | M | G | R | U |
| 1058 | A | M | G | R | V |
| 1059 | A | M | G | R | U |
| 1060 | A | M | G | R | V |
| 1061 | A | N | E | Q | U |
| 1062 | A | N | E | Q | V |
| 1063 | A | N | E | Q | U |
| 1064 | A | N | E | Q | V |
| 1065 | A | N | E | Q | U |
| 1066 | A | N | E | Q | V |
| 1067 | A | N | E | Q | U |
| 1068 | A | N | E | Q | V |
| 1069 | A | N | E | Q | U |
| 1070 | A | N | E | Q | V |
| 1071 | A | N | E | R | U |
| 1072 | A | N | E | R | V |
| 1073 | A | N | E | R | U |
| 1074 | A | N | E | R | V |
| 1075 | A | N | E | R | U |
| 1076 | A | N | E | R | V |
| 1077 | A | N | E | R | U |
| 1078 | A | N | E | R | V |
| 1079 | A | N | E | R | U |
| 1080 | A | N | E | R | V |
| 1081 | A | N | F | Q | U |
| 1082 | A | N | F | Q | V |
| 1083 | A | N | F | Q | U |
| 1084 | A | N | F | Q | V |
| 1085 | A | N | F | Q | U |
| 1086 | A | N | F | Q | V |
| 1087 | A | N | F | Q | U |
| 1088 | A | N | F | Q | V |
| 1089 | A | N | F | Q | U |
| 1090 | A | N | F | Q | V |
| 1091 | A | N | F | R | U |
| 1092 | A | N | F | R | V |
| 1093 | A | N | F | R | U |
| 1094 | A | N | F | R | V |
| 1095 | A | N | F | R | U |
| 1096 | A | N | F | R | V |
| 1097 | A | N | F | R | U |
| 1098 | A | N | F | R | V |
| 1099 | A | N | F | R | U |
| 1100 | A | N | F | R | V |
| 1101 | A | N | G | Q | U |
| 1102 | A | N | G | Q | V |
| 1103 | A | N | G | Q | U |
| 1104 | A | N | G | Q | V |
| 1105 | A | N | G | Q | U |
| 1106 | A | N | G | Q | V |
| 1107 | A | N | G | Q | U |
| 1108 | A | N | G | Q | V |
| 1109 | A | N | G | Q | U |
| 1110 | A | N | G | Q | V |
| 1111 | A | N | G | R | U |
| 1112 | A | N | G | R | V |
| 1113 | A | N | G | R | U |
| 1114 | A | N | G | R | V |
| 1115 | A | N | G | R | U |
| 1116 | A | N | G | R | V |
| 1117 | A | N | G | R | U |
| 1118 | A | N | G | R | V |
| 1119 | A | N | G | R | U |
| 1120 | A | N | G | R | V |
| 1121 | B | M | E | Q | U |
| 1122 | B | M | E | Q | V |
| 1123 | B | M | E | Q | U |
| 1124 | B | M | E | Q | V |
| 1125 | B | M | E | Q | U |
| 1126 | B | M | E | Q | V |
| 1127 | B | M | E | Q | U |
| 1128 | B | M | E | Q | V |
| 1129 | B | M | E | Q | U |
| 1130 | B | M | E | Q | V |
| 1131 | B | M | E | R | U |
| 1132 | B | M | E | R | V |
| 1133 | B | M | E | R | U |
| 1134 | B | M | E | R | V |
| 1135 | B | M | E | R | U |
| 1136 | B | M | E | R | V |
| 1137 | B | M | E | R | U |
| 1138 | B | M | E | R | V |
| 1139 | B | M | E | R | U |
| 1140 | B | M | E | R | V |
| 1141 | B | M | F | Q | U |
| 1142 | B | M | F | Q | V |
| 1143 | B | M | F | Q | U |
| 1144 | B | M | F | Q | V |
| 1145 | B | M | F | Q | U |
| 1146 | B | M | F | Q | V |
| 1147 | B | M | F | Q | U |
| 1148 | B | M | F | Q | V |
| 1149 | B | M | F | Q | U |
| 1150 | B | M | F | Q | V |
| 1151 | B | M | F | R | U |
| 1152 | B | M | F | R | V |
| 1153 | B | M | F | R | U |
| 1154 | B | M | F | R | V |
| 1155 | B | M | F | R | U |
| 1156 | B | M | F | R | V |
| 1157 | B | M | F | R | U |
| 1158 | B | M | F | R | V |
| 1159 | B | M | F | R | U |
| 1160 | B | M | F | R | V |
| 1161 | B | M | G | Q | U |
| 1162 | B | M | G | Q | V |
| 1163 | B | M | G | Q | U |
| 1164 | B | M | G | Q | V |
| 1165 | B | M | G | Q | U |
| 1166 | B | M | G | Q | V |
| 1167 | B | M | G | Q | U |
| 1168 | B | M | G | Q | V |
| 1169 | B | M | G | Q | U |
| 1170 | B | M | G | Q | V |
| 1171 | B | M | G | R | U |
| 1172 | B | M | G | R | V |
| 1173 | B | M | G | R | U |
| 1174 | B | M | G | R | V |
| 1175 | B | M | G | R | U |
| 1176 | B | M | G | R | V |
| 1177 | B | M | G | R | U |
| 1178 | B | M | G | R | V |
| 1179 | B | M | G | R | U |
| 1180 | B | M | G | R | V |
| 1181 | B | N | E | Q | U |
| 1182 | B | N | E | Q | V |
| 1183 | B | N | E | Q | U |
| 1184 | B | N | E | Q | V |
| 1185 | B | N | E | Q | U |
| 1186 | B | N | E | Q | V |
| 1187 | B | N | E | Q | U |
| 1188 | B | N | E | Q | V |
| 1189 | B | N | E | Q | U |
| 1190 | B | N | E | Q | V |
| 1191 | B | N | E | R | U |
| 1192 | B | N | E | R | V |
| 1193 | B | N | E | R | U |
| 1194 | B | N | E | R | V |
| 1195 | B | N | E | R | U |
| 1196 | B | N | E | R | V |
| 1197 | B | N | E | R | U |
| 1198 | B | N | E | R | V |
| 1199 | B | N | E | R | U |
| 1200 | B | N | E | R | V |
| 1201 | B | N | F | Q | U |
| 1202 | B | N | F | Q | V |
| 1203 | B | N | F | Q | U |
| 1204 | B | N | F | Q | V |
| 1205 | B | N | F | Q | U |
| 1206 | B | N | F | Q | V |
| 1207 | B | N | F | Q | U |
| 1208 | B | N | F | Q | V |
| 1209 | B | N | F | Q | U |
| 1210 | B | N | F | Q | V |
| 1211 | B | N | F | R | U |
| 1212 | B | N | F | R | V |
| 1213 | B | N | F | R | U |
| 1214 | B | N | F | R | V |
| 1215 | B | N | F | R | U |
| 1216 | B | N | F | R | V |
| 1217 | B | N | F | R | U |
| 1218 | B | N | F | R | V |
| 1219 | B | N | F | R | U |
| 1220 | B | N | F | R | V |
| 1221 | B | N | G | Q | U |
| 1222 | B | N | G | Q | V |
| 1223 | B | N | G | Q | U |
| 1224 | B | N | G | Q | V |
| 1225 | B | N | G | Q | U |
| 1226 | B | N | G | Q | V |
| 1227 | B | N | G | Q | U |
| 1228 | B | N | G | Q | V |
| 1229 | B | N | G | Q | U |
| 1230 | B | N | G | Q | V |
| 1231 | B | N | G | R | U |
| 1232 | B | N | G | R | V |
| 1233 | B | N | G | R | U |
| 1234 | B | N | G | R | V |
| 1235 | B | N | G | R | U |
| 1236 | B | N | G | R | V |
| 1237 | B | N | G | R | U |
| 1238 | B | N | G | R | V |
| 1239 | B | N | G | R | U |
| 1240 | B | N | G | R | V |
| 1241 | C | M | E | Q | U |
| 1242 | C | M | E | Q | V |
| 1243 | C | M | E | Q | U |
| 1244 | C | M | E | Q | V |
| 1245 | C | M | E | Q | U |
| 1246 | C | M | E | Q | V |
| 1247 | C | M | E | Q | U |
| 1248 | C | M | E | Q | V |
| 1249 | C | M | E | Q | U |
| 1250 | C | M | E | Q | V |
| 1251 | C M | M | E | R | U |
| 1252 | C | M | E | R | V |
| 1253 | C | M | E | R | U |
| 1254 | C | M | E | R | V |
| 1255 | C | M | E | R | U |
| 1256 | C | M | E | R | V |
| 1257 | C | M | E | R | U |
| 1258 | C | M | E | R | V |
| 1259 | C | M | E | R | U |
| 1260 | C | M | E | R | V |
| 1261 | C | M | F | Q | U |
| 1262 | C | M | F | Q | V |
| 1263 | C | M | F | Q | U |
| 1264 | C | M | F | Q | V |
| 1265 | C | M | F | Q | U |
| 1266 | C | M | F | Q | V |
| 1267 | C | M | F | Q | U |
| 1268 | C | M | F | Q | V |
| 1269 | C | M | F | Q | U |
| 1270 | C | M | F | Q | V |
| 1271 | C | M | F | R | U |
| 1272 | C | M | F | R | V |
| 1273 | C | M | F | R | U |
| 1274 | C | M | F | R | V |
| 1275 | C | M | F | R | U |
| 1276 | C | M | F | R | V |
| 1277 | C | M | F | R | U |
| 1278 | C | M | F | R | V |
| 1279 | C | M | F | R | U |
| 1280 | C | M | F | R | V |
| 1281 | C | M | G | Q | U |
| 1282 | C | M | G | Q | V |
| 1283 | C | M | G | Q | U |
| 1284 | C | M | G | Q | V |
| 1285 | C | M | G | Q | U |
| 1286 | C | M | G | Q | V |
| 1287 | C | M | G | Q | U |
| 1288 | C | M | G | Q | V |
| 1289 | C | M | G | Q | U |
| 1290 | C | M | G | Q | V |
| 1291 | C | M | G | R | U |
| 1292 | C | M | G | R | V |
| 1293 | C | M | G | R | U |
| 1294 | C | M | G | R | V |
| 1295 | C | M | G | R | U |
| 1296 | C | M | G | R | V |
| 1297 | C | M | G | R | U |
| 1298 | C | M | G | R | V |
| 1299 | C | M | G | R | U |
| 1300 | C | M | G | R | V |
| 1301 | C | N | E | Q | U |
| 1302 | C | N | E | Q | V |
| 1303 | C | N | E | Q | U |
| 1304 | C | N | E | Q | V |
| 1305 | C | N | E | Q | U |
| 1306 | C | N | E | Q | V |
| 1307 | C | N | E | Q | U |
| 1308 | C | N | E | Q | V |
| 1309 | C | N | E | Q | U |
| 1310 | C | N | E | Q | V |
| 1311 | C | N | E | R | U |
| 1312 | C | N | E | R | V |
| 1313 | C | N | E | R | U |
| 1314 | C | N | E | R | V |
| 1315 | C | N | E | R | U |
| 1316 | C | N | E | R | V |
| 1317 | C | N | E | R | U |
| 1318 | C | N | E | R | V |
| 1319 | C | N | E | R | U |
| 1320 | C | N | E | R | V |
| 1321 | C | N | F | Q | U |
| 1322 | C | N | F | Q | V |
| 1323 | C | N | F | Q | U |
| 1324 | C | N | F | Q | V |
| 1325 | C | N | F | Q | U |
| 1326 | C | N | F | Q | V |
| 1327 | C | N | F | Q | U |
| 1328 | C | N | F | Q | V |
| 1329 | C | N | F | Q | U |
| 1330 | C | N | F | Q | V |
| 1331 | C | N | F | R | U |
| 1332 | C | N | F | R | V |
| 1333 | C | N | F | R | U |
| 1334 | C | N | F | R | V |
| 1335 | C | N | F | R | U |
| 1336 | C | N | F | R | V |
| 1337 | C | N | F | R | U |
| 1338 | C | N | F | R | V |
| 1339 | C | N | F | R | U |
| 1340 | C | N | F | R | V |
| 1341 | C | N | G | Q | U |
| 1342 | C | N | G | Q | V |
| 1343 | C | N | G | Q | U |
| 1344 | C | N | G | Q | V |
| 1345 | C | N | G | Q | U |
| 1346 | C | N | G | Q | V |
| 1347 | C | N | G | Q | U |
| 1348 | C | N | G | Q | V |
| 1349 | C | N | G | Q | U |
| 1350 | C | N | G | Q | V |
| 1351 | C | N | G | R | U |
| 1352 | C | N | G | R | V |
| 1353 | C | N | G | R | U |
| 1354 | C | N | G | R | V |
| 1355 | C | N | G | R | U |
| 1356 | C | N | G | R | V |
| 1357 | C | N | G | R | U |
| 1358 | C | N | G | R | V |
| 1359 | C | N | G | R | U |
| 1360 | C | N | G | R | V |
| 1361 | D | M | E | Q | U |
| 1362 | D | M | E | Q | V |
| 1363 | D | M | E | Q | U |
| 1364 | D | M | E | Q | V |
| 1365 | D | M | E | Q | U |
| 1366 | D | M | E | Q | V |
| 1367 | D | M | E | Q | U |
| 1368 | D | M | E | Q | V |
| 1369 | D | M | E | Q | U |
| 1370 | D | M | E | Q | V |
| 1371 | D | M | E | R | U |
| 1372 | D | M | E | R | V |
| 1373 | D | M | E | R | U |
| 1374 | D | M | E | R | V |
| 1375 | D | M | E | R | U |
| 1376 | D | M | E | R | V |
| 1377 | D | M | E | R | U |
| 1378 | D | M | E | R | V |
| 1379 | D | M | E | R | U |
| 1380 | D | M | E | R | V |
| 1381 | D | M | F | Q | U |
| 1382 | D | M | F | Q | V |
| 1383 | D | M | F | Q | U |
| 1384 | D | M | F | Q | V |
| 1385 | D | M | F | Q | U |
| 1386 | D | M | F | Q | V |
| 1387 | D | M | F | Q | U |
| 1388 | D | M | F | Q | V |
| 1389 | D | M | F | Q | U |
| 1390 | D | M | F | Q | V |
| 1391 | D | M | F | R | U |
| 1392 | D | M | F | R | V |
| 1393 | D | M | F | R | U |
| 1394 | D | M | F | R | V |
| 1395 | D | M | F | R | U |
| 1396 | D | M | F | R | V |
| 1397 | D | M | F | R | U |
| 1398 | D | M | F | R | V |
| 1399 | D | M | F | R | U |
| 1400 | D | M | F | R | V |
| 1401 | D | M | G | Q | U |
| 1402 | D | M | G | Q | V |
| 1403 | D | M | G | Q | U |
| 1404 | D | M | G | Q | V |
| 1405 | D | M | G | Q | U |
| 1406 | D | M | G | Q | V |
| 1407 | D | M | G | Q | U |
| 1408 | D | M | G | Q | V |
| 1409 | D | M | G | Q | U |
| 1410 | D | M | G | Q | V |
| 1411 | D | M | G | R | U |
| 1412 | D | M | G | R | V |
| 1413 | D | M | G | R | U |
| 1414 | D | M | G | R | V |
| 1415 | D | M | G | R | U |
| 1416 | D | M | G | R | V |
| 1417 | D | M | G | R | U |
| 1418 | D | M | G | R | V |
| 1419 | D | M | G | R | U |
| 1420 | D | M | G | R | V |
| 1421 | D | N | E | Q | U |
| 1422 | D | N | E | Q | V |
| 1423 | D | N | E | Q | U |
| 1424 | D | N | E | Q | V |
| 1425 | D | N | E | Q | U |
| 1426 | D | N | E | Q | V |
| 1427 | D | N | E | Q | U |
| 1428 | D | N | E | Q | V |
| 1429 | D | N | E | Q | U |
| 1430 | D | N | E | Q | V |
| 1431 | D | N | E | R | U |
| 1432 | D | N | E | R | V |
| 1433 | D | N | E | R | U |
| 1434 | D | N | E | R | V |
| 1435 | D | N | E | R | U |
| 1436 | D | N | E | R | V |
| 1437 | D | N | E | R | U |
| 1438 | D | N | E | R | V |
| 1439 | D | N | E | R | U |
| 1440 | D | N | E | R | V |
| 1441 | D | N | F | Q | U |
| 1442 | D | N | F | Q | V |
| 1443 | D | N | F | Q | U |
| 1444 | D | N | F | Q | V |
| 1445 | D | N | F | Q | U |
| 1446 | D | N | F | Q | V |
| 1447 | D | N | F | Q | U |
| 1448 | D | N | F | Q | V |
| 1449 | D | N | F | Q | U |
| 1450 | D | N | F | Q | V |
| 1451 | D | N | F | R | U |
| 1452 | D | N | F | R | V |
| 1453 | D | N | F | R | U |
| 1454 | D | N | F | R | V |
| 1455 | D | N | F | R | U |
| 1456 | D | N | F | R | V |
| 1457 | D | N | F | R | U |
| 1458 | D | N | F | R | V |
| 1459 | D | N | F | R | U |
| 1460 | D | N | F | R | V |
| 1461 | D | N | G | Q | U |
| 1462 | D | N | G | Q | V |
| 1463 | D | N | G | Q | U |
| 1464 | D | N | G | Q | V |
| 1465 | D | N | G | Q | U |
| 1466 | D | N | G | Q | V |
| 1467 | D | N | G | Q | U |
| 1468 | D | N | G | Q | V |
| 1469 | D | N | G | Q | U |
| 1470 | D | N | G | Q | V |
| 1471 | D | N | G | R | U |
| 1472 | D | N | G | R | V |
| 1473 | D | N | G | R | U |
| 1474 | D | N | G | R | V |
| 1475 | D | N | G | R | U |
| 1476 | D | N | G | R | V |
| 1477 | D | N | G | R | U |
| 1478 | D | N | G | R | V |
| 1479 | D | N | G | R | U |
| 1480 | D | N | G | R | V |

Another aspect of the invention relates to a pharmaceutical composition comprising a compound according to any one of the above embodiments and a pharmaceutically acceptable carrier.

Another aspect of the invention relates to a method of treatment of inflammation comprising administering a therapeutically-effective amount of a compound according to any one of the above embodiments.

Another aspect of the invention relates to a method of inhibition of T cell activation and proliferation in a mammal in need thereof, comprising administering a therapeutically-effective amount of a compound according to any one of the above embodiments.

Another aspect of the invention relates to a method of treatment of arthritis, rheumatoid arthritis, psoriatic arthritis, or osteoarthritis in a mammal comprising administering a therapeutically-effective amount of a compound according to any one of the above embodiments.

Another aspect of the invention relates to a method of treatment of organ transplant, acute transplant or heterograft or homograft rejection, or transplantation tolerance induction in a mammal comprising administering a therapeutically-effective amount of a compound according to any one of the above embodiments.

Another aspect of the invention relates to a method of treatment of ischemic or reperfusion injury, myocardial infarction, or stroke in a mammal in need thereof, comprising administering a therapeutically-effective amount of a compound according to any one of the above embodiments.

Another aspect of the invention relates to a method of treatment of multiple sclerosis, inflammatory bowel disease, including ulcerative colitis, Crohn's disease, lupus, contact hypersensitivity, delayed-type hypersensitivity, and gluten-sensitive enteropathy, type 1 diabetes, psoriasis, contact dermatitis, Hashimoto's thyroiditis, Sjogren's syndrome, autoimmune hyperthyroidism, Addison's disease, autoimmune polyglandular disease, autoimmune alopecia, pernicious anemia, vitiligo, autoimmune hypopituatarism, Guillain-Barre syndrome, glomerulonephritis, serum sickness, uticaria, allergic diseases, asthma, hayfever, allergic rhinitis, scleracielma, mycosis fungoides, dermatomyositis, alopecia areata, chronic actinic dermatitis, eczema, Behcet's disease, Pustulosis palmoplanteris, Pyoderma gangrenum, Sezary's syndrome, atopic dermatitis, systemic schlerosis, morphea or atopic dermatitis in a mammal comprising administering a therapeutically-effective amount of a compound according to any one of the above embodiments.

Another aspect of the invention relates to a method of treatment of colon carcinoma or thymoma in a mammal comprising administering a therapeutically-effective amount of a compound according to any one of the above embodiments.

Another aspect of the invention relates to the manufacture of a medicament comprising a compound according to any one of the above embodiments.

Another aspect of the invention relates to the manufacture of a medicament for the treatment of inflammation comprising a therapeutically-effective amount of a compound according to any one of the above embodiments.

Another aspect of the invention relates to the manufacture of a medicament for the inhibition of T cell activation and proliferation in a mammal in need thereof, comprising a therapeutically-effective amount of a compound according to any one of the above embodiments.

Another aspect of the invention relates to the manufacture of a medicament for the treatment of arthritis, rheumatoid arthritis, psoriatic arthritis, or osteoarthritis in a mammal comprising a therapeutically-effective amount of a compound according to any one of the above embodiments.

Another aspect of the invention relates to the manufacture of a medicament for the treatment of organ transplant, acute transplant or heterograft or homograft rejection, or transplantation tolerance induction in a mammal comprising a therapeutically-effective amount of a compound according to any one of the above embodiments.

Another aspect of the invention relates to the manufacture of a medicament for the treatment of ischemic or reperfusion injury, myocardial infarction, or stroke in a mammal in need thereof, comprising a therapeutically-effective amount of a compound according to any one of the above embodiments.

Another aspect of the invention relates to the manufacture of a medicament for the treatment of multiple sclerosis, inflammatory bowel disease, including ulcerative colitis, Crohn's disease, lupus, contact hypersensitivity, delayed-type hypersensitivity, and gluten-sensitive enteropathy, type 1 diabetes, psoriasis, contact dermatitis, Hashimoto's thyroiditis, Sjogren's syndrome, autoimmune hyperthyroidism, Addison's disease, autoimmune polyglandular disease, autoimmune alopecia, pernicious anemia, vitiligo, autoimmune hypopituatarism, Guillain-Barre syndrome, glomerulonephritis, serum sickness, uticaria, allergic diseases, asthma, hayfever, allergic rhinitis, scleracielma, mycosis fungoides, dermatomyositis, alopecia areata, chronic actinic dermatitis, eczema, Behcet's disease, Pustulosis palmoplanteris, Pyoderma gangrenum, Sezary's syndrome, atopic dermatitis, systemic schlerosis, morphea or atopic dermatitis in a mammal comprising a therapeutically-effective amount of a compound according to any one of the above embodiments.

Another aspect of the invention relates to the manufacture of a medicament for the treatment of colon carcinoma or thymoma in a mammal comprising a therapeutically-effective amount of a compound according to any one of the above embodiments.

Another aspect of the invention relates to a method of making a compound as described herein, the method comprising the steps of: reacting a pyrimidine ester (A) 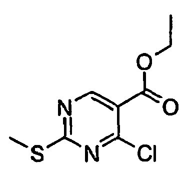 with a benzimidazole (B) 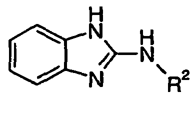 in the presence of a base to form a tetracyclic pyrimidine (C) 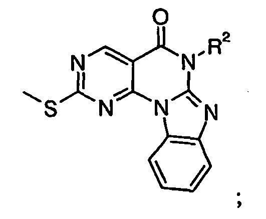 oxidizing the sulfur of the tetracyclic pyrimidine (C) to form a tetracyclic pyrimidine sulfone (D) 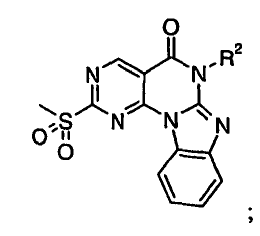 and
reacting the tetracyclic pyrimidine sulfone (D) with R¹NH to make a compound 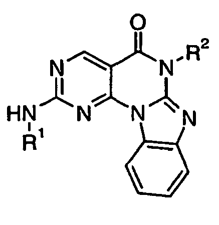 according to Claim 5.

The compounds of this invention may have in general several asymmetric centers and are typically depicted in the form of racemic mixtures. This invention is intended to encompass racemic mixtures, partially racemic mixtures and separate enantiomers and diasteromers.

The specification and claims contain listing of species using the language "selected from ... and ..." and "is ... or ..." (sometimes referred to as Markush groups). When this language is used in this application, unless otherwise stated it is meant to include the group as a whole, or any single members thereof, or any subgroups thereof. The use of this language is merely for shorthand purposes and is not meant in any way to limit the removal of individual elements or subgroups from the genus.

Unless otherwise specified, the following definitions apply to terms found in the specification and claims:
"Aryl" means a phenyl or naphthyl radical, wherein the phenyl may be fused with a C₃₋₄cycloalkyl bridge.
"Benzo group", alone or in combination, means the divalent radical C₄H₄=, one representation of which is -CH=CH-CH=CH-, that when vicinally attached to another ring forms a benzene-like ring--for example tetrahydronaphthylene, indole and the like.
"C_{α-β}alkyl" means an alkyl group comprising from α to β carbon atoms in a branched, cyclical or linear relationship or any combination of the three. The alkyl groups described in this section may also contain double or triple bonds. Examples of C₁₋₈alkyl include, but are not limited to the following: 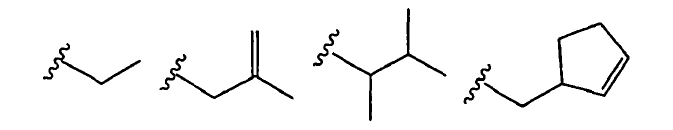
"Halogen" and "halo" mean a halogen atoms selected from F, Cl, Br and I.
"C_{α-β}haloalkyl" means an alkyl group, as described above, wherein any number--at least one--of the hydrogen atoms attached to the alkyl chain are replaced by F, Cl, Br or I.
"Heterocycle" means a ring comprising at least one carbon atom and at least one other atom selected from N, O and S. Examples of heterocycles that may be found in the claims include, but are not limited to, the following: 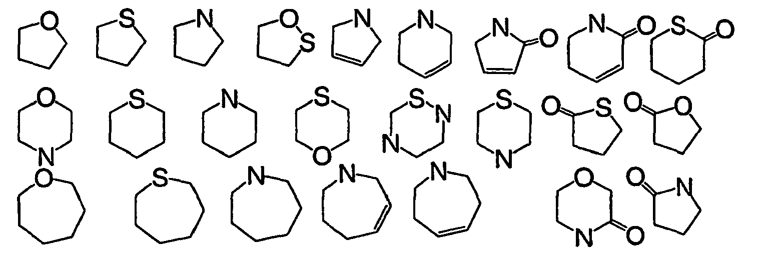 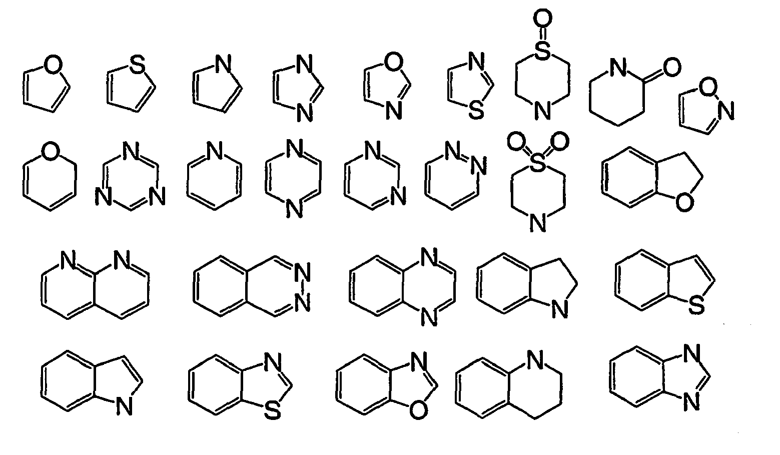 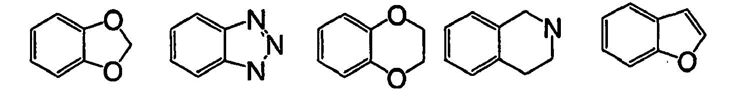 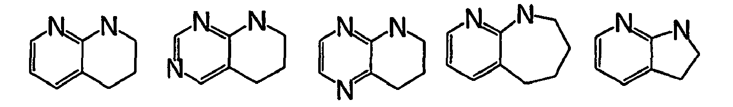 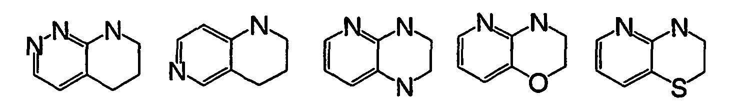 and 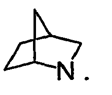
"Saturated or unsaturated" means a substitutent that is completely saturated, completely unsaturated, or has any degree of unsaturation in between. Examples of a saturated or unsaturated 6-membered ring carbocycle would include phenyl, cyclohexyl, cyclohexenyl and cyclohexadienyl.

Substituents, including rings and alkyl groups, may be either monovalent or polyvalent depending on the context of their usage. For example, if description contained the group R^{α}-R^{β}-R^{γ} and R^{β} was defined as C₁₋₆alkyl, then the R^{β} alkyl would be considered polyvalent because it must be bonded to at least R^{α} and R^{γ}. Alternatively, if R^{γ} was defined as C₁₋₆alkyl, then the R^{γ} alkyl would be monovalent (excepting any further substitution language).

"Pharmaceutically-acceptable salt" means a salt prepared by conventional means, and are well known by those skilled in the art. The "pharmacologically acceptable salts" include basic salts of inorganic and organic acids, including but not limited to hydrochloric acid, hydrobromic acid, sulfuric acid, phosphoric acid, methanesulphonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. When compounds of the invention include an acidic function such as a carboxy group, then suitable pharmaceutically acceptable cation pairs for the carboxy group are well known to those skilled in the art and include alkaline, alkaline earth, ammonium, quaternary ammonium cations and the like. For additional examples of "pharmacologically acceptable salts," *see infra* and Berge et al., J. Pharm. Sci. 66:1 (1977).

"Leaving group" generally refers to groups readily displaceable by a nucleophile, such as an amine, a thiol or an alcohol nucleophile. Such leaving groups are well known in the art. Examples of such leaving groups include, but are not limited to, N-hydroxysuccinimide, N-hydroxybenzotriazole, halides, triflates, tosylates and the like. Preferred leaving groups are indicated herein where appropriate.

"Protecting group" generally refers to groups well known in the art which are used to prevent selected reactive groups, such as carboxy, amino, hydroxy, mercapto and the like, from undergoing undesired reactions, such as nucleophilic, electrophilic, oxidation, reduction and the like. Preferred protecting groups are indicated herein where appropriate. Examples of amino protecting groups include, but are not limited to, aralkyl, substituted aralkyl, cycloalkenylalkyl and substituted cycloalkenyl alkyl, allyl, substituted allyl, acyl, alkoxycarbonyl, aralkoxycarbonyl, silyl and the like. Examples of aralkyl include, but are not limited to, benzyl, ortho-methylbenzyl, trityl and benzhydryl, which can be optionally substituted with halogen, alkyl, alkoxy, hydroxy, nitro, acylamino, acyl and the like, and salts, such as phosphonium and ammonium salts. Examples of aryl groups include phenyl, naphthyl, indanyl, anthracenyl, 9-(9-phenylfluorenyl), phenanthrenyl, durenyl and the like. Examples of cycloalkenylalkyl or substituted cycloalkylenylalkyl radicals, preferably have 6-10 carbon atoms, include, but are not limited to, cyclohexenyl methyl and the like. Suitable acyl, alkoxycarbonyl and aralkoxycarbonyl groups include benzyloxycarbonyl, t-butoxycarbonyl, iso-butoxycarbonyl, benzoyl, substituted benzoyl, butyryl, acetyl, tri-fluoroacetyl, tri-chloro acetyl, phthaloyl and the like. A mixture of protecting groups can be used to protect the same amino group, such as a primary amino group can be protected by both an aralkyl group and an aralkoxycarbonyl group. Amino protecting groups can also form a heterocyclic ring with the nitrogen to which they are attached, for example, 1,2-bis(methylene)benzene, phthalimidyl, succinimidyl, maleimidyl and the like and where these heterocyclic groups can further include adjoining aryl and cycloalkyl rings. In addition, the heterocyclic groups can be mono-, di- or tri-substituted, such as nitrophthalimidyl. Amino groups may also be protected against undesired reactions, such as oxidation, through the formation of an addition salt, such as hydrochloride, toluenesulfonic acid, trifluoroacetic acid and the like. Many of the amino protecting groups are also suitable for protecting carboxy, hydroxy and mercapto groups. For example, aralkyl groups. Alkyl groups are also suitable groups for protecting hydroxy and mercapto groups, such as tert-butyl.

Silyl protecting groups are silicon atoms optionally substituted by one or more alkyl, aryl and aralkyl groups. Suitable silyl protecting groups include, but are not limited to, trimethylsilyl, triethylsilyl, tri-isopropylsilyl, tert-butyldimethylsilyl, dimethylphenylsilyl, 1,2-bis(dimethylsilyl)benzene, 1,2-bis(dimethylsilyl)ethane and diphenylmethylsilyl. Silylation of an amino groups provide mono- or di-silylamino groups. Silylation of aminoalcohol compounds can lead to a N,N,O-tri-silyl derivative. Removal of the silyl function from a silyl ether function is readily accomplished by treatment with, for example, a metal hydroxide or ammonium fluoride reagent, either as a discrete reaction step or in situ during a reaction with the alcohol group. Suitable silylating agents are, for example, trimethylsilyl chloride, tert-butyl-dimethylsilyl chloride, phenyldimethylsilyl chloride, diphenylmethyl silyl chloride or their combination products with imidazole or DMF. Methods for silylation of amines and removal of silyl protecting groups are well known to those skilled in the art. Methods of preparation of these amine derivatives from corresponding amino acids, amino acid amides or amino acid esters are also well known to those skilled in the art of organic chemistry including amino acid/amino acid ester or aminoalcohol chemistry.

Protecting groups are removed under conditions which will not affect the remaining portion of the molecule. These methods are well known in the art and include acid hydrolysis, hydrogenolysis and the like. A preferred method involves removal of a protecting group, such as removal of a benzyloxycarbonyl group by hydrogenolysis utilizing palladium on carbon in a suitable solvent system such as an alcohol, acetic acid, and the like or mixtures thereof. A t-butoxycarbonyl protecting group can be removed utilizing an inorganic or organic acid, such as HCl or trifluoroacetic acid, in a suitable solvent system, such as dioxane or methylene chloride. The resulting amino salt can readily be neutralized to yield the free amine. Carboxy protecting group, such as methyl, ethyl, benzyl, tert-butyl, 4-methoxyphenylmethyl and the like, can be removed under hydrolysis and hydrogenolysis conditions well known to those skilled in the art.

It should be noted that compounds of the invention may contain groups that may exist in tautomeric forms, such as cyclic and acyclic amidine and guanidine groups, heteroatom substituted heteroaryl groups (Y' = O, S, NR), and the like, which are illustrated in the following examples: 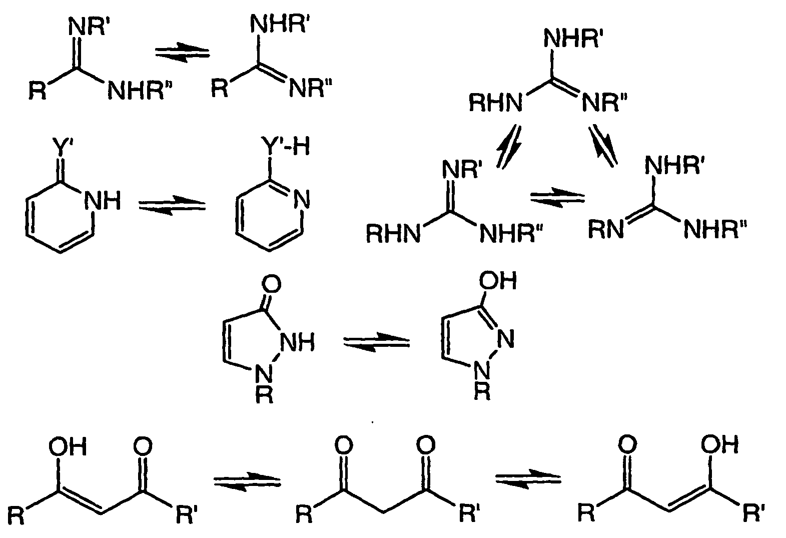 and though one form is named, described, displayed and/or claimed herein, all the tautomeric forms are intended to be inherently included in such name, description, display and/or claim.

"Derivative" generally refers to simple modifications, readily apparent to those of ordinary skill in the art, on the parent core structure of Formulas I-III, which does not significantly affect (generally decrease) the activity of the compound in-vitro as well as in vivo, in a subject. For example, a pro-drug is encompassed within the term "derivative".

Prodrugs of the compounds of this invention are, therefore, also contemplated by this invention. A pro-drug is a compound, which when administered to the body of subject, breaks down in the subject's metabolic pathway to provide an active compound of Formulas I-III. Thus, a prodrug is an active or inactive compound that is modified chemically through in vivo physiological action, such as hydrolysis, metabolism and the like, into a compound of this invention following administration of the prodrug to a patient. The suitability and techniques involved in making and using prodrugs are well known by those skilled in the art. For a general discussion of prodrugs involving esters see Svensson and Tunek Drug Metabolism Reviews 165 (1988) and Bundgaard Design of Prodrugs, Elsevier (1985). Examples of a masked carboxylate anion include a variety of esters, such as alkyl (for example, methyl, ethyl), cycloalkyl (for example, cyclohexyl), aralkyl (for example, benzyl, p-methoxybenzyl), and alkylcarbonyloxyalkyl (for example, pivaloyloxymethyl). Amines have been masked as arylcarbonyloxymethyl substituted derivatives which are cleaved by esterases in vivo releasing the free drug and formaldehyde (Bundgaard J. Med. Chem. 2503 (1989)). Also, drugs containing an acidic NH group, such as imidazole, imide, indole and the like, have been masked with N-acyloxymethyl groups (Bundgaard Design of Prodrugs, Elsevier (1985)). Hydroxy groups have been masked as esters and ethers. EP 039,051 (Sloan and Little, 4/11/81) discloses Mannich-base hydroxamic acid prodrugs, their preparation and use.

"Cytokine" means a secreted protein that affects the functions of other cells, particularly as it relates to the modulation of interactions between cells of the immune system or cells involved in the inflammatory response. Examples of cytokines include but are not limited to interleukin 1 (IL-1), preferably IL-1β, interleukin 6 (IL-6), interleukin 8 (IL-8) and TNF, preferably TNF-α (tumor necrosis factor-α).

"TNF, IL-1, IL-6, and/or IL-8 mediated disease or disease state" means all disease states wherein TNF, IL-1, IL-6, and/or IL-8 plays a role, either directly as TNF, IL-1, IL-6, and/or IL-8 itself, or by TNF, IL-1, IL-6, and/or IL-8 inducing another cytokine to be released. For example, a disease state in which IL-1 plays a major role, but in which the production of or action of IL-1, is a result of TNF, would be considered mediated by TNF.

### Synthesis

Compounds according to the invention can be synthesized according to one or more of the following methods. It should be noted that the general procedures are shown as it relates to preparation of compounds having unspecified stereochemistry. However, such procedures are generally applicable to those compounds of a specific stereochemistry, e.g., where the stereochemistry about a group is (S) or (R). In addition, the compounds having one stereochemistry (e.g., (R)) can often be utilized to produce those having opposite stereochemistry (i.e., (S)) using well-known methods, for example, by inversion.

The following Examples are presented for illustrative purposes only and are not intended, nor should they be construed, as limiting the invention in any manner. Those skilled in the art will appreciate that modifications and variations of the compounds disclosed herein can be made without violating the spirit or scope of the present invention.

### Synthesis

General schemes A-F describe various exemplary methods of synthesizing anilines and substituted anilines. Each General scheme further describes general methods to carry out each step of the general schemes. General Methods A-C, following General schemes A-F, describe the synthesis of tetracyclic pyrimidines of Formulas I-III, which utilize the various anilines described in schemes A-F.

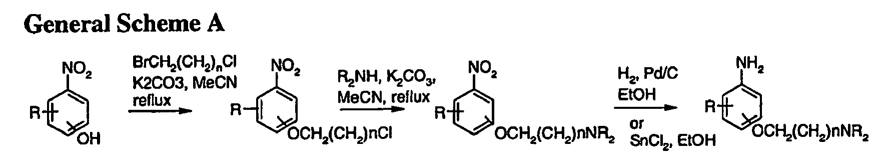

### General Method A

### 3-(4-Nitrophenoxy)propyl chloride

Nitrophenol (10 g, 72 mmol) was dissolved in acetonitrile (100 mL) and potassium carbonate (24.9 g, 180 mmol) added followed by bromochloropropane (113.2 g, 720 mmol). The mixture was heated and stirred under reflux overnight. The reaction was cooled to room temperature, the solid was then filtered off and the solvent evaporated under reduced pressure, taking care to remove all excess alkylating agent, to give the title compound.

### N,N-dimethyl-3-(4-nitrophenoxy)propylamine

A mixture of 3-(4-nitrophenoxy)propyl chloride (2 g, 9.27 mmol), potassium carbonate (7.69 g, 46.4 mmol) and acetonitrile (15 mL) were stirred in a sealed tube and dimethylamine hydrochloride (3.78 g, 46.4 mmol) added quickly. The mixture was stirred and heated overnight at 80 °C. The mixture was cooled well before opening the pressure tube, then water and dichloromethane were added and the aqueous layer was extracted with dichloromethane. The combined organics were dried and evaporated giving the title product. ¹H NMR (400 MHz, CDCl₃): 1.95 (2H, t, *J*7 Hz); 2.2 (6H, s); 2.35-2.45 (2H, m); 4.05 (2H, t, *J* 7 Hz); 6.9 (2H, d, J 8 Hz); 8.1 (2H, d, J 8 Hz)

### N,N-dimethyl-3-(4-aminophenoxy)propylamine

*N,N*-dimethyl-3-(4-nitrophenoxy)propylamine (4.4 g, 19.6 mmol) was hydrogenated over Pd (10% on C, 0.4 g) in ethanol (ca 50 mL) for 16 h. The catalysts was filtered off and the solvent removed under reduced pressure to afford the title compound as a brown oil. ¹H NMR (400 MHz, dmso-d6): 1.95 (2H, t, *J* 6.5 Hz); 2.25 (6H, s); 2.35-2.45 (2H, m); 3.95 (2H, t, *J* 6.5 Hz); 4.7 (2H, bs); 6.9 (2H, d, *J* 8 Hz); 8.1 (2H, d, *J* 8 Hz); 6.65 (2H, d, *J* 8 Hz); 6.75 (2H, d, *J* 8 Hz)

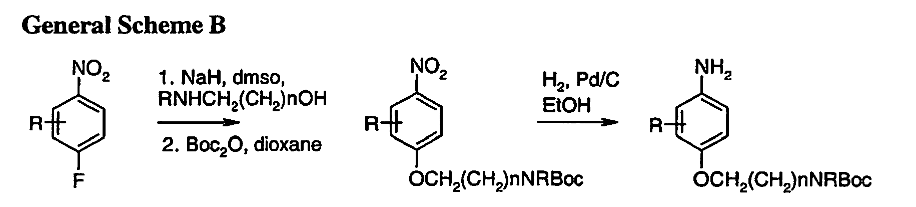

### General Method B

### Isopropyl-[2-(4-nitrophenoxy)ethyl]amine

Deprotonation of DMSO (anhydrous, 5 mL) was effected with NaH (0.40g, 60 wt% in mineral oil, 10 mmol) over 30 min at 40 °C with stirring under a nitrogen atmosphere. When 2-isopropylaminoethanol (1.15 mL, 10 mmol) was added to the solution of the DMSO anion at room temperature, some effervescence occurred. 4-Fluoronitrobenzene (1.06 mL, 10 mmol) was added after 10 min and the dark red solution was then stirred at room temperature for further 20 min. The reaction was diluted with dichloromethane (100 mL), washed with water (50 mL) and then extrected twice with 3M HCl (100 mL). The combined acidic extracts were washed once with dichloromethane (50 mL). Ethyl acetate (125 mL) was then added and the mixture was cooled to 6-8 °C before the aqueous layer was adjusted to pH 11 by gradual addition of 5M aq. NaOH (ca. 150 mL), with vigorous stirring. The organic layer was separated and washed twice with water (50 mL) dried over magnesium sulfate, and concentrated in vacuo at 35 °C to afford the title compound as a yellow oil. ¹H NMR (400 MHz, CDCl₃): 1.10 (6H, d, J 6.25), 2.88 (1H, m, J 6.25), 3.04 (2H, t, J 5.2), 4.16 (2H, t, J 5.2), 6.96 (2H, d, J 9.3), 8.18 (2H, d, J 9.3); MS: 225

### Isopropyl-[2-(4-nitrophenoxy)ethyl]carbamic acid tert-butyl ester

Isopropyl-[2-(4-nitrophenoxy)ethyl]amine (1.80 g, 8.05 mmol) was dissolved in 1,4-dioxane (containing 1% water, 20 mL) and cooled to 0-5 °C. Di-*tert*-butyldicarbonate (1.76 g, 8.05 mmol) was added slowly with vigorous stirring. The reaction was stirred at 0 °C for 0.5 h, then at room temperature for 20 h. The solvent was removed in vacuo and the residue taken up into EtOAc. The organic layer was washed twice with water (25 mL), the aqueous washes are extracted back with EtOAc (25 mL). The combined organic extracts were washed twice with 0.3 M HCl (25 mL), then brine and are dried over sodium sulfate. The solvent was removed in vacuo to afford a yellow solid, which was recrystallised from hot n-hexane to give the crystalline title compound as fine, light-yellow needles. ¹H NMR: (400 MHz, CDCl₃): 1.06 (6H, d, J 6.8), 1.37 (9H, s), 3.90 (2H, bm, 2H), 4.06 (2H, bm), 4.26 (1H, bm), 6.86 (2H, d, J 9.0), 8.09 (2H, d, J 9.2). MS: 225 [M+H⁺-Boc]).

### Isopropyl-[2-(4-aminophenoxy)ethyl]carbamic acid tert-butyl ester

A solution of isopropyl-[2-(4-aminophenoxy)ethyl]carbamic acid *tert*-butyl ester (2.09 g, 6.45 mmol) in ethanol/tetrahydrofuran (30 mL, 2:1) was reduced over palladium on carbon (10 wt%, 50% wet, 0.4 g) with hydrogen under atmospheric pressure at room temperature for 20 h. The catalyst was separated by filtration through celite. The solvent was removed *in vacuo* to afford the title compound as a red oil. ¹H NMR: (400 MHz, CDCl₃): 1.08 (6H, d, J 6.7), 1.39 (9H, s), 3.34 (2H, bm), 3.90 (2H, bm), 4.26 (1H, bm), 6.56 (2H, d, J 8.9), 6.67 (2H, d, J 8.9); MS: 195 [M+H⁺-Boc], 295 [M+H⁺]

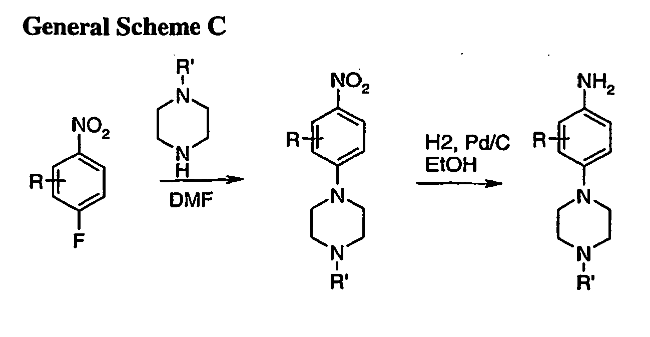

### General Method C

### 1-(2-Fluoro-4-nitrophenyl)-4-methylpiperazine

N-Methylpiperazine (30 mL, 27.1 g, 0.268 mol) was cooled in ice/water while adding 3,4-difluoronitrobenzene (2.0 g, 0.0126 mol) with stirring. The mixture was then heated at 100 °C overnight, evaporated to remove all excess *N*-methylpiperazine and the residue dissolved in 1M hydrochloric acid (30 mL). After washing twice with 20 mL portions of dichloromethane the solution was basified with 5M sodium hydroxide (10 mL). The product was extracted into dichloromethane (twice with 20 mL), dried over sodium sulphate and evaporated giving a yellow oil which solidified on standing. ¹H NMR (CDCl₃) 8.00 (m, 1H) 7.91 (m, 1H) 6.92 (m, 1H) 3.33 (m, 4H) 2.63 (m, 4H) 2.39 (s, 3H).

### 1-(2-Fluoro-4-aminophenyl)-4-methylpiperazine

Obtained by hydrogenation over Pd-10%C of the corresponding nitro compound in ethanol. ¹H NMR (CDCl₃) 6.75 (m, 1H) 6.33 (m, 2H) 3.48 (m, 2H) 2.94 (m, 4H) 2.53 (m, 4H) 2.29 (s, 3H).

### Specific syntheses:

### tert-Butyl 4-(2-difluoromethoxy-4-nitrophenyl)piperazine-1-carboxylate 1-(Z-Difluoromethoxy-4-nitrophenyl)piperazine

A stirred mixture of 1-bromo-2-difluoromethoxy-4-nitrobenzene (prepared from the corresponding phenol following the procedure outlined in WO9749710A1; 2.68 g, 10 mmol), piperazine (1.12 g, 13 mmol), potassium carbonate (2.07 g, 15 mmol), tetrabutylammonium bromide (0.03 g, 0.1 mmol) and dry dimethyl sulphoxide (20 mL) was heated under nitrogen at 120 °C for 3 h. The product was added to water (100 mL) and 6M hydrochloric acid (10 mL, 60 mmol), washed with ethyl acetate until washings colourless and the aqueous layer basified with 5M sodium hydroxide solution (20 mL, 100 mmol). Extraction with ethyl acetate (3x with 50 mL), drying (sodium sulphate) and evaporating gave product as viscous orange oil. ¹H NMR (CDCl₃) 8.00 (m, 1H) 7.92 (m, 1H) 6.93 (m,1H) 6.47 (t, J = 73.6, 1H) 3.18 (m, 2H) 2.98 (m, 2H) 2.54 (s, 1H)

### tert-Butyl 4-(2-difluoromethoxy-4-nitrophenyl)piperazine-1-carboxylate

The above product (1.64 g, 6 mmol) was dissolved in dry tetrahydrofuran (25 mL) and di-*tert*-butyl dicarbonate (1.26 g, 6 mmol) added. After stirring overnight the mixture was evaporated and the resulting orange solid recrystallised from ethyl acetate giving the final product. ¹H NMR (CDCl₃) 8.03 (m, 1H) 7.93 (m, 1H) 6.48 (t, 1H) 3.53 (m, 2H) 3.15 (m, 2H) 1.42 (s, 9H).

### tert-Butyl 4-(2-difluoromethoxy-4-aminophenyl)piperazine-1-carboxylate

Obtained by hydrogenation over Pd-10%C of the corresponding nitro compound in ethanol. ¹H NMR (CDCl₃) 7.73 (m, 1H) 6.56 (t, 1H) 6.42 (m, 2H) 3.46 (m, 2H) 2.80 (m, 2H) 1.40 (s, 9H).

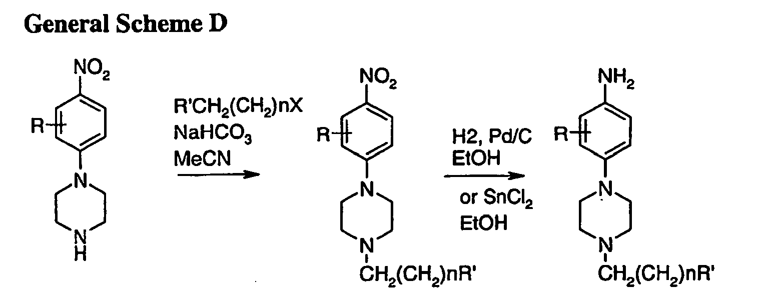

### General Method D

### 4-(4-(3-dimethylaniinopropyl)piperazino)nitrobenzene

Prepared according to a slightly modified procedure from US Pat. No. 3,331,845. A mixture of 4-nitrophenylpiperazine (2.1 g, 10 mmol), sodium hydrogen carbonate (2.5 g, 30 mmol), *N*,*N*-dimethyl-*N*-(3-chloropropyl)amine hydrochloride (1.9 g, 12 mmol) in isopropanol (80 mL) was heated at 80 °C for 18 h. The mixture was then allowed to cool, the solid filtered off and the solvent removed under reduced pressure. Ethyl acetate (ca. 200 mL) was added and the residue was washed with saturated brine twice (50 mL each time). The organic layer was dried over sodium sulphate and the solvent evaporated under reduced pressure. The crude compound was purified by column, eluting with dichloromethane/methanol 9/1 (containing 1% *N,N*-dimethylethylamine) to give the title compound as a yellow solid. ¹H NMR (CDCl₃, 400 MHz): 1.6 (2H, m); 2.15 (6H, s); 2.25 (2H, m); 2.35 (2H, m); 2.5-2.55 (4H, m); 3.35-3.4 (4H, m); 6.75 (2H, d, *J* 8 Hz); 8.05 (2H, d, *J* 8 Hz) MS:293,248

### 4-(4-(3-Dimethylaminopropyl)piperazino)aniline

A solution of 4-(4-(3-dimethylaminopropyl)piperazino)nitrobenzene (1.5 g) in methanol (50 mL) was hydrogenated at atmospheric pressure over Pd (5% on carbon) (0.3 g; 50% water content) for 4 h. The catalyst was filtered off and the solvent removed under reduced pressure to give the title compound as a brown solid ¹H NMR (CDCl₃, 400 MHz): 1.65 (2H, m); 2.15 (6H, s); 2.25 (2H, m); 2.35 (2H, m); 2.5-2.55 (4H, m); 2.95-3.05 (4H, m); 6.55 (2H, d, *J* 7 Hz); 6.75 (2H, d, *J* 7 Hz) MS: 263, 218

### 4-(4-(3-chlorobenzyl)piperazino)aniline

4-(4-(3-chlorobenzyl)piperazino)nitrobenzene (3 g, 9 mmol, prepared as in the general method) was dissolved in ethanol (100 mL). Tin (II) chloride dihydrate (10.1 g, 45 mmol) was added and the reaction heated to 80 °C for 66 h. The reaction mixture was concentrated under reduced pressure. A saturated solution (200 mL) of Rochelle's salt (sodium potassium tartrate) was prepared, and solid NaHCO₃ was added to this until no more would dissolve. Ethyl acetate (200 mL) was added to the vessel, followed by the reaction mixture. The solution was then stirred until clear. The phases were separated, and the aqueous layer washed with ethyl acetate (50 mL). The organic layers were combined, washed with saturated brine, dried over magnesium sulphate, and evaporated to give the title compound. ¹H NMR (CDCl₃, 400 MHz): 2.5-2.55 (4H, m); 2.95-3.05 (4H, m); 3.47 (2H, s); 6.5-6.6 (2H, m); 6.7-6.8 (4H, m); 7.15-7.25 (4H,m); MS: 302, 304

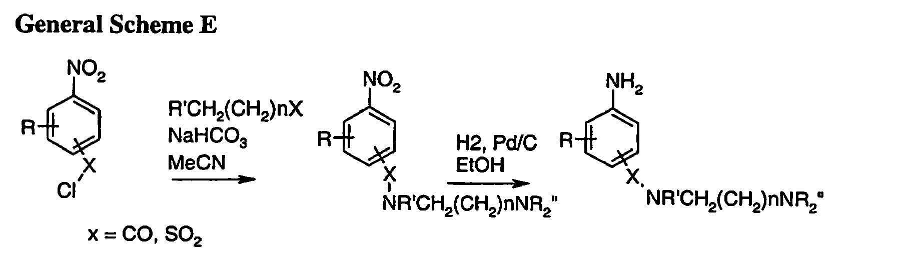

### General Method E

### N-(2-dimethylaminoethyl)-3-nitrobenzamide

3-Nitrobenzoyl chloride (2 g, 10.77 mmol) was loaded into a round bottomed flask, placed under a N₂ atmosphere and dissolved in anhydrous dichloromethane (10 mL). The mixture was cooled to 0 °C and *N,N* dimethylethylenediamine (0.98 mL, 8.98 mmol) was added to the reaction. The reaction was allowed to warm to room temperature and left to stir for 18 h. After 18 h the reaction had given a precipitate which was isolated by filtration and washed with dichloromethane to give 2.28 g of a white solid, which was partitioned between dichloromethane and a saturated aqueous NaHCO₃ solution. The organic layer was removed under educed pressure and the aqueous layer was then re-extracted dichloromethane. The organic layers were combined, dried over Na₂SO₄ and the solvent was removed under reduced pressure to afford the title compound as a yellow solid. MS: 193, 238; ¹H NMR (400 MHz, dmso-d6): 2.19 (6H, s), 2.42 (2H, t, *J* 6.8 Hz), 3.39 (2H, q, *J* 12.4 Hz, 6.7 Hz), 7.78 (1H, t, *J* 7.9 Hz), 8.29 (1H, ddd, *J* 7.9 1.8, 1.1 Hz), 8.38 (1H, ddd, *J* 8.1 Hz, 2.3, 1.0 Hz), 8.68 (1H, t, *J* 1.8 Hz), 8.81 (1H, t, *J* 5.7 Hz).

### 3-(N-(2b-dimethylaminoethylcarbamoyl))aniline

Palladium on carbon (200 mg, 10%w/w) was loaded to a three-necked flask and ethanol (1 mL) was added. This was then fitted with a three-way tap with balloon. The flask was then placed under vacuum then purged with nitrogen, this was repeated twice more. The amide (2.0 g, 8.4 mmol) was dissolved in ethanol (20 mL), this was then added to the reaction. The reaction was then placed under vacuum and purged with nitrogen three more times. It was then placed under vacuum again then purged with hydrogen, this was repeated once more leaving the balloon filled with hydrogen. The reaction was left at room temperature overnight under a hydrogen atmosphere. The reaction solution was then filtered through a celite plug washing with ethanol. The filtrates were combined and solvent removed to give a clear colourless oil. MS: 208; ¹H NMR (400 MHz, CDCl₃): 2.22 (6H, s), 2.27 (2H, t, *J* 5.9 Hz), 3.45 (2H, q, *J* 11.6, 5.3 Hz), 6.71 (1H, ddd, *J* 7.9, 2.4, 1.0 Hz), 6.85 (1H, bs), 7.0-7.15 (3H, m)

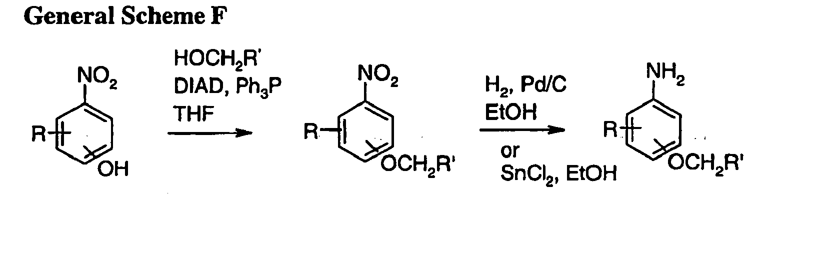

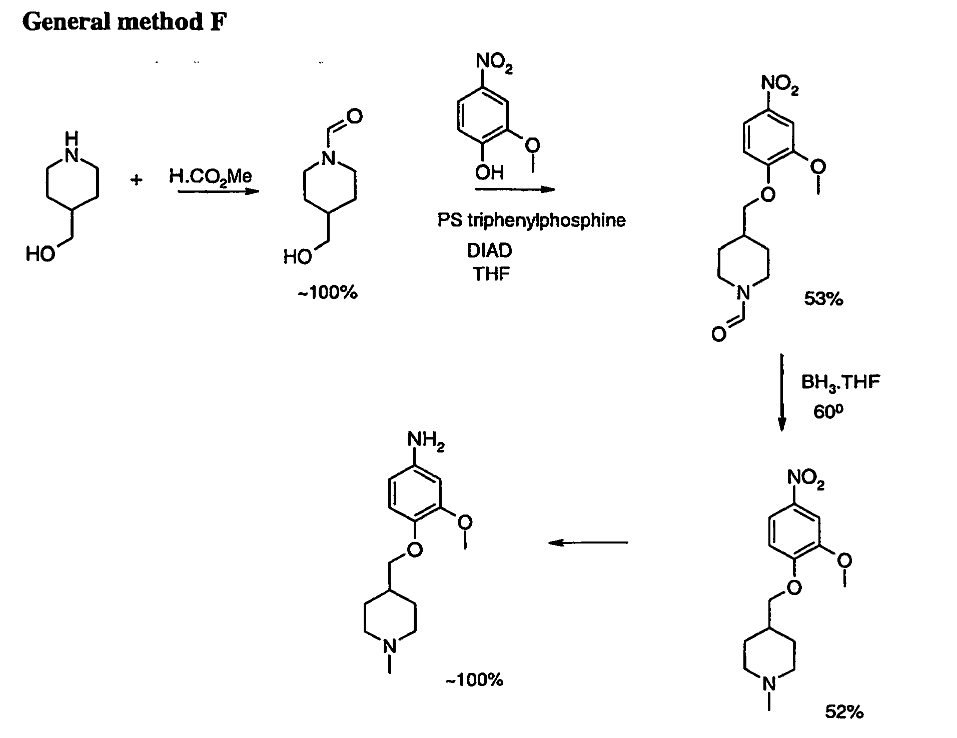

### N-Formyl-4-piperidinemethanol

4-Piperidinemethanol (10 g, 87 mmol) was dissolved in methyl formate (7 mL, 113 mmol) 0 °C, and maintained at that temperature for 30 min, then allowed to reach 20 °C and stirred 90 min. Solid sodium hydroxide was added (0.87 g, pellets) and the mixture was left overnight. Dichloromethane was added, the NaOH removed by filtration and the solution treated with 1M HCl in ether (10 mL). The mixture was filtered through Celite and the solvent was removed under reduced pressure to afford the crude title compound. ¹H NMR (400 MHz, CDCl₃): 0.85-1.1 (2H, m); 1.55-1.85 (3H, m); 2.5-2.7 (1H, m); 2.95-3.1 (1H, m); 3.3 (2H, d, J 7 Hz); 3.6-3.7 (1H, m); 4.1-4.3 (1H, m); 8 (1H, s)

### N-Formyl-4-(2-methoxy-4-nitrophenoxymethyl)piperidine

4-Nitroguaiacol (2 g, 11.8 mmol), N-formyl 4-piperidinemethanol (1.13 g, 7.89 mmol) and polymer-supported triphenylphosphine (3 mmol/g, 3.94 g, 11.8 mmol) were dissolved in tetrahydrofuran (30 mL). The mixture was cooled to 0 °C and diisopropyl azodicarboxylate (2.33 mL, 11.8 mmol) was added dropwise. The mixure was stirred at 0 °C for 30 min then at 20 °C overnight. The resin was filtered off, washed with dichloromethane then methanol and the filtrate evaporated to give a deep orange oil. The oil was taken up in dichloromethane, washed with 2M NaOH, 2M HCl then brine, dried and evaporated giving a pale brown oil. This was taken up in 50:50 ethyl acetate : hexane, filtered through celite, filtrate evaporated, taken up in ethyl acetate and washed further with 1M NaOH. The organic layer was separated, dried over Na₂SO₄, the solvent removed under reduced pressure and the residue columned in 50:50 ethyl acetate : hexane to remove impurities. The product was then eluted with 9:1 dichloromethane : methanol to give a yellow oil, which crystallised on cooling. ¹H NMR (400 MHz, CDCl₃): 1.15-1.3 (2H, m); 1.85-1.9 (1H, m); 2.6-2.7 (1H, m); 3-3.1 (1H, m); 3.7-3.8 (1H, m); 4.0 (2H, d, *J*7 Hz); 4.15-4.25 (1H, m); 7.2 (1H, d, *J* 8 Hz); 7.75 (1H, d, *J*2 Hz); 7.9 (1H, dd, *J*2 and 8 Hz); 8 (1H, s)

### N-Methyl-4-(2-methoxy-4-nitrophenoxymethyl)piperidine

A suspension of *N*-formyl-4-(2-methoxy-4-nitrophenoxymethyl)piperidine (1.24 g, 4.2 mmol) in tetrahydrofuran (5 mL) under nitrogen was stirred while adding the borane solution (8.4 mL of a 1M soln in THF) then heated to 60 °C for 2 h. Further borane solution (to a total of 5 equivalents) and 20 mL tetrahydrofuran (20 mL) were added and the mixture was heated overnight. The mixture was cooled, methanol (25 mL) was added carefully followed by dichloromethane. The mixture was then washed with brine, 2M NaOH, dried over Na₂SO₄ and solvent evaporated. The residue was dissolved in methanol, a few drops of acetic acid added and the mixture was heated under reflux for 3 days. Evaporation of the solvent and chromatography in 9:1 dichloromethane : methanol containing 1% triethylamine afforded the product as a brown solid. ¹H NMR (400 MHz, dmso-*d₆*): 1.4-1.5 (2H, m); 1.85-2 (3H, m); 2-2.1 (2H, m); 2.8-3 (2H, m); 4.05 (3H, s); 4.15 (2H, d, *J* 7 Hz); 7.35 (1H, d, *J* 8 Hz); 7.9 (1H, d, *J* 2 Hz); 8.05 (1H, dd, *J* 2 and 8 Hz)

### N-Methyl-4-(2-methoxy-4-nitrophenoxymethyl)piperidine

Catalytic reduction over Pd (10%C) in EtOH gave the aniline as a red-brown solid. ¹H NMR (400 MHz, CDCl₃): 1.3-1.5 (2H, m); 1.7-1.9 (3H, m); 2-2.1 (2H, m); 2.9-3 (2H, m); 3.4 (2H, broad s); 3.7 (2H, d, *J* 7 Hz); 3.75 (3H, s); 6.15 (1H, dd, *J* 1 and 7 Hz); 6.25 (1H, d, *J* 1 Hz); 6.65 (1H, d, *J* 7 Hz)

Further examples of analines include the following (NMR spectra at 400 MHz, in CDCl₃ unless otherwise stated):

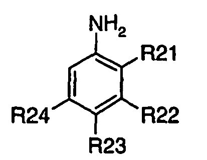

| **Ex.** | **Method** | **R21** | **R22** | **R23** | **R24** | **MS** | **NMR** |
|---|---|---|---|---|---|---|---|
| 1 | A (chloro-alkyl phenol displace ment) | H | H | 3-(di-methyl-amino)- ethoxy | H | 181 | 2.25 (6H, s); 2.65 (2H, t, *J* 7 Hz); 3.9 (2H, t, *J* 7 Hz); 6.5- 7 (2H, m); 6.65-6.75 (2H, m) |
| 2 | A (chloro-alkyl phenol displace ment) | H | H | 3-(di-methyl-amino)- propoxy | | | See specific example |
| 3 | A (chloro-alkyl phenol displace ment) | H | OCH₃ | 2-((4- CH₃)piperazin-1- yl)ethoxy | OCH₃ | 296 | 2.25 (3H, s); 2.4-2.7 (8H, m); 2.75 (2H, t, *J* 7 Hz); 3.7 (6H, s); 3.9 (2H, t, *J* 7 Hz); 5.9 (2H, s) |
| 4 | A (chloro-alkyl phenol displace ment) | H | OCH₃ | 3-((4- CH₃)piperazin-1- yl)propoxy | OCH₃ | 310 | 1.8-1.9 (2H, m); 2.2 (3H, s); 2.3-2.6 (10 H, m); 3.7 (6H, s); 3.85 (2H, t, *J* 7 Hz); 5.9 (2H, s) |
| 5 | A (chloro-alkyl phenol displace ment) | H | OCH₃ | 2-((4- CH₃)piperazin-1- yl)ethoxy | H | 266 | 2.35 (3H, s); 2.55-2.8 (10H, m); 3.7 (3H, s); 4 (2H, t, *J* 7Hz); 6.1 (1H, dd, *J* 2 and 8 Hz); 6.2 (1H, Hz); 6.2 (1H, d, *J* 2 Hz); 7.7 (1H, d, *J* 8 Hz) |
| 6 | A (chloro-alkyl phenol displace ment) | H | OCH₃ | 3-((4-CH₃)piperazin-1-yl)prop-oxy | H | 280 | 1.9-2.1 (2H, m); 2.35 (3H, s); 2.4-2.6 (10H, m); 3.8 (3H, s); 4 (2H, *J* 7 Hz); 6.2 (1H, dd, *J* 2 and 8 Hz); 6.3 (1H, d, *J* 2 Hz); 6.8 (1H, d, *J* 8 Hz) |
| 7 | A (chloroal kyl phenol displacement) | H | OCH₃ | OCH₃ | 2-((4-CH₃)piperazin-1-yl)ethoxy | 296 | 2.2 (3H, s); 2.3-2.5 (4H, m); 2.5-2.7 (4H, m); 2.8 (2H, t, *J* 7Hz); 3.65 (3H, s); 3.75 (3H, s); 4 (2H, t, *J* 7 Hz); 5.8-5.85 (2H, m) |
| 8 | A (chloro-alkyl phenol displacement) | H | OCH₃ | OCH₃ | 3-((4-CH₃)piperazin-1-yl)propoxy | .310 | 1.85-1.95 (2H, m); 2.2 (3H, s); 2.3-2.5 (10H, m); 3.65 (3H, s); 3.75 (3H, s); 3.95 (2H, t, *J* 7Hz); 6.85-6.9 (2H, m) |
| 9 | A (chloro-alkyl phenol displacement) | H | OCH₃ | 2-(piperidino)- ethoxy | OCH₃ | 281 | 1.3-1.4 (2H, m); 1.5-1.6 (4H, m); 2.45- 2.6 (4H, m); 2.75 (2H, t, *J* 7 Hz); 3.65 (6H, s); 3.95 (2H, t, *J* 7 Hz); 5.85 (2H, s) |
| 10 | A (phenol alkylation) | H | H | 2-(morpholino)ethoxy | H | 223 | 2.45-2.55 (4H, m); 2.7 (2H, t, *J* 7 Hz); 3.65-3.7 (4H, m); 3.95 (2H, t, *J* 7 Hz); 6.5-6.6 (2H, m); 6.65-6.7 (2H, m) |
| 11 | A (chloro-alkyl phenol displace ment) | H | OCH₃ | 2-(morpholino)ethoxy | OCH₃ | 283 | 2.5-2.55 (4H, m); 2.7 (2H, t, *J* 7 Hz); 3.6-3.7 (4H, m); 3.7 (6H, s); 4.95 (2H, t, *J* 7 Hz); 5.8 (2H, s) |
| 12 | A (chloro-alkyl phenol displace ment) | H | H | (S)-((1-CH₃)pyrr olidin-2-yl)methoxy | H | 207 | (dmso-*d*₆) 1.5-1.6 (1H, m); 1.6-1.65 (2H, m); 1.9-2 (1H, m); 2.15-2.25 (1H, m); 2.35 (3H, s); 2.5-2.6 (1H, m); 2.9-3 (1H, m); 4.65-4.7 (1H, m); 4.7-4.75 (1H, m); 6.45-6.5 (2H, m); 6.6-6.65 (2H, m) |
| 13 | A (chloro-alkyl phenol displace ment) | H | F | 3-((4-CH₃)piperazin-1-yl)propoxy | H | 268 | 1.8-1.9 (2H, m); 2.2 (3H, s); 2.3-2.55 (10H, m); 3.9 (2H, t, *J* 7 Hz); 6.3 (1H, m); 6.4 (1H, m); 6.7 (1H, m) |
| 14 | A (chloro-alkyl phenol displacement) | H | F | 3-(piperidino)propoxy | H | 253 | 1.3-1.4(2H, m); 1.4-1.5 (4H, m); 1.7-1.8 (2H, m); 2.25-2.4 (6H, m); 3.9 (2H, t, *J* 7 Hz); 6.25-6.3 (1H, m); 6.35-6.4 (1H, m); 6.75-6.85 (1H, m) |
| 15 | A (chloro-alkyl phenol displace meat) | H | F | 3-(di-ethylamino)-propoxy | H | 241 | 1.05(6H, t, *J* 7 Hz); 1.9-2 (2H, m); 2.5-2.7 (6H, m); 4 (2H, t, *J* 7 Hz); 6.35-6.4 (1H, m); 6.4-6.45 m);6.4-6.45 (1H, m); 6.8-6.9 (1H, m) |
| 16 | A (chloro-alkyl phenol displacement) | H | F | 2-((4-CH₃)-piperazin-1-yl)ethoxy | H | 254 | 2.2 (3H, s); 2.3-2.4 (4H, m); 2.4-2.65 (4H, m); 2.75 (4H, m); 2.75 (2H, t, *J* 7 Hz); 4 (2H, t, *J* 7 Hz); 6.25-6.3 (1H, m); 6.3-6.35 (1H, m); 6.75-6.85 (1H, m) |
| 17 | A (chloroal kyl phenol displace ment) | H | 3-(piperidino)pro poxy | H | H | 235 | 1.3-1.4(2H, m); 1.45-155 (4H, m): 2.3-2.5 (6H, m); 3.9 (2H, t, *J* 7 Hz); 6.1-6.3 (3H, m); 6.9-7 (1H, m) |
| 18 | A (chloroal kyl phenol displace ment) | H | 3-((4-CH₃)-piperazin -1-yl)propoxy | H | H | 250 | 1.9-2(2H,m); 2.3 (3H, s); 2.4-2.7(10H, m);4(2H.m); 6.2-6.4 (3H, m); 7-7.1 (1H, m) |
| 19 | F (Mitsunobu) | H | OCH₃ | (R)-(pyrrolidi n-2-yl)-methoxy | 323, 223 (as *N*-Boc protected) | | (as *N*-Boc protected); (dmso-d*₆*): 1.4 (9H, broad s); 1.7-1.8 (2H, m); 1.8-2 (3H, m); 3.2-3.25 (2H, m); 3.65 (3H, s); 3.75-3.85 (2H,m); , 4.7-4.8 (2H, broad s); 6 (1H, dd, *J* 2 and 8 Hz); 6.25 (1H, d, *J* 2 Hz); 6.65 (1H, d, *J* 8 Hz) |
| 20 | A (chloro-alkyl phenol displacement) | H | Cl | 2-(piperidino)eth oxy | H | 255 257 | 1.3-1.4 (2H, m); 1.5-1.6 (4H, m); 2.4-2.6 (4H, m); 2.8 (2H, t, *J* 7 Hz); 4.1 (2H, t, *J* 7 Hz); 6.55 (1H, dd, *J* 2 and 8 Hz); 7.7 (1H,d,*J* 2 Hz); 7.8 (1H, d, *J* 8 Hz) |
| 21 | A (chloro-alkyl phenol displace ment) | H | F | 2-((4-*iso-*propyl)pi perazin-1-yl)-ethoxy | H | 282 | 1.05 (6H, d, *J* 7 Hz); 2.5-2.7 (9H, m); 2.8 (2H, t, *J* 7 Hz); 4.1 (2H, t, *J* 7 Hz); 6.35-6.4 (1H, m); 6.45 (1H, m); 6.8-6.9 (1H, m) |
| 22 | A (chloro-alkyl phenol displacement) | H | OCH₃ | 2-((4-*iso*-propyl)pi perazin-1-yl)-ethoxy | H | 294 | 1.05 (6H, d, *J* 7 Hz); 2.5-2.7 (9H, m); 2.8 (2H, t, *J* 7 Hz);3.8 (3H, s); 4.1(2H,t, *J* 7 Hz); 6.2 (1H, d, *J* 2 and 8 Hz); 6.3 (1H, d, *J* 2 Hz); 6.75 (1H, d, *J* 8 Hz) |
| 23 | A (chloro-alkyl phenol displace ment) | H | OCH₃ | 3-((4-isopropyl)-piperazin -1-yl)propoxy | H | 308 | 1.05 (6H, d, *J* 7 Hz); 1.9-2 (2H, m); 2.5-2.7 (11H, m); 3.4 (2H, broad s); 3.8 (3H, s); 4 (2H, t, *J* 7 Hz); 6.2 (1H, d, *J* 2 and 8 Hz); 6.3 (1H, d, *J* 2 Hz); 6.75 (1H, d, *J* 8 Hz) |
| 24 | A (chloro-alkyl phenol displacement) | CH₃ | H | 3-((4-CH₃)piperazin-1-yl)propoxy | H | 264 | 1.9-2 (2H, m); 2.1 (3H, s); 2.35 (3H, s); 2.4-2.7 (10H, m); 4 (2H, t, *J* 7 Hz); 6.25-6.4 (2H, m); 6.4(2H,m); 6.9-7 (1H, m) |
| 25 | A (chloro-alkyl phenol displace ment) | CH₃ | H | 3-(piperidino)pro poxy | H | 249 | 1.4-1.55 (2H, m); 1.6-1.7 (4H, m); 1.95-2.05 (2H, m); 2.1 (3H, s); 2.4-2.6 (6H, m); 4 (2H, t, *J* 7 Hz); 6.3-6.4 (2H, m); 6.9-7 (1H, m) |
| 26 | F(Mitsunobu) | H | OCH₃ | ((1-CH₃)-piperidin -4-yl)-methoxy | H | 251 | See specific example |
| 27 | F (Mitsunobu) | H | OCH₃ | 2-((1-CH₃)pipe ridin-4-yl)ethoxy | H | 265 | 1.2-1.35 (2H, m); 1.4-1.55 (1H, m); 1.65-1.75 (4H, m); 1.85-1.95 (2H, m); 2.2 (3H, s); 2.8-2.9 (2H, m); 3.7 (3H, s); 3.9 (2H, t, *J* 7 Hz); 6.15 (1H, dd, *J* 2 and 8 Hz); 6.2(1H, d, *J* 2 Hz); 6.65 (1H, d, *J* 8 Hz) |
| 28 | F (Mitsunobu) | H | H | 2-((1-CH₃)pipe ridin-4-yl)ethoxy | H | 235 | 1.2-1.35 (2H, m); 1.4-1.55 (1H, m); 1.6-1.7 (4H, m); 1.85-1.95 (2H, m); 2.2 (3H, s); 2.8-2.9 (2H, m); 3.8-3.9 (2H, m); 6.5-6.6 (2H, m); 6.7-6.8 (2H, m) |
| 29 | F (Mitsunobu) | H | H | (*S*)-(pyrrolidin-2-yl)methoxy | H | 293 193 | (as N-Boc protected); (dmso-*d*₆): 1.15 (9H, s); 1.4-1.7 (4H, m); 3-3.05 (2H, m); 3.4-3.45 (1H, m); 3.5-3.55 (2H, m); 4.4 (2H, broad s); 6.2-6.3 (2H, m); 6.4-6.5 (2H, m) |
| 30 | B (Halide displace ment via alkoxy anion) | H | OCH₃ | 2*-*(*iso-*propylam ino)ethoxy | H | | (as *N*-Boc protected) 1.15 (6H, d, *J* 7Hz);1.45 7 Hz); 1.45 (9H, s); 3.35-3.5 (2H, m); 3.8 (3H, s); 3.9-4.1 (2H, m); 4.3-4.45 (1H, m); 6.2 (1H, dd, *J* 2 and 8 Hz); 6.3 (1H, d, *J* 2 Hz); 6.8 (1H, |
| 31 | B (Halide displace ment via alkoxy anion) | H | Cl | 2-(*iso-* propyl-amino)-ethoxy | H | | (as *N*-Boc protected) 1.15 (6H, broad d, *J* 7 Hz); 1.45 (9H, s); 3.35-3.5 (2H, m); 3.9-4.1 (2H, m); 4.3-4.45 (1H, m); 6.5 (1H, dd, *J* 2 and 8 Hz); 6.7 (1H, d, *J* 2 Hz); 6.8 (1H, d, J 8 Hz) |
| 32 | C (Halide displace ment) | H | OCH₃ | (4-CH₃)-piperazin -1-yl | H | 222 | 2.4 (3H, s); 2.5-2.7 (4H, m); 2.9-3.1 (4H, m); 3.8 (3H, s); 6.2-6.4 (2H, m); 6.8-6.9 (1H, m) |
| 33 | C (Halide displace ment) | H | OCH₃ | *4-(tert-* butoxyca rbonyl)pi perazin-1-yl | H | | (as *N*-Boc protected) 1.4 (9H, s); 2.8-2.9 (4H, m); 35-3.6 94H, m); 3.75 (3H, s); 6.1-6.25 (2H, m); 6.65-6.8 (1H, m) |
| 34 | C (Halide displace ment) | H | H | 4-(*tert-* butoxy-carbonyl) piperazin -1-yl | H | 278 | (as *N*-Boc protected) 1.4 (9H, s); 2.85-2.95 (4H, m); 3.4 (2H, broad s); 3.45-3.55 (4H, m); 6.6 (2H. d, *J* 8 Hz); 6.75 (2H, d, *J* 8Hz) |
| 35 | C (Halide displace ment) | H | H | 4-(*iso-*propyl)-piperazin -1-yl | H | 220 | 1.0 (6H, d, *J* 7 Hz); 2.55-2.7 (6H, m); 2.95-3.05 (4H, m, 3.35 (2H, broad s); 6.5-6.65 (2H, m); 6.7-6.8 (2H, m) |
| 36 | D (piperazine alkylation) | H | H | 4-(carbamoyl-methyl)piperazin-1-yl | H | 235 | 2.6-2.7 (4H, m); 2.9-3.1 (4H, m); 3.4 (2H, broad s); 5.4 (1H, broad s); 6.55 (2H, d, *J* 8Hz); 66.7 (2H, d, *J* 8Hz); 7 (1H, broad s) |
| 37 | D (piperazine alkylation) | H | H | 4-(cyclohexylmethyl)-piperazin -1-yl 1 | H | 274 | 0.7-0.9 (2H, m); 1.1-1.3 (3H, m); 1.4-1.5 (1H, m); 1.6-1.8 (5H, m); 2.1 (2H, d, *J* 7 Hz); 2.4-2.55 (4H, m); 2.9-3 (4H, m); 3.35 (2H, broad s); 6.5-6.65 (2H, m); 7.8-7.9 (2H, m) |
| 38 | D (piperazine alkylation) | H | H | 4-(((3-Cl)pheny 1)methyl) piperazin -1-yl | H | 302 /30 4 | See specific example |
| 39 | D (piperazine alkylation) | H | H | 4-(((3-cyano)-phenyl)-methyl)-piperazin -1-yl | H | 293 | 2.5-2.6 (4H, m); 2.9-3.1 (4H, m); 3.5 (2H, s); 6.5-6.6 (2H, m); 6.7-6.8 (2H, m); 7.3-7.4 (1H, m); 7.5-7.6 (2H, m); 7.6 (1H, m) |
| 40 | D (piperazine alkylation) | H | H | 4-(((3-OCH₃)-phenyl)-methyl)-piperazi -1-yl | H | | 2.5-2.6 (4H, m); 2.9-3.1 (4H, m); 3.45 (2H, s); 3.75 (3H, s); 6.5-6.6 (2H, m); 6.7-6.8 (3H, m); 6.8-6.9 (2H, m); 7.2-7.3 (1H, m) |
| 41 | C (Halide displace ment) | H | Cl | (4-CH₃)-piperazin -1-yl | H | | 2.35 (3H, s); 2.5-2.7 (4H, m); 2.9-3 (4H, m); 3.5 (2H, broad s); 6.5 (1H, dd, *J* 2 and 8 Hz); 6.7 (1H, d, *J* 2 hz); 6.9(1H, d, *J* 8 Hz) |
| 42 | C (Halide displace ment) | H | OCH₃ | *4-*(*iso-* propyl)- piperazin -1-yl | H | | 1.1 (6H, d, *J* 7 Hz); 2.6-2.7 (6H, m); 2.9-3.1 (4H, m); 3.75 (3H, s); 6.15-6.3 (2H, m); 6.7 (1H, d, *J* 8 Hz) |
| 43 | C (Halide displace ment) | H | F | 4*-(iso-* propyl)-piperazin -1-yl | H | | 1.1 (6H, d, *J* 7 Hz); 2.6-2.7 (6H. m); 2.9-3 (4H, m); 3.5 (H, broad s); 6.3-6.4 (2H, m); 6.7-6.8 (1H, m) |
| 44 | C (Halide displace ment) | H | (4-CH₃)-piperazin -1-yl | H | H | 192 | 2.25 (3H, s); 2.45-2.5 (4H, m); 3.1-3.2 (4H, m); 3.6 (2H, broad s); 6.1 (1H, dd, *J* 2 and 8 Hz); 6.2(1H,m;6.3 (1H, d, *J* 2 and 8 Hz); 6.95 (1H, t, *J* 8 Hz) |
| 45 | C (Halide displace ment) | CH₃ | H | (4-CH₃)-piperazin -1-yl | H | 206 | 2.1 (3H, s); 2.3 (3H, s); 2.5-2.6 (4H, m); 2.9-3.1 (4H, m); 6.5-6.6 (1H, m); 6.6-6.7 (2H, m) |
| 46 | D (piperazine alkylation) | H | H | (4-(2-dimethyl amino-ethyl))-piperazin -1-yl | H | 249 | 2.2 (6H, s); 2.3-2.4 (2H, m); 2.4-2.5 (2H, m); 2.5-2.6 (4H, m); 2.95-3.05 (4H, m); 6.55 (2H, d, *J* 8 Hz); 6.75 (2H, d, *J* 8 Hz) |
| 47 | D (piperazine alkylation) | H | H | (4-((2-methoxy) ethyl))-piperazin-1-yl | H | | 2.5-2.6 (6H, m); 2.95-3.05 (4H, m); 3.3 (3H, s); 3.5 (2H, t, *J*7 Hz); 6.55 (2H, d, *J* 8 Hz); 6.75 (2H, d, *J* 8 Hz) |
| 48 | D (piperazine alkylation) | H | H | (4-(3-dimethyl aminopro pyl))pipe razin-1-yl | H | | See specific example |
| 49 | E (amide formation) | H | H | (*N*-(2- diethyl-amino)-ethyl)-(*N*-methyl))carbamoyl | H | 250 | dmso-*d₆*: 0.8-1.0 (6H, m); 2.3-2.6 (6H, m); 3.0 (3H, broad s); 3.35-3.5 (2H, m); 5.45 (2H, broad s); 6.5 (2H, d, *J* 8 Hz); 7.1 (2H, d, *J* 8 Hz) |
| 50 | E (amide formation) | H | (*N-*(2-dimethyl amino)-ethyl)car bamoyl | H | H | 208 | See specific example |
| 51 | E (amide form-ation) | H | *(N*-(2- diethylamino)-ethyl)-(*N*-methyl))carbyl))carbamoyl | H | H | 250 | dmso-*d₆*: 0.8-1.0 (6H, 2 broad m); 2.2-2.8 (4H, 2 broad m); 2.9-3.0 (3H, 2 broad s); 3.2-3.5 (2H, 2 broad m); 5.2 (2H, broad s); 6.4 (1H, d, *J* 8 Hz); 6.5 (1H, d, *J* 2 Hz); 6.6 (1H, dd, *J* 2 and 8 Hz); 7.05 (1H, m) |

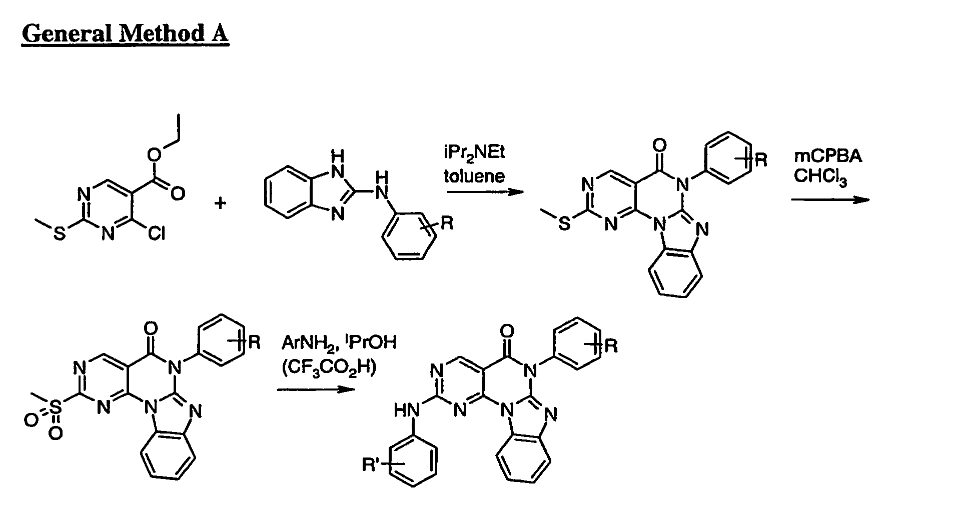

### 2-(2,6-dimethylphenylamino)benzimidazole hydrochloride

2-Chlorobenzimidazole (28.2 g, 185 mmol) were refluxed with 2,6-dimethylaniline (278 mmol, 34 mL) for 24 h. The mixture was cooled and the solvent removed under reduced pressure to give an oily residue which was triturated from ethyl acetate (ca 100 mL) to give an off-white solid (as the HCl salt). ¹H NMR (400 MHz, D₂O): 2.13 (6H, s); 7.15-7.3 (7H, m); MS: 238 [MH+].

### 6-(2,6-Dimethylphenyl)-2-methylthio-benzo[4,5]imidazolo[1,2-a]pyrimido[5,6-e]pyrimidin-5-one

A mixture of ethyl 4-chloro-2-methylthiopyrimidine-5-carboxylate (12.5 g, 54 mmol), 2-(2,6-dimethylphenylamino)benzimidazole hydrochloride (13.5 g, 49 mmol) and Hunig's base (17.6 mL, 102 mmol) in toluene (30 mL) were refluxed for 3 days. The solvent was removed under reduced pressure and the residue taken into dichloromethane (*ca* 200 mL - not completely soluble) and washed with HCl 2N twice (*ca* 70 mL each time) then brine twice (*ca* 70 mL each time). The residue was then dissolved completely by heating, then concentrated to *ca* 100 mL. The product was collected by filtration and washed three times with little dichloromethane to give a white amorphous solid.¹H NMR (400 MHz, D₂O): 2.3 (6H, s); 3.0 (3H, s); 7.2-7.3 (2H, m); 7.5-7.7 (3H, m); 7.8 (1H, m); 8.75 (1H, m); 9.5 (1H, s); MS: 388[MH+]

### 6 -(2,6-Dimethylphenyl)-2-methanesulphonyl-benzo[4,5]imidazolo[1,2-a]pyrimido[5,4-e]pyrimidin-5-one

6-(2,6-Dimethylphenyl)-2-methylthio-benzo[4,5]imidazolo[1,2-a]pyrimido [5,6e]-pyrimidin-5-one (2 g, 5.16 mmol) were suspended in chloroform (50 mL) and a solution of *m*-chloroperoxybenzoic acid (2.79 g, 11.3 mmol assuming 70% purity) in 50 mL chloroform pre-dried over MgSO₄ prior to addition to the sulphide) was added dropwise. The mixture was stirred for 15 h, then 1.5 mL of dimethylsulphoxide were added and the mixture stirred for further 1 h. Water (10 mL) was added and the layers separated. The chloroform layer was washed again with water (10 mL) and then with saturated NaHCO₃ (twice, 20 mL each time) and finally with brine. The solvent was removed under reduced pressure to give crude material as a yellow solid. ¹H NMR (400 MHz, D₂O): 2.1 (6H, s); 3.5 (3H, s); 7.2-7.5 (5H, m); 7.6 (1H, m); 8.6 (1H, m); 9.6 (1H, s); MS: 420 [MH+]

### 6-(2,6-Dimethylphenyl)-2-[4-(3-dimethylaminopropoxy))phenylamino]-benzo[4,5]imidazolo[1,2-a]pyrimido[5,4-e]pyrimidin-5-one

The sulfone (1.0 g, 2.4 mmol) was weighed out in a sealed tube, followed by the aniline (0.47 g, 2.6 mmol). Isopropanol (30 mL) was added to the other components in the sealed tube. Trifluoroacetic acid (0.60 g, 4.8 mmol) was weighed out into a glass vial and transferred to the sealed tube. The vial was washed with *iso*propanol (5 mL), which was also added to the above mixture. The tube was flushed with nitrogen for 2 min, fitted with a screwcap lid and heated at 85 °C for 15 h. The reaction mixture was allowed to cool to room temperature, the isopropanol evaporated and the crude material, in form of a green solid, taken up in dichloromethane (30 mL). The dichloromethane solution was washed with aqueous sodium hydroxide solution (10 mL, 1M) and the basic aqueous solution extracted with dichloromethane (3x30 mL). The combined organic layers were dried over magnesium sulphate, filtered and evaporated to give a yellow solid. Purification was achieved by elution on silica from a dichloromethane and methanol solution (95:5). The expected compound was isolated as a yellow solid. ¹H-NMR (400 MHz, CDCl₃) 9.23 (1H, s), 8.32 (1H, m), 7.71 (1H, m), 7.65 (1H, m), 7.55 (1H, m), 7.52 (1H, m), 7.34 (2H, m), 7.25 (2H, m), 7.04 (2H, m), 4.11 (2H, broad m), 2.53 (2H, broad m), 2.31 (6H, s), 2.14 (6H, s), 2.04 (2H, broad m).

### 6-(2,6-Dimethylphenyl)-2-[4-(4-methylpiperazino)phenylamino]-benzo[4,5]-imidazolo[1,2-a]pyrimido[5,4-e]pyrimidin-5-one

The sulfone (3.8 g, 9.0 mmol) was weighed out in a sealed tube, followed by the aniline (1.8 g, 10.0 mmol). Isopropanol (75 mL) was then added to the other components and the tube was flushed with nitrogen for 2 min, fitted with a screwcap lid and heated at 85 °C for 15 h. The reaction mixture was allowed to cool to room temperature, the isopropanol evaporated and the crude material, in form of a dark-green oil, taken up in dichloromethane (30 mL). The dichloromethane solution was washed with aqueous sodium hydroxide solution (15 mL, 1M) and the basic aqueous solution extracted with dichloromethane (3x40 mL). The combined organic layers were dried over magnesium sulphate, filtered and evaporated to give dark-green oil. Purification was achieved by elution on silica from a dichloromethane and methanol solution (9:1). The title compound was isolated as a yellow solid. ¹H-NMR (400 MHz, CDCl₃) 9.22 (1H, s), 8.35 (1H, m), 7.78 (1H, m), 7.63 (1H, m), 7.56 (1H, m), 7.53 (1H, m), 7.35 (2H, m), 7.25 (2H, m), 7.06 (2H, m), 3.31 (4H, broad m), 2.63 (4H, broad m), 2.40 (3H, s), 2.15 (6H, s).

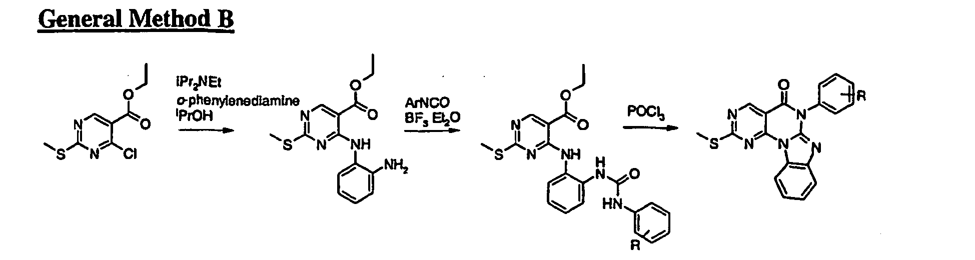

### Ethyl 4-((2-aminophenyl)amino)-2-methylthiopyrimidinecarboxylate

Ethyl 4-chloro-2-methylthiopyrimidinecarboxylate (10.0 g, 0.043 mol) and phenylenediamine (5.6 g, 0.051 mol) were placed in a dry, 250 mL round-bottomed flask together with Huenig's base (9.2 g, 0.064 mol) and isopropanol (100 mL). The reaction mixture was heated overnight at 50 °C. The isopropanol was evaporated and the residue dissolved in dichloromethane (100 mL). The dichloromethane solution was washed with brine (3x50 mL) and dried over MgSO₄ before evaporation to give a brown-yellow solid. Purification on silica (dichloromethane : MeOH 9:1) afforded the product a yellow solid. ¹H NMR (400 MHz, CDCl₃): 1.3 (3H, q, *J* 7 Hz); 2.3 (3H, s); 3.7 (2H, bs); 4.3 (2H, q, *J* 7 Hz); 6.7-6.8 (2H, m); 7-7.05 (1H, m); 7.4-7.5 (1H, m); 8.7 (1H, s); 9.8 (1H, s)

### Ethyl 4-(2-(3-(2,6-dimethylphenyl)ureido)phenylamino)-2-methylthiopyrimidinecarboxylate

Ethyl 4-(2-aminophenylamino)-2-methylthiopyrimidinecarboxylate (7.0 g, 0.023 mol) was added together with 2,6-dimethylphenyl isocyanate (5.1 g, 0.035 mol) and boron trifluoride etherate (4.9 g, 0.035 mol) to a dry 500 mL round-bottomed flask. Anhydrous THF (200 mL) was added and the mixture heated at 50°C overnight. A dense, white precipitate formed which was collected by filtration under reduced pressure washing with hexane.

### 6 -(2,6-Dimethylphenyl)-2-methylthiobenzo[4,5]imidazolo[1,2-a]pyrimido[5,6-e]pyrimidin-5-one

Ethyl 4-(2-(3-(2,6-dimethylphenyl)ureido)phenylamino)-2-methylthiopyrimidine-carboxylate (9.5 g, 0.021 mol) was added to a dry 250 mL round-bottomed flask together with phosphorous oxychloride (60 mL). The resulting slurry was heated to 100°C and a dark-orange solution formed. The temperature was lowered to 80 °C and the reaction left stirring overnight, then worked up as follows: the reaction vessel was put into an ice bath and a mixture of water/ice was added slowly, under vigorous stirring. When the heat evolution ceased, the resulting acidic solution was allowed to cool to room temperature before extraction with dichloromethane (3x75 mL). The dichloromethane layer was washed with brine (1x30 mL), dried over MgSO₄ and evaporated under reduced pressure. The solid obtained was washed with a cold mixture of dichloromethane and hexane (3:7, ~2x10 mL) on a Buechner funnel.

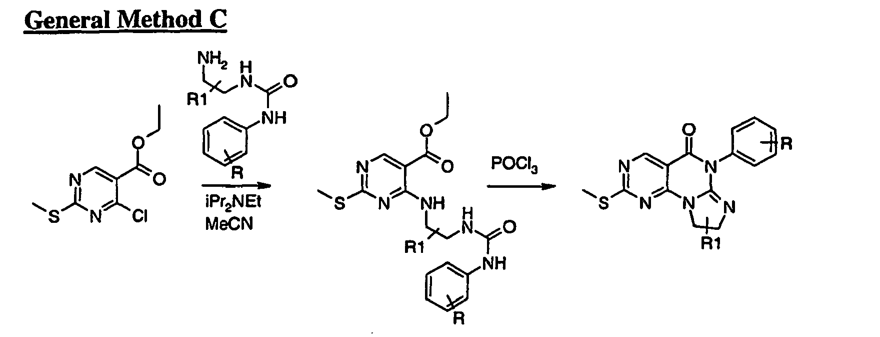

### N-(2-Aminoethyl)-N'-phenylurea

A solution of ethylenediamine (15 mL, 13.49 g) in *tert*-butyl methyl ether (100 mL) was stirred under nitrogen at -70 °C while adding dropwise, over 15 min, a solution of 2,6-dimethylphenyl isocyanate (4.22 g, 0.0288 mol) in *tert*-butyl methyl ether (60 mL). After stirring for 2 h at 20 °C the colourless solid was collected rapidly and washed well with *tert*-butyl methyl ether. The solid was stirred with 1M hydrochloric acid (100 mL) for 30 min, filtered and the solid washed with water. The solid was discarded. The filtrate was evaporated, the residue boiled with isopropanol then evaporated again to give the hydrochloride salt of the product.

### N-(2-(5-Ethoxycarbonyl-2-methylthiopyrimidin-4-yl)aminoethyl)-N'-(2,6-dimethylphenyl)urea

*N*-(2-Aminoethyl)-*N*'-phenylurea (1.22 g, 5 mmol), ethyl 4-chloro-2-methylthiopyrimidine-5-carboxylate (1.16 g, 5 mmol), diisopropylethylamine (2.58 g, 20 mmol) and acetonitrile (40 mL) were stirred and heated in a sealed tube at 80 °C after thorough shaking. After 30 min the solid was collected, washed well with acetonitrile and dried in air giving colourless solid. ¹H NMR (DMSO) 8.68 (s, 1H) 8.51 (br. s, 1H) 7.62 (s, 1H) 7.13 (m, 3H) 6.29 (br.s, 1H) 4.40 (q, J = 7.4, 2H) 3.72 (m, 2H) 2.61 (s, 3H) 2.24 (s, 6H) 1.42 (t, J = 7.4, 3H).

### 6-(2,6-Dimethylphenyl)-2-methylthio-8,9-dihydroimidazolo[1,2-a]pyrimido-[5,6-e]pyrimidin-5-one

*N*-(2-(5-Ethoxycarbonyl-2-methylthiopyrimidin-4-yl)aminoethyl)-*N*'-(2,6-dimethylphenyl)urea (2.37 g, 5.87 mmol) was stirred and heated with phosphoryl chloride (40 mL) under reflux overnight. The product ws evaporated and the colourless, gummy residue shaken with dichloromethane (100 mL) and saturated sodium hydrogen carbonate solution (100 mL) until no further gas evolution. The organic layer on standing deposited solid which was collected and washed twice with 5 mL portions of dichloromethane. The solid starting material was discarded and the filtrate evaporated giving product as white solid. ¹H NMR (CDCl₃) 8.84 (s, 1H) 7.26 (m, 1H) 7.17 (m, 2H) 4.23 (t, J = 8.6, 2H) 4.01 (t, J = 8.6, 2H) 2.61 (s, 3H) 2.17 (s, 6H).

### 6-(2,6-Dimethylphenyl)-2-methanesulphonyl-8,9-dihydroimidazolo[1,2-a]pyrimido[5,4-e]pyrimidin-5-one

The above compound (46 mg, 0.135 mmol) was suspended in 2 mL chloroform and treated with a dried solution of 3-chloroperoxybenzoic acid (70% - 73 mg, 0.298 mmol) in 2 mL chloroform. After stirring overnight, dimethyl sulphoxide (40 µL) was added, stirred for 1 h and left overnight. The solution was washed twice with 2 mL portions of saturated sodium hydrogen carbonate solution, dried (sodium sulphate) and 80% of the solution evaporated giving a yellow glass used for the next stage. ¹H NMR (CDCl₃) 9.11 (s, 1H) 7.28 (m, 1H) 7.19 (m, 2H) 4.32 (t, J = 8.8, 2H) 4.07 (t, J = 8.8, 2H) 3.39 (s, 3H) 2.18 (s, 6H).

### 6-(2,6-Dimethylphenyl)-2-[4-(4-methylpiperazino)phenylamino]-8,9-dihydroimidazolo[1,2-a]pyrimido[5,4-e]pyrimidin-5-one

The above compound (0.108 mmol) was treated with a solution of 1-(4-aminophenyl)-4-methylpiperazine (21 mg, 0.148 mmol) in 3 mL isopropanoland heated at 80° for 1 h. The solution was evaporated, dissolved in dichloromethane and chromatographed on 6 mL silica, previously washed with a 5% solution of triethylamine in dichloromethane and eluting with 4% methanol / 96% dichloromethane. The product was obtained as greenish yellow gum. ¹H NMR (CDCl₃) 8.80 (s,1H) 7.53 (m 3H) 7.24 (m, 1H) 7.16 (m, 2H) 6.95 (m, 1H) 4.20 (m, 2H) 3.98 (m, 2H) 3.23 (m, 3H) 2.65 (m, 5H) 2.33 (m, 4H) 2.18 (s, 6H).

The following compounds were prepared using the above carboxamide synthesis:

| **No.** | **Name** | **Rt** | **Purity** | **Mass found** |
|---|---|---|---|---|
| 1 | 6-(2,6-dimethylphenyl)-2-((4-(4-methyl-1-piperazinyl)phenyl)amino)pyrimido[5',4':5,6]-pyrimido[1,2-a]benzimidazol-5(6H)-one | 1.21 | 100 | 286,531, 266 |
| 2 | 6-(2,6-dimethylphenyl)-2-((4-(1-piperazinyl)phenyl)-amino)pyrimido[5',4':5,6]pyrimido[1,2-a] benzimidazol-5(6H)-one | 1.18 | 100 | 279,517, 259 |
| 3 | 1,1-dimethylethyl 4-(4-((6-(2,6-dimethylphenyl)-5-oxo-5,6-dihydropyrimido[5',4':5,6]pyrimido[1,2-a]-benzimidazol-2-yl)amino)phenyl)-1-piperazinecarboxylate | 1.57 | 89 | 617,279, 517 |
| 4 | 6-(2,6-dimethylphenyl)-2-((3-(methyloxy)-4-((2-(1-methyl-4-piperidinyl)ethyl)oxy)phenyl)amino)-pyrimido[5',4':5,6]pyrimido[1,2-a]benzimidazol-5(6H)-one | 1.26 | 90 | 323,302, 304 |
| 5 | 6-(2,6-dimethylphenyl)-2-((3-fluoro-4-((3-(1-piperidinyl)propyl)oxy)phenyl)amino)-pyrimido[5',4':5,6]pyrimido[1,2-a]benzimidazol-5(6H)-one | 1.33 | 100 | 296,592 |
| 6 | 2-((4-((3-(diethylamino)propyl)oxy)-3-fluorophenyl)-amino)-6-(2,6-dimethylphenyl)pyrimido[5',4':5,6]-pyrimido[1,2-a]benzimidazol-5(6H)-one | 1.32 | 100 | 290,580 |
| 7 | 6-(2,6-dimethylphenyl)-2-((3-fluoro-4-((3-(4-methyl-1-piperazinyl)propyl)oxy)phenyl)amino)-pyrimido[5',4':5,6]pyrimido[ 1,2-a]benzimidazol-5(6H)-one | 1.15 | 100 | 304, 607 |
| 8 | 2-((4-((2-(dimethylamino)ethyl)oxy)phenyl)amino)-6-(2,6-dimethylphenyl)pyrimido[5',4':5,6]pyrimido[1,2-a]benzimidazol-5 (6H)-one | 1.19 | 99.6 | 260,520 |
| 9 | 2-((3,5-bis(methyloxy)-4-((3-(4-methyl-1-piperazinyl)propyl)oxy)phenyl)amino)-6-(2,6-dimethylphenyl)pyrimido[5',4':5,6]pyrimido[1,2-a]-benzimidazol-5(6H)-one | 1.14 | 95.5 | 325,649 |
| 10 | 6-(2,6-dimethylphenyl)-2-((3-(methyloxy)-4-((2-(4-methyl-1-piperazinyl)ethyl)oxy)phenyl)amino)-pyrimido[5',4':5,6]pyrimido[1,2-a]benzimidazol-5(6H)-one | 1.11 | 96.1 | 303, 605 |
| 11 | 2-((3,4-bis(methyloxy)-5-((2-(4-methyl-1-piperazinyl)ethyl)oxy)phenyl)amino)-6-(2,6-dimethylphenyl)pyrimido[5',4':5,6]pyrimido[1,2-a]-benzimidazol-5(6H)-one | 1.14 | 88.2 | 318,635 |
| 12 | 6-(2,6-dimethylphenyl)-2-((3-(methyloxy)-4-(((1-methyl-4-piperidinyl)methyl)oxy)phenyl)amino)-pyrimido[5',4':5,6]pyrimido[1,2-a]benzimidazol-5(6H)-one | 1.23 | 96.3 | 295,590 |
| 13 | 6-(2,6-dimethylphenyl)-2-((2-methyl-4-(4-methyl-1-piperazinyl)phenyl)amino)pyrimido[5',4':5,6]-pyrimido[1,2-a]benzimidazol-5(6H)-one | 1.19 | 91.2 | 294,545 |
| 14 | 6-(2,6-dimethylphenyl)-2-((4-(((2S)-2-pyrrolidinylmethyl)oxy)phenyl)amino)-pyrimido[5',4':5,6]pyrimido[1,2-a]benzimidazol-5(6H)-one | 1.23 | 100 | 287,532 |
| 15 | 6-(2,6-dimethylphenyl)-2-((2-methyl-4-((3-(1-piperidinyl)propyl)oxy)phenyl)amino)-pyrimido[5',4':5,6]pyrimido[1,2-a]benzimidazol-5(6H)-one | 1.33 | 100 | 294, 588 |
| 16 | 6-(2,6-dimethylphenyl)-2-((2-methyl-4-((3-(4-methyl-1-piperazinyl)propyl)oxy)phenyl)amino)-pyrimido[5',4':5,6]pyrimido[1,2-a]benzimidazol-5(6H)-one | 1.17 | 100 | 302, 603, 322 |
| 17 | 2-((4-((3-(dimethylamino)propyl)oxy)phenyl)amino)-6-(2,6-dimethylphenyl)pyrimido[5',4':5,6]-pyrimido[1,2-a]benzimidazol-5(6H)-one | 1.23 | 95.6 | 267,534 |
| 18 | 6-(2,6-dimethylphenyl)-2-((3,4,5-tris(methyloxy)-phenyl)amino)pyrimido[5',4':5,6]pyrimido[1,2-a]-benzimidazol-5(6H)-one | 1.5 | 99.3 | 523 |
| 19 | 6-(2,6-dimethylphenyl)-2-((3-(methyloxy)-4-(4-methyl-1-piperazinyl)phenyl)amino)-pyrimido[5',4':5,6]pyrimido[1,2-a]benzimidazol-5(6H)-one | 1.18 | 100 | 301,281, 561 |
| 20 | 6-(2,6-dimethylphenyl)-2-((3-(methyloxy)-4-((3-(4-methyl-1-piperazinyl)propyl)oxy)phenyl)amino)-pyrimido[5',4':5,6]pyrimido[1,2-a]benzimidazol-5(6H)-one | 1.12 | 96.8 | 310,619 |
| 21 | 2-((3,4-bis(methyloxy)-5-((3-(4-methyl-1-piperazinyl)propyl)oxy)phenyl)amino)-6-(2,6-dimethylphenyl)pyrimido[5',4':5,6]pyrimido[1,2-a]-benzimidazol-5(6H)-one | 1.14 | 100 | 325,649, 345 |
| 22 | 2-((3,5-bis(methyloxy)-4-((2-(4-methyl-1-piperazinyl)ethyl)oxy)phenyl)amino)-6-(2,6-dimethylphenyl)pyrimido[5',4':5,6]pyrimido[1,2-a]-benzimidazol-5(6H)-one | 1.12 | 87 | 318,635 |
| 23 | 6-(2,6-dimethylphenyl)-2-((4-(((2R)-2-pyrrolidinylmethyl)oxy)phenyl)amino)-pyrimido[5',4':5,6]pyrimido[1,2-a]benzimidazol-5(6H)-one | 1.21 | 100 | 532, 287 |
| 24 | 6-(2,6-dimethylphenyl)-2-((4-((2-(1-methyl-4-piperidinyl)ethyl)oxy)phenyl)amino)-pyrimido[5',4':5,6]pyrimido[1,2-a]benzimidazol-5(6H)-one | 1.3 | 98.6 | 308,287, 574 |
| 25 | 2-((3,5-bis(methyloxy)-4-((2-(1-piperidinyl)ethyl)-oxy)phenyl)amino)-6-(2,6-dimethylphenyl)-pyrimido[5',4':5,6]pyrimido[1,2-a]benzimidazol-5(6H)-one | 1.31 | 100 | 311,620 |
| 26 | 6-(2,6-dimethylphenyl)-2-((3-fluoro-4-(4-methyl-1-piperazinyl)phenyl)amino)pyrimido[5',4':5,6]-pyrimido[1,2-a]benzimidazol-5(6H)-one | 1.25 | 100 | 295,546, 275 |
| 27 | 6-(2,6-dimethylphenyl)-2-((4-((2-(1-piperidinyl)-ethyl)oxy)phenyl)amino)pyrimido[5',4':5,6]-pyrimido[1,2-a]benzimidazol-5(6H)-one | 1.3 | 89.7 | 280,560 |
| 28 | 6-(2,6-dimethylphenyl)-2-((4-((((2S)-1-methyl-2-pyrrolidinyl)methyl)oxy)phenyl)amino)-pyrimido[5',4':5,6]pyrimido[1,2-a]benzimidazol-5(6H)-one | 1.27 | 94.3 | 273,546 |
| 29 | 6-(2,6-dimethylphenyl)-2-((4-((2-(4-morpholinyl)-ethyl)oxy)phenyl)amino)pyrimido[S',4':5,6]-pyrimido[1,2-a]benzimidazol-5(6H)-one | 1.19 | 96.9 | 562,281 |
| 30 | 2-((3,5-bis(methyloxy)-4-((2-(4-morpholinyl)ethyl)-oxy)phenyl)amino)-6-(2,6-dimethylphenyl)-pyrimido[5',4':5,6]pyrimido[1,2-a]benzimidazol-5(6H)-one | 1.25 | 100 | 311,622 |
| 31 | 6-(2,6-dimethylphenyl)-2-((4-(4-(1-methylethyl)-1-piperazinyl)phenyl)amino)pyrimido[5',4':5,6]-pyrimido[1,2-a]benzimidazol-5(6H)-one | 1.25 | 96.8 | 280,559, 300 |
| 32 | 6-(2,6-dimethylphenyl)-2-((4-((3-(1-piperidinyl)-propyl)oxy)phenyl)amino)pyrimido[5',4':5,6]-pyrimido[1,2-a]benzimidazol-5(6H)-one | 1.31 | 100 | 287,574 |
| 33 | 6-(2,6-dimethylphenyl)-2-((4-((2-(methylamino)-ethyl)oxy)phenyl)amino)pyrimido[5',4':5,6]-pyrimido[1,2-a]benzimidazol-5(6H)-one | 1.19 | 100 | 274,506 |
| 34 | 6-(2,6-dimethylphenyl)-2-((4-((2-(4-methyl-1-piperazinyl)ethyl)oxy)phenyl)amino)-pyrimido[5',4':5,6]pyrimido[1,2-a]benzimidazol-5(6H)-one | 1.11 | 100 | 309,288, 575 |
| 35 | 6-(2,6-dimethylphenyl)-2-((3-fluoro-4-((2-(1-piperidinyl)ethyl)oxy)phenyl)amino)-pyrimido[5',4':5,6]pyrimido[1,2-a]benzimidazol-5(6H)-one | 1.31 | 95.4 | 289,578 |
| 36 | 6-(2,6-dimethylphenyl)-2-((3-((3-(1-piperidinyl)-propyl)oxy)phenyl)amino)pyrimido [5',4': 5,6]-pyrimido[1,2-a]benzimidazol-5(6H)-one | 1.36 | 100 | 287,574 |
| 37 | 6-(2,6-dimethylphenyl)-2-((3-(methyloxy)-4-(1-piperazinyl)phenyl)amino)pyrimido[5',4':5,6]-pyrimido[1,2-a]benzimidazol-5(6H)-one | 1.18 | 98 | 274,547 |
| 38 | 6-(2,6-dimethylphenyl)-2-((3-fluoro-4-((2-(4-methyl-1-piperazinyl)ethyl)oxy)phenyl)amino)-pyrimido[5',4':5,6]pyrimido[1,2-a]benzimidazol-5(6H)-one | 1.15 | 99 | 297,593 |
| 39 | 6-(2,6-dimethylphenyl)-2-((3-((3-(4-methyl-1-piperazinyl)propyl)oxy)phenyl)amino)-pyrimido[5',4':5,6]pyrimido[1,2-a]benzimidazol-5(6H)-one | 1.17 | 95 | 295, 589, 316 |
| 40 | 2-((3-chloro-4-(4-methyl-1-piperazinyl)phenyl)-amino)-6-(2,6-dimethylphenyl)pyrimido[5',4':5,6]-pyrimido[1,2-a]benzimidazol-5(6H)-one | 1.27 | 93.7 | 283,303, 565/567 |
| 41 | 2-((3-chloro-4-(4-(1-methylethyl)-1-piperazinyl)-phenyl)amino)-6-(2,6-dimethylphenyl)-pyrimido[5',4':5,6]pyrimido[1,2-a]benzimidazol-5(6H)-one | 1.33 | 95.7 | 297,593/595 |
| 42 | 2-((4-(4-(cyclohexylmethyl)-1-piperazinyl)phenyl)-anuno)-6-(2,6-dimethylphenyl)pyrimido[5',4':5,6]-pyrimido[1,2-a]benzimidazol-5(6H)-one | 1.4 | 87.5 | 307,613 |
| 43 | 6-(2,6-dimethylphenyl)-2-((4-((2-((1-methylethyl)-amino)ethyl)oxy)-3-(methyloxy)phenyl)amino)-pyrimido[5',4':5,6]pyrimido[1,2-a]benzimidazol-5(6H)-one | 1.24 | 98.3 | 282,564 |
| 44 | 6-(2,6-dimethylphenyl)-2-((3-fluoro-4-(4-(1-methylethyl)-1-piperazinyl)phenyl)amino)-pyrimido[5',4':5,6]pyrimido[1,2-a]benzimidazol-5(6H)-one | 1.3 | 94 | 289,577 |
| 45 | 2-((4-(4-(2-(dimethylamino)ethyl)-1-piperazinyl)-phenyl)amino)-6-(2,6-dimethylphenyl)-pyrimido[5',4':5,6]pyrimido[1,2-a]benzimidazol-5(6H)-one | 1.13 | 100 | 272, 294, 588 |
| 46 | 6-(2,6-dimethylphenyl)-2-((4-(4-(1-methylethyl)-1-piperazinyl)-3-(methyloxy)phenyl)amino)-pyrimido[5',4':5,6]pyrimido[1,2-a]benzimidazol-5(6H)-one | 1.25 | 95.3 | 295,589 |
| 47 | 2-((3-chloro-4-((2-((1-methylethyl)amino)ethyl)oxy)-phenyl)amino)-6-(2,6-dimethylphenyl)-pyrimido[5',4':5,6]pyrimido[1,2-a]benzimidazol-5(6H)-one | 1.29 | 99.1 | 284,568 |
| 48 | 6-(2,6-dimethylphenyl)-2-((3-(4-methyl-1-piperazinyl)phenyl)amino)pyrimido[5',4':5,6]-pyrimido[1,2-a]benzimidazol-5(6H)-one | 1.2 | 92.1 | 286,266, 631 |
| 49 | 2-((4-(4-(3-(dimethylamino)propyl)-1-piperazinyl)-phenyl)amino)-6-(2,6-dimethylphenyl)-pyrimido[5',4':5,6]pyrimido[1,2-a]benzimidazol-5(6H)-one | 1.11 | 100 | 302,602 |
| 50 | 2-(4-(4-((6-(2,6-dimethylphenyl)-5-oxo-5,6-dihydropyrimido[5',4':5,6]pyrimido[1,2-a]-benzimidazol-2-yl)amino)phenyl)-1-piperazinyl)-acetamide | 1.15 | 100 | 287,265, 574 |
| 51 | 2-((4-(4-((3-chlorophenyl)methyl)-1-piperazinyl)-phenyl)amino)-6-(2,6-dimethylphenyl)-pyrimido[5',4':5,6]pyrimido[1,2-a]benzimidazol-5(6H)-one | 1.37 | 90 | 321,641/643 |
| 52 | 6-(2,6-dimethylphenyl)-2-((4-(4-((3-(methyloxy)-phenyl)methyl)-1-piperazinyl)phenyl)amino)-pyrimido[5',4':5,6]pyrimido[1,2-a]benzimidazol-5(6H)-one | 1.4 | 99.4 | 319,637 |
| 53 | 3-((4-(4-((6-(2,6-dimethylphenyl)-5-oxo-5,6-dihydropyrimido[5',4':5,6]pyrimido[1,2-a]-benzimidazol-2-yl)amino)phenyl)-1-piperazinyl)-methyl)benzonitrile | 1.33 | 97.3 | 337,317, 632 |
| 54 | 6-(2,6-dimethylphenyl)-2-((4-(4-(2-(methyloxy)ethyl)-1-piperazinyl)phenyl)amino)pyrimido [5',4':5,6]-pyrimido[1,2-a]benzimidazol-5(6H)-one | 1.23 | 97.8 | 288,575 |
| 55 | 6-(2,6-dimethylphenyl)-2-((3-(methyloxy)-4-(((2R)-2-pyrrolidinylmethyl)oxy)phenyl)amino)-pyrimido[5',4':5,6]pyrimido[1,2-a]benzimidazol-5(6H)-one | 1.27 | 100 | 562, 534, 282, 302 |
| 56 | 6-(2,6-dimethylphenyl)-2-((4-((2-((1-methylethyl)-amino)ethyl)oxy)phenyl)amino)pyrimido[5',4':5,6]-pyrimido[1,2-a]benzimidazol-5(6I-I)-one | 1.28 | 95.1 | 534, 267, 288 |
| 57 | 6-(4-(methyloxy)phenyl)-2-((4-(4-methyl-1-piperazinyl)phenyl)amino)pyrimido[5',4':5,6]-pyrimido[1,2-a]benzimidazol-5(6H)-one | 1.1 | 98.6 | 287,267, 533 |
| 58 | 6-(2,6-dimethylphenyl)-2-((4-(4-methyl-1-piperazinyl)phenyl)amino)-8,9-dihydroimidazo[1,2-a]pyrimido[5,4-e]pyrimidin-5(6H)-one | 0.87 | 92.2 | 483,262 |
| 59 | 2-((3-chloro-4-((2-(1-piperidinyl)ethyl)oxy)phenyl)-amino)-6-(2,6-dimethylphenyl)pyrimido[5',4':5,6]-pyrimido[1,2-a]benzimidazol-5(6H)-one | 1.28 | 91.1 | 594, 297 |
| 60 | 2-((4-(4-methyl-1-piperazinyl)phenyl)amino)-6-((1S)-1-phenyethyl)pyrimido[5',4':5,6]pyrimido[1,2-a]-benzimidazol-5(6H)-one | 1.31 | 94.8 | 531 |
| 61 | 6-(2,6-dimethylphenyl)-2-((3-fluoro-4-((2-(4-(1-methylethyl)-1-piperazinyl)ethyl)oxy)phenyl)amino)-pyrimido[5',4':5,6]pyrimido[1,2-a]benzimidazol-5(6H)-one | 1.12 | 98.3 | 311,557, 621 |
| 62 | 6-(2,6-dimethylphenyl)-2-((4-((2-(4-(1-methylethyl)-1-piperazinyl)ethyl)oxy)-3-(methyloxy)phenyl)-amino)pyrimido[5',4':5,6]pyrimido[1,2-a]-benzimidazol-5(6H)-one | 1.1 | 92.5 | 317,633 |
| 63 | 6-(2,6-dimethylphenyl)-2-((4-((3-(4-(1-methylethyl)-1-piperazinyl)propyl)oxy)-3-(methyloxy)phenyl)-amino)pyrimido[5',4':5,6]pyrimido[1,2-a]-benzimidazol-5(6H)-one | 1.1 | 97.3 | 647,324 |
| 64 | N-(2-(diethylamino)ethyl)-4-((6-(2,6-dimethylphenyl)-5-oxo-5,6-dihydropyrimido[5',4':5,6]pyrimido[1,2-a]-benzimidazol-2-yl)amino)-N-methylbenzamide | 1.22 | 87 | 295,589, 258 |
| 65 | N-(2-(dimethylamino)ethyl)-3-((6-(2,6-dimethylphenyl)-5-oxo-5,6-dihydropyrimido[5',4':5,6]pyrimido[1,2-a]-benzimidazol-2-yl)amino)benzamide | 1.17 | 89.9 | 547,274 |
| 66 | N-(2-(diethylamino)ethyl)-3-((6-(2,6-dimethylphenyl)-5-oxo-5,6-dihydropyrimido[5',4':5,6]pyrimido[1,2-a]-benzimidazol-2-yl)amino)-N-methylbenzamide | 1.21 | 92.3 | 258, 295, 589 |
| 67 | 6-(2,6-dichlorophenyl)-2-((4-(4-methyl-1-piperazinyl)phenyl)amino)pyrimido[5',4':5,6]-pyrimido[1,2-a]benzinudazol-5(6H)-one | 1.18 | 99.5 | 571/573, 306,286 |
| 68 | 2-((4-(4-methyl-1-piperazinyl)phenyl)amino)-6-((1R)-1-phenylethyl)pyrimido[5',4':5,6]pyrimido[1,2-a]-benzimidazol-5(6H)-one | 1.3 | 90.4 | 531 |
| 69 | 6-(2,6-dimethylphenyl)-2-((4-(4-(2-hydroxyethyl)-l-piperazinyl)phenyl)amino)pyrimido[5',4':5,6]-pyrimido[1,2-a]benzimidazol-5(6H)-one | 1.16 | 99.1 | 281,561 |
| 70 | 2-((3-chloro-4-((2-(4-(1-methylethyl)-1-piperazinyl)-ethyl)oxy)phenyl)amino)-6-(2,6-dimethylphenyl)-pyrimido[5',4':5,6]pyrimido[1,2-a]benzimidazol-5(6H)-one | 1.17 | 95.2 | 319,637 |
| 71 | N-(2-(dimethylamino)ethyl)-4-((6-(2,6-dimethylphenyl)-5-oxo-5,6-dihydropyrimido[5',4':5,6]pyrimido[1,2-a]-benzimidazol-2-yl)amino)benzamide | 1.16 | 94.9 | 274,251, 547 |
| 72 | 6-(2,6-dimethylphenyl)-2-((3-fluoro-4-((3-(4-(1-methylethyl)-1-piperazinyl)propyl)oxy)phenyl)-amino)pyrimido[5',4':5,6]pyrimido[1,2-a]-benzimidazol-5(6H)-one | 1.15 | 99.8 | 318,635 |
| 73 | 2-((3-chloro-4-((3-(4-methyl-1-piperazinyl)propyl)-oxy)phenyl)amino)-6-(2,6-dimethylphenyl)-pyrimido[5',4':5,6]pyrimido[1,2-a]benzimidazol-5(6H)-one | 1.14 | 98.6 | 312,623 |
| 74 | 6-(2,6-dimethylphenyl)-2-((4-(4-methyl-1,4-diazepan-1-yl)phenyl)amino)pyrimido[5',4':5,6]pyrimido[1,2-a]benzimidazol-5(6H)-one | 1.21 | 97.7 | 273,545 |
| 75 | 2-((4-(1,4-diazepan-1-yl)phenyl)amino)-6-(2,6-dimethylphenyl)pyrimido[5',4':5,6]pyrimido[1,2-a]-benzimidazol-5(6H)-one | 1.2 | 100 | 266,286, 531 |
| 76 | 6-(5-chloro-2-(methyloxy)phenyl)-2-((4-(4-methyl-1-piperazinyl)phenyl)amino)pyrimido[5',4':5,6]-pyrimido[1,2-a]benzimidazol-5(6H)-one | 1.21 | 85 | 567, 304, 284 |
| 77 | 2-((4-(4-methyl-1-piperazinyl)phenyl)amino)-6-(2,4,6-trichlorophenyl)pyrimido[5',4':5,6]-pyrimido[1,2-a]benzimidazol-5(6H)-one | 1.36 | 99.5 | 605/607/609 |
| 78 | 6-(4-methylphenyl)-2-((4-(4-methyl-1-piperazinyl)-phenyl)amino)pyrimido[5',4':5,6]pyrimido[1,2-a]-benzimidazol-5(6H)-one | 1.17 | 96.6 | 280,517, 259 |
| 79 | 6-(2-fluorophenyl)-2-((4-(4-methyl-1-piperazinyl)-phenyl)amino)pyrimido[5',4':5,6]pyrimido[1,2-a]-benzimidazol-5(6H)-one | 1.13 | 93.1 | 521,281, 261 |
| 80 | 6-(3-methylphenyl)-2-((4-(4-methyl-1-piperazinyl)-phenyl)amino)pyrimido[5',4':5,6]pyrimido[1,2-a]-benzimidazol-5(6H)-one | 1.25 | 100 | 259,517 |
| 81 | 6-(3-(methyloxy)phenyl)-2-((4-(4-methyl-1-piperazinyl)phenyl)amino)pyrimido[5',4':5,6]-pyrimido[1,2-a]benzimidazol-5(6H)-one | 1.19 | 100 | 267,533 |
| 82 | 6-(4-(butyloxy)phenyl)-2-((4-(4-methyl-1-piperazinyl)phenyl)amino)pyrimido[5',4':5,6]-pyrymido[1,2-a]benzimidazol-5(6H)-one | 1.44 | 100 | 288,575 |
| 83 | 6-(3-fluorophenyl)-2-((4-(4-methyl-1-piperazinyl)-phenyl)amino)pyrimido[5',4':5,6]pyrimido[1,2-a]-benzimidazol-5(6H)-one | 1.22 | 100 | 261,521 |
| 84 | 6-(4-fluorophenyl)-2-((4-(4-methyl-1-piperazinyl)-phenyl)amino)pyrimido[5',4':5,6]pyrimido[1,2-a]-benzimidazol-5(6H)-one | 1.2 | 100 | 261,521 |
| 85 | 6-(2-(methyloxy)phenyl)-2-((4-(4-methyl-1-piperazinyl)phenyl)amino)pyrimido[5',4':5,6]-pyrimido[1,2-a]benzimidazol-5(6H)-one | 1.19 | 100 | 267,533 |
| 86 | 6-(4-chlorophenyl)-2-((4-(4-methyl-1-piperazanyl)-phenyl)amino)pyrimido[5',4':5,6]pyrimido[1,2-a]-benzimidazol-5(6H)-one | 1.29 | 100 | 269,537 |
| 87 | 6-(2,6-dimethylphenyl)-2-((4-(4-methyl-1-piperazinyl)phenyl)amino)imidazo[1,2-a]-pyrimido[5,4-e]pyrimidin-5(6H)-one | 1.08 | 92.3 | 241, 481 |
| 88 | 6-(2,6-difluorophenyl)-2-((4-(4-methyl-1-piperazinyl)-phenyl)amino)pyrimido[5',4':5,6]pyrimido[1,2-a]-benzimidazol-5(6H)-one | 1.25 | 97 | 270,539 |
| 89 | 2-((4-(4-methyl-1-piperazinyl)phenyl)amino)-6-(pentafluorophenyl)pyrimido[5',4':5,6]pyrimido[1,2-a]benzimidazol-5(6H)-one | 1.4 | 100 | 593, 297 |

### LC method:

Unless otherwise indicated all LC analyses were run on a Waters 2790 system with a Phenomenex Luna C₈ (3µ) reverse phase column (100 x 2 mm) run with a flow rate of 1.00 mL/min.

The mobile phase used solvent A (H₂O/0.1% TFA) and solvent B (CH₃CN/0.1% TFA) with a 9.5 min gradient from 5% to 95% CH₃CN. The gradient was followed by a 0.2 min return to 5% CH₃CN and a 3.8 min flush. The peaks of interest eluted on the LC profiles at the times indicated.

### LC-MS method:

1. LCMS analyses were run on an Micromass Single Quadrupole LCMS system comprising an Agilent HP-1100 LC with a Hypersil BDS C₁₈ (5µ) reverse phase column (2.1 x 50 mm)) run with a flow rate of 1.00 mL/min.
2. The mobile phase used solvent A (H₂O/0.1% TFA) and solvent B (CH₃CN/0.1% TFA) with a 2.1 min gradient from 0% to 95% CH₃CN. The gradient was followed by a 0.2 min return to 0% CH₃CN and a 0.1 min flush.
3. The peaks of interest eluted on the LC profiles at the times indicated.

### Proton NMR Spectra:

Unless otherwise indicated all ¹H NMR spectra were run on an Bruker Avance 400 MHz instrument. All observed protons are reported as parts per million (ppm) downfield from tetramethylsilane (TMS) or other internal reference in the appropriate solvent indicated.

### BIOLOGICAL ASSAYS

The following assays can be employed to determine the degree of activity of a compound as a protein kinase inhibitor. Compounds described herein have been tested in one or more of these assays, and have shown activity. Representative compounds of the invention were tested and found to exhibit IC₅₀ values of at least < 10 µM in any one of the described assays, thereby demonstrating and confirming the utility of the compounds of the invention as protein kinase inhibitors and in the prophylaxis and treatment of immune diseases, hyperproliferative disorders, etc.

### LCK-Homogeneous Time Resolved Fluorescent (HTRF) Kinase Assay:

The LCK HTRF assay begins with LCK in the presence of ATP phosphorylating the biotinylated peptide Gastrin. The reaction incubates for 90 min. To quench the assay detection reagents are added which both stop the reaction by diluting out the enzyme and chelating the metals due to the presence of EDTA. Once the detection reagents are added the assay incubates for 30 min to allow for equilibration of the detection reagents.

The LCK HTRF assay is comprised of 10 µL of compound in 100% DMSO, - 15 µL of ATP and biotinylated Gastrin, and 15 µL of LCK KD GST (225-509) for a final volume of 40 µL. The final concentration of gastrin is 1.2µM. The final concentration of ATP is 0.5µM (Km app= 0.6µM+/-0.1) and the final concentration of LCK is 250pM. Buffer conditions are as follows: 50mM HEPES pH 7.5, 50mM NaCl, 20mM MgCl, 5mM MnCl, 2mM DTT, 0.05% BSA.

The assay is quenched and stopped with 160 µL of detection reagent. Detection reagents are as follows: Buffer made of 50mM Tris, pH 7.5, 100mM NaCl, 3mM EDTA, 0.05% BSA, 0.1% Tween20. Added to this buffer prior to reading is Steptavidin allophycocyanin (SA-APC) at a final conc in the assay of 0.0004 mg/mL, and europilated anti-phosphotyrosine Ab (Eu-anti-PY) at a final conc of 0.025nM.

The assay plate is read in either a Discovery or a RubyStar. The eu-anti-PY is excited at 320 nm and emits at 615 nm to excite the SA-APC which in turn emits at 655 nm. The ratio of SA-APC at 655 nm (excited due to close proximity to the Eu-anti-PY because of phosphorylation of the peptide) to free Eu-anti-PY at 615 nm will give substrate phosphorylation.

Assays for other kinases are done in a similar way as described above, varying the concentrations of enzyme, peptide substrate, and ATP added to the reaction, depending on the specific activity of the kinase and measured Km's for the substrates.

The following compounds exhibit activity of better than 1µM in the LCK-HTRF Kinase Assay:
1,1-dimethylethyl 4-(4-((6-(2,6-dimethylphenyl)-5-oxo-5,6-dihydropyrimido-[5',4':5,6]pyrimido[1,2-a]benzimidazol-2-yl)amino)phenyl)-1-piperazinecarboxylate;
2-((3,4-bis(methyloxy)-5-((2-(4-methyl-1-piperazinyl)ethyl)oxy)phenyl)amino)-6-(2,6-dimethylphenyl)pyrimido[5',4':5,6]pyrimido[1,2-a]benzimidazol-5(6H)-one;
2-((3,4-bis(methyloxy)-5-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-one;
2-((3,5-bis(methyloxy)-4-((2-(1-piperidinyl)ethyl)oxy)phenyl)amino)-6-(2,6-dimethylphenyl)pyrimido[5',4':5,6]pyrimido[1,2-a]benzimidazol-5(6H)-one;
2-((3,5-bis(methyloxy)-4-((2-(4-methyl-1-piperazinyl)ethyl)oxy)phenyl)amino)-6-(2,6-dimethylphenyl)pyrimido[5',4':5,6]pyrimido[1,2-a]benzimidazol-5(6H)-one;
2-((3,5-bis(methyloxy)-4-((2-(4-morpholinyl)ethyl)oxy)phenyl)amino)-6-(2,6-dimethylphenyl)pyrimido[5',4':5,6]pyrimido[1,2-a]benzimidazol-5(6H)-one;
2-((3,5-bis(methyloxy)-4-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-one;
2-((3-chloro-4-((2-((1-methylethyl)amino)ethyl)oxy)phenyl)amino)-6-(2,6-dimethylphenyl)pyrimido[5',4':5,6]pyrimido[1,2-a]benzimidazol-5(6H)-one;
2-((3-chloro-4-((2-(1-piperidinyl)ethyl)oxy)phenyl)amino)-6-(2,6-dimethylphenyl)-pyrimido[5',4':5,6]pyrimido[1,2-a]benzimidazol-5(6H)-one;
2-((3-chloro-4-((2-(4-(1-methylethyl)-1-piperazinyl)ethyl)oxy)phenyl)amino)-6-(2,6-dimethylphenyl)pyrimido[5',4':5,6]pyrimido[1,2-a]benzimidazol-5(6H)-one;
2-((3-chloro-4-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-one;
2-((3-chloro-4-(4-(1-methylethyl)-1-piperazinyl)phenyl)amino)-6-(2,6-dimethylphenyl)pyrimido[5',4':5,6]pyrimido[1,2-a]benzimidazol-5(6H)-one;
2-((3-chloro-4-(4-methyl-1-piperazinyl)phenyl)amino)-6-(2,6-dimethylphenyl)-pyrimido[5',4':5,6]pyrimido[1,2-a]benzimidazol-5(6H)-one;
2-((4-((2-(dimethylamino)ethyl)oxy)phenyl)amino)-6-(2,6-dimethylphenyl)-pyrimido[5',4':5,6]pyrimido[1,2-a]benzimidazol-5(6H)-one;
2-((4-((3-(diethylamino)propyl)oxy)-3-fluorophenyl)amino)-6-(2,6-dimethylphenyl)-pyrimido[5',4':5,6]pyrimido[1,2-a]benzimidazol-5(6H)-one;
2-((4-((3-(dimethylamino)propyl)oxy)phenyl)amino)-6-(2,6-dimethylphenyl)-pyrimido[5',4':5,6]pynmido[1,2-a]benzimidazol-5(6H)-one;
2-((4-(1,4-diazepan-1-yl)phenyl)amino)-6-(2,6-dimethylphenyl)pyrimido[5',4':5,6]-pyrimido[1,2-a]benzimidazol-5(6H)-one;
2-((4-(4-((3-chlorophenyl)methyl)-1-piperazinyl)phenyl)amino)-6-(2,6-dimethylphenyl)pyrimido[5',4':5,6]pyrimido[1,2-a]benzimidazol-5(6H)-one;
2-((4-(4-(2-(dimethylamino)ethyl)-1-piperazinyl)phenyl)amino)-6-(2,6-dimethylphenyl)pyrimido[5',4':5,6]pyrimido[1,2-a]benzimidazol-5(6H)-one;
2-((4-(4-(3-(dimethylamino)propyl)-1-piperazinyl)phenyl-)amino)-6-(2,6-dimethylphenyl)pyrimido[5',4':5,6]pyrimido[1,2-a]benzimidazol-5(6H)-one;
2-((4-(4-(cyclohexylmethyl)-1-piperazinyl)phenyl)amino)-6-(2,6-dimethylphenyl)-pyrimido[5',4':5,6]pyrimido[1,2-a]benzimidazol-5(6H)-one;
2-((4-(4-methyl-1-piperazinyl)phenyl)amino)-6-((1R)-1-phenylethyl)pyrimido-[5',4':5,6]pyrimido[1,2-a]benzimidazol-5(6H)-one;
2-((4-(4-methyl-1-piperazinyl)phenyl)amino)-6-((1S)-1-phenylethyl)pyrimido-[5',4':5,6]pyrimido[1,2-a]benzimidazol-5(6H)-one;
2-((4-(4-methyl-1-piperazinyl)phenyl)amino)-6-(2,4,6-trichlorophenyl)pyrimido-[5',4':5,6]pyrimido[1,2-a]benzimidazol-5(6H)-one;
2-((4-(4-methyl-1-piperazinyl)phenyl)amino)-6-(pentafluorophenyl)pyrimido-[5',4':5,6]pyrimido[1,2-a]benzimidazol-5(6H)-one;
2-(4-(4-((6-(2,6-dimethylphenyl)-5-oxo-5,6-dihydropyrimido[5',4':5,6]pyrimido[1,2-a]benzimidazol-2-yl)amino)phenyl)-1-piperazinyl)acetamide;
3-((4-(4-((6-(2,6-dimethylphenyl)-5-oxo-5,6-dihydropyrimido[5',4':5,6]-pyrimido[1,2-a]benzimidazol-2-yl)amino)phenyl)-1-piperazinyl)methyl)-benzonitrile;
6-(2,6-dichlorophenyl)-2-((4-(4-methyl-1-piperazinyl)phenyl)amino)pyrimido-[5',4':5,6]pyrimido[1,2-a]benzimidazol-5(6H)-one;
6-(2,6-difluorophenyl)-2-((4-(4-methyl-1-piperazinyl)phenyl)amino)pyrimido-[5',4':5,6]pyrimido[1,2-a]benzimidazol-5(6H)-one;
6-(2,6-dimethylphenyl)-2-((2-methyl-4-((3-(1-piperidinyl)propyl)oxy)phenyl)-amino)pyrimido[5',4':5,6]pyrimido[1,2-a]benzimidazol-5(6H)-one;
6-(2,6-dimethylphenyl)-2-((2-methyl-4-((3-(4-methyl-1-piperazinyl)propyl)oxy)-phenyl)amino)pyrimido[5',4':5,6]pyrimido[1,2-a]benzimidazol-5(6H)-one;
6-(2,6-dimethylphenyl)-2-((2-methyl-4-(4-methyl-1-piperazinyl)phenyl)amino)-pynmido[5',4':5,6]pyrimido[1,2-a]benzimidazol-5(6H)-one;
6-(2,6-dimethylphenyl)-2-((3-((3-(1-piperidinyl)propyl)oxy)phenyl)amino)pyrimido-[5',4':5,6]pyrimido[1,2-a]benzimidazol-5(6H)-one;
6-(2,6-dimethylphenyl)-2-((3-((3-(4-methyl-1-piperazinyl)propyl)oxy)phenyl)-amino)pyrimido[5',4':5,6]pyrimido[1,2-a]benzimidazol-5(6H)-one;
6-(2,6-dimethylphenyl)-2-((3-(4-methyl-1-piperazinyl)phenyl)amino)pyrimido-[5',4':5,6]pyrimido[1,2-a]benzimidazol-5(6H)-one;
6-(2,6-dimethylphenyl)-2-((3-(methyloxy)-4-(((1-methyl-4-piperidinyl)methyl)oxy)-phenyl)amino)pyrimido[5',4':5,6]pyrimido[1,2-a]benzimidazol-5(6H)-one;
6-(2,6-dimethylphenyl)-2-((3-(methyloxy)-4-(((2R)-2-pyrrolidinylmethyl)oxy)-phenyl)amino)pyrimido[5',4':5,6]pyrimido[1,2-a]benzimidazol-5(6H)-one;
6-(2,6-dimethylphenyl)-2-((3-(methyloxy)-4-((2-(1-methyl-4-piperidinyl)ethyl)oxy)-phenyl)amino)pyrimido[5',4':5,6]pyrimido[1,2-a]benzimidazol-5(6H)-one;
6-(2,6-dimethylphenyl)-2-((3-(methyloxy)-4-((2-(4-methyl-1-piperazinyl)ethyl)oxy)-phenyl)amino)pyrimido[5',4':5,6]pyrimido[1,2-a]benzimidazol-5(6H)-one;
6-(2,6-dimethylphenyl)-2-((3-(methyloxy)-4-((3-(4-methyl-1-piperazinyl)propyl)-oxy)phenyl)amino)pyrimido[5',4':5,6]pyrimido[1,2-a]benzimidazol-5(6H)-one;
6-(2,6-dimethylphenyl)-2-((3-(methyloxyl)-4-(1-piperazinyl)phenyl)amino)-pyrimido[5',4':5,6]pyrimido[1,2-a]benzimidazol-5(6H)-one;
6-(2,6-dimethylphenyl)-2-((3-(methyloxy)-4-(4-methyl-1-piperazinyl)phenyl)-amino)pyrimido[5',4':5,6]pyrimido[1,2-a]benzimidazol-5(6H)-one;
6-(2,6-dimethylphenyl)-2-((3,4,5-tris(methyloxy)phenyl)amino)pyrimido[5',4':5,6]-pyrimido[1,2-a]benzimidazol-5(6H)-one;
6-(2,6-dimethylphenyl)-2-((3-fluoro-4-((2-(1-piperidinyl)ethyl)oxy)phenyl)amino)-pyrimido[5',4':5,6]pyrimido[1,2-a]benzimidazol-5(6H)-one;
6-(2,6-dimethylphenyl)-2-((3-fluoro-4-((2-(4-(1-methylethyl)-1-piperazinyl)ethyl)-oxy)phenyl)amino)pyrimido[5',4':5,6]pyrimido[1,2-a]benzimidazol-5(6H)-one;
6-(2,6-dimethylphenyl)-2-((3-fluoro-4-((2-(4-methyl-1-piperazinyl)ethyl)oxy)-phenyl)amino)pyrimido[5',4':5,6]pyrimido[1,2-a]benzimidazol-5(6H)-one;
6-(2,6-dimethylphenyl)-2-((3-fluoro-4-((3-(1-piperidinyl)propyl)oxy)phenyl)amino)-pyrimido[5',4':5,6]pyrimido[1,2-a]benzimidazol-5(6H)-one;
6-(2,6-dimethylphenyl)-2-((3-fluoro-4-((3-(4-(1-methylethyl)-1-piperazinyl)propyl)-oxy)phenyl)amino)pyrimido[5',4':5,6]pyrimido[1,2-a]benzimidazol-5(6H)-one;
6-(2,6-dimethylphenyl)-2-((3-fluoro-4-((3-(4-methyl-1-piperazinyl)propyl)oxy)-phenyl)amino)pyrimido[5',4':5,6]pyrimido[1,2-a]benzimidazol-5(6H)-one;
6-(2,6-dimethylphenyl)-2-((3-fluoro-4-(4-(1-methylethyl)-1-piperazinyl)phenyl)-amino)pyrimido[5',4':5,6]pyrimido[1,2-a]benzimidazol-5(6H)-one;
6-(2,6-dimethylphenyl)-2-((3-fluoro-4-(4-methyl-1-piperazinyl)phenyl)amino)-pyrimido[5',4':5,6]pyrimido[1,2-a]benzimidazol-5(6H)-one;
6-(2,6-dimethylphenyl)-2-((4-((((2S)-1-methyl-2-pyrrolidinyl)methyl)oxy)phenyl)-amino)pyrimido[5',4':5,6]pyrimido[1,2-a]benzimidazol-5(6H)-one;
6-(2,6-dimethylphenyl)-2-((4-(((2R)-2-pyrrolidinylmethyl)oxy)phenyl)amino)-pyrimido[5',4':5,6]pyrimido[1,2-a]benzimidazol-5(6H)-one;
6-(2,6-dimethylphenyl)-2-((4-(((2S)-2-pyrrolidinylmethyl)oxy)phenyl)amino)-pyrimido[5',4':5,6]pyrimido[1,2-a]benzimidazol-5(6H)-one;
6-(2,6-dimethylphenyl)-2-((4-((2-((1-methylethyl)amino)ethyl)oxy)-3-(methyloxy)-phenyl)amino)pyrimido[5',4':5,6]pyrimido[1,2-a]benzimidazol-5(6H)-one;
6-(2,6-dimethylphenyl)-2-((4-((2-((1-methylethyl)amino)ethyl)oxy)phenyl)amino)-pyrimido[5',4':5,6]pyrimido[1,2-a]benzimidazol-5(6H)-one;
6-(2,6-dimethylphenyl)-2-((4-((2-(1-methyl-4-piperidinyl)ethyl)oxy)phenyl)amino)-pyrimido[5',4':5,6]pyrimido[1,2-a]benzimidazol-5(6H)-one;
6-(2,6-dimethylphenyl)-2-((4-((2-(1-piperidinyl)ethyl)oxy)phenyl)amino)-pynmido[5',4':5,6]pyrimido[1,2-a]benzimidazol-5(6H)-one;
6-(2,6-dimethytphenyl)-2-((4-((2-(4-(1-methylethyl)-1-piperazinyl)ethyl)oxy)-3-(methyloxy)phenyl)amino)pyrimido[5',4':5,6]pyrimido[1,2-a]benzimidazol-5(6H)-one;
6-(2,6-dimethylphenyl)-2-((4-((2-(4-methyl-1-piperazinyl)ethyl)oxy)phenyl)amino)-pyrimido[5',4':5,6]pyrimido[1,2-a]benzimidazol-5(6H)-one;
6-(2,6-dimethylphenyl)-2-((4-((2-(4-morpholinyl)ethyl)oxy)phenyl)amino) pyrimido[5',4':5,6]pyrimido[1,2-a]benzimidazol-5(6H)-one;
6-(2,6-dimethylphenyl)-2-((4-((2-(methylamino)ethyl)oxy)phenyl)amino)-pyrimido[5',4':5,6]pyrimido[1,2-a]benzimidazol-5(6H)-one;
6-(2,6-dimethylphenyl)-2-((4-((3-(1-piperidinyl)propyl)oxy)phenyl)amino)-pyrimido[5',4':5,6]pyrimido[1,2-a]benzimidazol-5(6H)-one;
6-(2,6-dimethylphenyl)-2-((4-((3-(4-(1-methylethyl)-1-piperazinyl)propyl)oxy)-3-(methyloxy)phenyl)amino)pyrimido[5',4':5,6]pyrimido[1,2-a]benzimidazol-5(6H)-one;
6-(2,6-dimethylphenyl)-2-((4-(1-piperazinyl)phenyl)amino)pyrimido[5',4':5,6]-pyrimido[1,2-a]benzimidazol-5(6H)-one;
6-(2,6-dimethylphenyl)-2-((4-(4-((3-(methyloxy)phenyl)methyl)-1-piperazinyl)-phenyl)amino)pyrimido[5',4':5,6]pyrimido[1,2-a]benzimidazol-5 (6H)-one;
6-(2,6-dimethylphenyl)-2-((4-(4-(1-methylethyl)-1-piperazinyl)phenyl)amino)-pyrimido[5',4':5,6]pyrimido[1,2-a]benzimidazol-5(6H)-one;
6-(2,6-dimethylphenyl)-2-((4-(4-(1-methylethyl)-1-piperazinyl)-3-(methyloxy)-phenyl)amino)pyrimido[5',4':5,6]pyrimido[1,2-a]benzimidazol-5(6H)-one;
6-(2,6-dimethylphenyl)-2-((4-(4-(2-(methyloxy)ethyl)-l-piperazinyl)phenyl)amino)-pyrimido[5',4':5,6]pyrimido[1,2-a]benzimidazol-5(6H)-one;
6-(2,6-dimethylphenyl)-2-((4-(4-(2-hydroxyethyl)-1-piperazinyl)phenyl)amino)-pyrimido[5',4':5,6]pyrimido[1,2-a]benzimidazol-5(6H)-one;
6-(2,6-dimethylphenyl)-2-((4-(4-methyl-1,4-diazepan-1-yl)phenyl)amino)-pyrimido[5',4':5,6]pyrimido[1,2-a]benzimidazol-5(6H)-one;
6-(2,6-dimethylphenyl)-2-((4-(4-methyl-1-piperazinyl)phenyl)amino)-pyrimido[5',4':5,6]pyrimido[1,2-a]benzimidazol-5(6H)-one;
6-(2,6-dimethylphenyl)-2-((4-(4-methyl-1-piperazinyl)phenyl)amino)-8,9-dihydroimidazo[1,2-a]pyrimido[5,4-e]pyrimidin-5(6H)-one;
6-(2,6-dimethylphenyl)-2-((4-(4-methyl-1-piperazinyl)phenyl)amino)imidazo[1,2-a]pyrimido[5,4-e]pyrimidin-5(6H)-one;
6-(2-fluorophenyl)-2-((4-(4-methyl-1-piperazinyl)phenyl)amino)pyrimido[5',4':5,6]-pyrimido[1,2-a]benzimidazol-5(6H)-one;
6-(3-(methyloxy)phenyl)-2-((4-(4-methyl-1-piperazinyl)phenyl)amino)pyrimido-[5',4':5,6]pyrimido[1,2-a]benzimidazol-5(6H)-one;
6-(3-fluorophenyl)-2-((4-(4-methyl-1-piperazinyl)phenyl)amino)pyrimido[5',4':5,6]-pyrimido[1,2-a]benzimidazol-5(6H)-one;
6-(3-methylphenyl)-2-((4-(4-methyl-1-piperazinyl)phenyl)amino)-pyrimido[5',4':5,6]pyrimido[1,2-a]benzimidazol-5(6H)-one;
6-(4-(methyloxy)phenyl)-2-((4-(4-methyl-1-piperazinyl)phenyl)amino)pyrimido-[5',4':5,6]pyrimido[1,2-a]benmidazol-5(6H)-one;
6-(4-fluorophenyl)-2-((4-(4-methyl-1-piperazinyl)phenyl)amino)pyrimido[5',4':5,6]-pyrimido[1,2-a]benzimidazol-5(6H)-one;
6-(4-methylphenyl)-2-((4-(4-methyl-1-piperazinyl)phenyl)amino)-pyrimido[5',4':5,6]pyrimido[1,2-a]benzimidazol-5(6H)-one;
6-(5-chloro-2-(methyloxy)phenyl)-2-((4-(4-methyl-1-piperazinyl)phenyl)amino)-pyrimido[5',4':5,6]pyrimido[1,2-a]benzimidazol-5(6H)-one;
N-(2-(diethylamino)ethyl)-3-((6-(2,6-dimethylphenyl)-5-oxo-5,6-dihydropyrimido-[5',4':5,6]pyrimido[1,2-a]benzimidazol-2-yl)amino)-N-methylbenzamide;
N-(2-(diethylamino)ethyl)-4-((6-(2,6-dimethylphenyl)-5-oxo-5,6-dihydropyrimido-[5',4':5,6]pyrimido[1,2-a]benzimidazol-2-yl)amino)-N-methylbenzamide;
N-(2-(dimethylamino)ethyl)-3-((6-(2,6-dimethylphenyl)-5-oxo-5,6-dihydropyrimido[5',4':5,6]pyrimido[1,2-a]benzimidazol-2-yl)amino)benzamide; and
N-(2-(dimethylamino)ethyl)-4-((6-(2,6-dimethylphenyl)-5-oxo-5,6-dihydropyrimido[5',4':5,6]pyrimido[1,2-a]benzimidazol-2-yl)amino)benzamide.

### Human mixed lymphocyte reaction (huMLR):

The purpose of this assay is to test the potency of T cell activation inhibitors in an *in vitro* model of allogeneic T cell stimulation. Human peripheral blood lymphocytes (hPBL; 2x10⁵/well) are incubated with mitomycin C-treated B lymphoblastoid cells (JY cell line; 1x10⁵/well) as allogeneic stimulators in the presence or absence of dilutions of potential inhibitor compound in 96-well roundbottom tissue culture plates. These cultures are incubated at 37 °C in 5% CO₂ for 6 days total. The proliferative response of the hPBL is measured by ³H-thymidine incorporation overnight between days 5 and 6 after initiation of culture. Cells are harvested onto glass fiber filters and ³H-thymidine incorporation into DNA is analyzed by liquid scintillation counter.

### Jurkat proliferation/survival assay:

The purpose of this assay is to test the general anti-proliferative/cytotoxic effect of compounds on the Jurkat human T cell line. Jurkat cells (1x10⁵/well) are plated in 96-well flat-bottom tissue culture plates with or without compound dilutions and cultured for 72 h at 37 °C in 5% CO₂. Viable cell number is determined during the last 4 h of culture by adding 10 µL/well WST-1 dye. WST-1 dye conversion relies on active mitochondrial electron transport for reduction of the tetrazolium dye. The dye conversion is read by OD at 450-600 nm.

### Anti-CD3/CD28-induced T cell IL-2 secretion and proliferation assay:

The purpose of this assay is to test the potency of T cell receptor (TCR; CD3) and CD28 signaling pathway inhibitors in human T cells. T cells are purified from human peripheral blood lymphocytes (hPBL) and pre-incubated with or without compound prior to stimulation with a combination of an anti-CD3 and an anti-CD28 antibody in 96-well tissue culture plates (1x10⁵ T cells/well). Cells are cultured for ∼20 h at 37 °C in 5% CO₂, then secreted IL-2 in the supernatants is quantified by cytokine ELISA (Pierce/Endogen). The cells remaining in the wells are then pulsed with ³H-thymidine overnight to assess the T cell proliferative response. Cells are harvested onto glass fiber filters and ³H-thymidine incorporation into DNA is analyzed by liquid scintillation counter. For comparison purposes, phorbol myristic acid (PMA) and calcium ionophore can be used in combination to induce IL-2 secretion from purified T cells. Potential inhibitor compounds can be tested for inhibition of this response as described above for anti-CD3 and -CD28 antibodies.

The compounds of the present invention are useful for treating various disorders and/or diseases related to kinase activity, as described herein. For example, for the treatment of Lck-mediated diseases and other diseases listed above, the compounds of the present invention may be administered orally, parentally, by inhalation spray, rectally, or topically in dosage unit formulations containing conventional pharmaceutically acceptable carriers, adjuvants, and vehicles. The term parenteral as used herein includes, subcutaneous, intravenous, intramuscular, intrasternal, infusion techniques or intraperitoneally.

Treatment of diseases and disorders herein is intended to also include the prophylactic administration of a compound of the invention, a pharmaceutical salt or derivative thereof, or a pharmaceutical composition of either to a subject (*i.e.,* an animal, preferably a mammal, most preferably a human) believed to be in need of preventative treatment, such as, for example, pain, inflammation and the like.

While it may be possible to administer a compound of the invention alone, in the methods described, the compound administered normally will be present as an active ingredient in a pharmaceutical composition. Thus, in another embodiment of the invention, there is provided a pharmaceutical composition comprising a compound of this invention in combination with a pharmaceutically acceptable carrier, which includes diluents, excipients and the like as described herein. A pharmaceutical composition of the invention may comprise an effective amount of a compound of the invention or an effective dosage amount of a compound of the invention. An effective dosage amount of a compound of the invention includes an amount less than, equal to or greater than an effective amount of the compound; for example, a pharmaceutical composition in which two or more unit dosages, such as in tablets, capsules and the like, are required to administer an effective amount of the compound, or alternatively, a multidose pharmaceutical composition, such as powders, liquids and the like, in which an effective amount of the compound is administered by administering a portion of the composition.

The dosage regimen for treating Lck-mediated diseases and other diseases listed above with the compounds of this invention and/or compositions of this invention is based on a variety of factors, including the type of disease, the age, weight, sex, medical condition of the patient, the severity of the condition, the route of administration, and the particular compound employed. Thus, the dosage regimen may vary widely, but can be determined routinely using standard methods. Dosage levels of the order from about 0.01 mg to 30 mg per kilogram of body weight per day, preferably from about 0.1 mg to 10 mg/kg, more preferably from about 0.25 mg to 1 mg/kg are useful for all methods of use disclosed herein.

The pharmaceutically active compounds of this invention can be processed in accordance with conventional methods of pharmacy to produce medicinal agents for administration to patients, including humans and other mammals.

For oral administration, the pharmaceutical composition may be in the form of, for example, a capsule, a tablet, a suspension, or liquid. The pharmaceutical composition is preferably made in the form of a dosage unit containing a given amount of the active ingredient. For example, these may contain an amount of active ingredient from about 1 to 2000 mg, preferably from about 1 to 500 mg, more preferably from about 5 to 150 mg. A suitable daily dose for a human or other mammal may vary widely depending on the condition of the patient and other factors, but, once again, can be determined using routine methods.

The active ingredient may also be administered by injection as a composition with suitable carriers including saline, dextrose, or water. The daily parenteral dosage regimen will be from about 0.1 to about 30 mg/kg of total body weight, preferably from about 0.1 to about 10 mg/kg, and more preferably from about 0.25 mg to 1 mg/kg.

Injectable preparations, such as sterile injectable aqueous or oleaginous suspensions, may be formulated according to the known are using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

Suppositories for rectal administration of the drug can be prepared by mixing the drug with a suitable non-irritating excipient such as cocoa butter and polyethylene glycols that are solid at ordinary temperatures but liquid at the rectal temperature and will therefore melt in the rectum and release the drug.

A suitable topical dose of active ingredient of a compound of the invention is 0.1 mg to 150 mg administered one to four, preferably one or two times daily. For topical administration, the active ingredient may comprise from 0.001 % to 10% w/w, *e.g*., from 1% to 2% by weight of the formulation, although it may comprise as much as 10% w/w, but preferably not more than 5% w/w, and more preferably from 0.1 % to 1% of the formulation.

Formulations suitable for topical administration include liquid or semi-liquid preparations suitable for penetration through the skin (*e.g*., liniments, lotions, ointments, creams, or pastes) and drops suitable for administration to the eye, ear, or nose.

For administration, the compounds of this invention are ordinarily combined with one or more adjuvants appropriate for the indicated route of administration. The compounds may be admixed with lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinyl-pyrrolidine, and/or polyvinyl alcohol, and tableted or encapsulated for conventional administration. Alternatively, the compounds of this invention may be dissolved in saline, water, polyethylene glycol, propylene glycol, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

The pharmaceutical compositions may be made up in a solid form (including granules, powders or suppositories) or in a liquid form (*e.g*., solutions, suspensions, or emulsions). The pharmaceutical compositions may be subjected to conventional pharmaceutical operations such as sterilization and/or may contain conventional adjuvants, such as preservatives, stabilizers, wetting agents, emulsifiers, buffers etc.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise, as in normal practice, additional substances other than inert diluents, *e.g*., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting, sweetening, flavoring, and perfuming agents.

While the compounds of the invention can be administered as the sole active pharmaceutical agent, in a composition, they (or the composition) can also be used in combination with one or more compounds of the invention, or with other agents, as appreciated by those of ordinary skill in the art. When administered as a combination, the therapeutic agents can be formulated as separate compositions that are given at the same time or different times, or the therapeutic agents can be given as a single composition.

The foregoing is merely illustrative of the invention and is not intended to limit the invention to the disclosed compounds. Variations and changes, which are obvious to one skilled in the art are intended to be within the scope and nature of the invention which are defined in the appended claims.

## Claims

1. **A compound of Formula I** 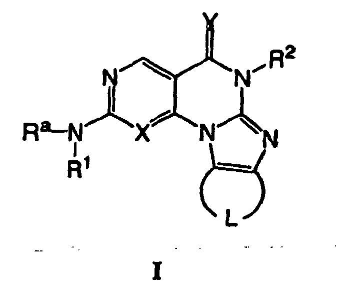 or a pharmaceutically-acceptable salt or derivative thereof, wherein
XisNorCH;
Y is NH, N(CN), O or S;
L is a 4-atom chain made up of C and N atoms, wherein the chain is substituted by 0 or 1 R³ groups and the chain is additionally substituted by 0, 1, 2 or 3 substituents independently selected from R^{c};
R¹ is selected from -R¹¹, -R¹¹-R¹²,-R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ and -R¹¹-R¹⁴-R¹³-R¹⁴, any of which is substituted by 0, 1, 2, 3, 4 or 5 substituents independently selected from R^{c};
R² is selected from -R²¹, -R²¹-R²², -R²¹-R²⁴, -R²²-R²⁴, -R²¹-R²²-R²⁴, -R²¹-R²³-R²⁴, -R²²-R²³-R²⁴, -R²¹-R²³-R²²-R²⁴ and -R²¹-R²²-R²³-R²⁴, any of which is substituted by 0, 1, 2, 3, 4 or 5 substituents independently selected from R^{c};
R³ is selected from -R³², -R³⁴, -R³²-R³⁴, -R³³-R³⁴, -R³³-R³²-R³⁴ and -R³²-R³³-R³⁴, any of which is substituted by 0, 1, 2, 3, 4 or 5 substituents independently selected from R^{c};
R¹¹ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R ¹² is independently at each instance C₁₋₈alkyl;
R¹³ is independently at each instance -C(=O)-, -C(=O)O-, -C(=O)N(R^{a})-, -C(=NR^{a})N(R^{a}) -, -O-, -OC(=O)-, -OC(=O)N(R^{a}) -, -OC(=O)N(R^{a})S(=O)₂-, -OC₂₋₆alkylN(R^{a}) -, -OC₂₋₆alkyl-, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})-, -N(R^{a}) -, -N(R^{a})C(=O)-, -N(R^{a})C(=O)O-, -N(R^{a})C(=O)N(R^{a}) -, -N(R^{a})C(=NR^{a})N(R^{a})-, -N(R^{a})S(=O)₂-, -N(R^{a})S(=O)₂N(R^{a})-, -NR^{a}C₂₋₆alkylN(R²)- or -NR²C₂₋₆alkylO-;
R¹⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R²¹ is independently at each instance a saturated or unsaturated 5-, 6- or 7-membered monocyclic or 6-, 7-, 8-, 9-,10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R²² is independently at each instance C₁₋₈alkyl;
R²³ is independently at each instance -C(=O)-, -C(=O)O-, -C(=O)N(R^{a})-, -C(=NR^{a})N(R^{a})-, -O-, -OC(=O)-. -OC(=O)N(R^{a})-, -OC(=O)N(R^{a}S(=O)₂-, -OC₂₋₆alkylN(R^{a})-, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})-, -N(R^{a}), -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a}) -, -NR^{a}C₂₋₆alkylN(R^{a})- or -NR^{a}C₂₋₆alkylO-;
R²⁴ is independently at each instance a saturated or unsaturated 5-, 6- or 7-membered monocyclic or 6-, 7-, 8-, 9-, 10- or 11-membered bicyclic ring containing 0,1,2,3 or 4 atoms selected from N, O and S, so long as the combination of O and S atoms is not greater than 2, wherein the carbon atoms of the ring are substituted by 0, 1 or 2 oxo groups;
R³² is independently at each instance C₁₋₈alkyl;
R³³ is independently at each instance -C(=O)-, -C(=O)O-, -C(=O)N(R^{a})-, -C(=NR²)N(R²) -*,* -O-, -OC(=O)-, -OC(=O)N(R^{a}) -, -OC(=O)N(R^{a})S(=O)₂-, -OC₂₋₆alkylN(R^{a})-, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a}) -, -NR^{a}C₂₋₆alkylN(R^{a})- or -NR^{a}C₂₋₆alkylO-;
R³⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, 0 and S, so long as the combination of 0 and S atoms is not greater than 2, wherein the carbon atoms of the ring are substituted by 0, 1 or 2 oxo groups;
R^{a} is independently at each instance H or R^{b};
R^{b} is independently at each instance C₁₋₈alkyl, CN, phenyl or benzyl; and
R^{c} is independently at each instance C₁₋₈alkyl, C₁₋₄haloalkyl, halo, cyano, nitro, -C₂₋₆alkylOR^{a}, -C₂₋₆alkyl C(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -OC₂₋₆alkylNR^{a}R^{a}, -OC₂₋₆alkylC(=O)NR^{a}R^{a}, -OC₂₋₆alkylOR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})*S*(=O)₂NR^{a}R^{a}, -NR^{a}C₂₋₆alkylNR^{a}R^{a} or -NR^{a}C₂₋₆alkylOR^{a}.

2. A compound according to Claim 1 wherein X is N and Y is O.

3. A compound according to Claim 1 wherein L is a 4-atom chain formed up of C and N atoms, wherein the chain is substituted by 0 or 1 R³ groups and the chain is additionally substituted by 0, 1, 2 or 3 substituents independently selected from R^{c}.

4. A compound according to Claim 1 wherein R¹¹ is phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine or morpholine.

5. A compound according to Claim 1 wherein R²¹ is phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine or morpholine.

6. A compound according to Claim 1 of Formula II 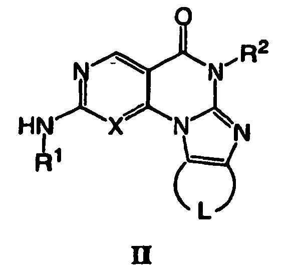 or a pharmaceutically-acceptable salt thereof, wherein
X is N or CH;
L is a 3- or 4-atom chain made up of C and N atoms, wherein the chain is substituted by 0 or 1 R³ groups and the chain is additionally substituted by 0, 1, 2 or 3 substituents independently selected from R^{c};
R¹ is selected from -R¹¹, -R¹¹-R¹², -R¹¹-R¹⁴, -R¹²-R¹⁴, -R¹¹-R¹²-R¹⁴, -R¹¹-R¹³-R¹⁴, -R¹²-R¹³-R¹⁴, -R¹¹-R¹³-R¹²-R¹⁴ and -R¹¹-R¹²-R¹³-R¹⁴, any of which is substituted by 0, 1, 2, 3 or 4 substituents independently selected from R^{c};
R² is selected from -R²¹, -R²¹-R²², -R²¹-R²⁴, -R²²-R²⁴, -R²¹-R²²-R²⁴, -R²¹-R²³-R²⁴ -R²²-R²³-R²⁴, -R²¹-R²³-R²¹-R²⁴ and -R²¹-R²²-R²³-R²⁴, any of which is substituted by 0, 1, 2, 3 or 4 substituents independently selected from R^{c};
R³ is selected from -R³², -R³⁴, -R³²-R³⁴, -R³³-R³⁴, -R³³-R³²-R³⁴ and -R³²-R³³-R³⁴, any of which is substituted by 0, 1, 2, 3 or 4 substituents independently selected from R^{c};
R¹¹ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R¹² is independently at each instance C₁₋₈alkyl;
R¹³ is independently at each instance -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-;
R¹⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R²¹ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R²² is independently at each instance C₁₋₈alkyl;
R²³ is independently at each instance -C(=O)-, -C(=O)O-, -C(=O)NR^{a}-, -C(=NR^{a})NR^{a}-, -0-, -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a} C₂₋₆alkylO-;
R²⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R³² is independently at each instance C₁₋₈alkyl;
R³³ is independently at each instance -C(=O)-, -C(=O)O-, -C(=O)NR^{a}-, -C(=NR^{a})NR^{a}-, -O-. -OC(=O)-, -OC(=O)NR^{a}-, -OC(=O)N(R^{a})S(=O)₂-, -OC₂₋₆alkylNR^{a}-, -OC₂₋₆alkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})- or -NR^{a}C₂₋₆alkylO-;
R³⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of O and S atoms is not greater than 2, wherein the carbon atoms of the ring are substituted by 0, 1 or 2 oxo groups;
R^{a} is independently at each instance H or R^{b};
R^{b} is independently at each instance C₁₋₈alkyl, phenyl or benzyl; and
R^{c} is independently at each instance C₁₋₈alkyl, C₁₋₄haloalkyl, halo, cyano, nitro, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -OC₂₋₆alkylNR^{a}R^{a}, -OC₂₋₆alkylOR^{a}, -SR^{a}, -S(=0)R^{b}, -S(=O)₂R^{b}, -S(=O)₂NR^{a}R^{a}, -S(=O)₂NR^{a})C(=O)R^{b}, -S(=O)₂N(R^{a})C(=O)OR^{b}, -S(=O)₂N(R^{a})C(=O)NR^{a}R^{a} -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{a}, -N(R^{a})S(=O)₂NR^{a}R^{a}, -NR^{a}C₂₋₆alkylNR^{a}R^{a} or -NR^{a}C₂₋₆alkylOR^{a}.

7. A compound according to Claim 1 wherein
X is N;
Y is O;
L is a 4-atom chain formed of C atoms, wherein the chain is substituted by 0 or 1 R³ groups and the chain is additionally substituted by 0, 1, 2 or 3 substituents independently selected from R^{c};
R¹ is selected from -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ and -R¹¹-R¹⁴-R¹³-R¹⁴, any of which is substituted by 0, 1, 2, 3, 4 or 5 substituents independently selected from R^{c};
R² is selected from -R²¹, -R²¹-R²²,-R²¹-R²⁴, -R²²-R²⁴, -R²¹-R²²-R²⁴, -R²¹-R²³-R²⁴ and -R²¹-R²³-R²⁴, any of which is substituted by 0, 1, 2, 3, 4 or 5 substituents independently selected from R^{c};
R³ is selected from -R³², -R³⁴, -R³²-R³⁴, -R³³-R³⁴, -R³³-R³²-R³⁴ and -R³²-R³³-R³⁴, any of which is substituted by 0, 1, 2, 3, 4 or 5 substituents independently selected from R^{c};
R¹¹ is independently at each instance a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine or morpholine;
R¹² is independently at each instance C₁₋₈alkyl;
R¹³ is independently at each instance -C(=O)-, -C(=O)O-, -C(=O)N(R^{a})-, -C(=NR^{a})N(R^{a}) -, -O-, -OC(=O)N(R^{a}) -, -OC₂₋₆alkylN(R^{a}) ₂-, -OC₂₋₆alkyl-, -OC₂₋₆alkylO-, -S-, -S(=O)-, -S(=O)₂-, -S(=O)₂N(R^{a})-, -S(=O)₂N(R^{a})C(=O)-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆alkylN(R^{a})-, or -NR^{a}C₂₋₆alkylO-;
R¹⁴ is independently at each instance a saturated or unsaturated 5-, 6- or 7-membered monocyclic or 6-, 7-, 8-, 9-, 10- or 11-membered bicyclic ring containing 0, 1, 2, 3 or 4 atoms selected from N, O and S, so long as the combination of 0 and S atoms is not greater than 2, wherein the carbon atoms of the ring are substituted by 0, 1 or 2 oxo groups;
R²¹ is independently at each instance a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine or morpholine;
R²² is independently at each instance C₁₋₈alkyl;
R²³ is independently at each instance -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -OC₂₋₆alkylN(R^{a})-, -OC₂₋₆alkylO-, S-, -S(=O)-*,* -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})-, -N(R^{a})-,-N(R^{a})C(=O)-, -N(R^{a})C(=O)O-, -N(R^{a})C(=O)N(R^{a}) -, -N(R^{a})C(=NR^{a})N(R^{a})-, -N(R^{a})S(=O)₂-, -N(R^{a})S(=O)₂N(R^{a}) -, -NR^{a}C₂₋₆alkylN(R^{a})- or -NR^{a}C₂₋₆alkylO-;
R²⁴ is independently at each instance a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine, morpholine, pyran, dioxane, cyclopropane, cyclobutane, cyclopentane or cyclohexane;
R³² is independently at each instance C₁₋₈alkyl;
R³³ is independently at each instance -C(=O)-, -C(=O)O-, -C(=O)N(R^{a})-, -C(=NR^{a})N(R^{a})-, -O-, -OC₂₋₆alkylN(R^{a})-, -OC₂₋₆alkylO-, -S-, -S(=O)-, -S(=O)₂-, -S(=O)₂N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a}) -, -N(R^{a})S(=O)₂-, -N(R^{a})S(=O)₂N(R^{a})-, -NR^{a}C₂₋₆alkylN(R^{a})-or -NR^{a}C₂₋₆alkylO-;
R³⁴ is independently at each instance a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine, morpholine, pyran, dioxane, cyclopropane, cyclobutane, cyclopentane or cyclohexane;
R^{a} is independently at each instance H or R^{b};
R^{b} is independently at each instance C₁₋₈alkyl, CN, phenyl or benzyl; and
R^{c} is independently at each instance C₁₋₈alkyl, C₁₋₄haloalkyl, halo, cyano, nitro, -C₂₋₆alkylOR^{a}, -C₂₋₆alkyl C(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -OC₂₋₆alkylNR^{a}R^{a}, -OC₂₋₆alkylC(=O)NR^{a}R^{a}, -OC₂₋₆alkylOR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkylNR^{a}R^{a} or -NR^{a}C₂₋₆alkylOR^{a}.

8. A compound according to Claim 7 wherein
L is an unsubstituted 4-atom chain formed of C atoms;
R¹¹ is a phenyl, naphthylene, pyridine, pyrazine, triazine, quinoline, isoquinoline or thiophene;
R¹⁴ is a phenyl, naphthylene, pyridine, pyrazine, triazine, quinoline, isoquinoline, thiophene, pyrrole, pyrrolidine, furan, tetrahyrofuran, thiazole, oxazole, imidazole, piperidine, piperazine, morpholine, pyran, dioxane, cyclopropane, cyclobutane, cyclopentane or cyclohexane; and
R²¹ is phenyl, pyridine, pyrazine, triazine or thiophene.

9. A compound according to Claim 7 wherein
R^{c} is independently at each instance methyl, ethyl, propyl, isopropyl, n-butyl, sec-butyl, tert-butyl, pentyl, C₁₋₄haloalkyl, F, Cl, Br, cyano, nitro, -C₂₋₆alkylOR^{a}, -C₂₋₆alkyl C(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC₂₋₆alkylNR^{a}R^{a}, -OC₂₋₆alkylC(=O)NR^{a}R, -OC₂₋₆alkylOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkylNR^{a}R^{a} or -NR^{a}C₂₋₆alkylOR^{a}.

10. A compound according to Claim 1 having a Formula III 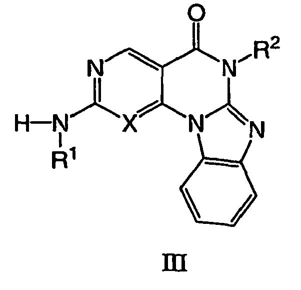 or a pharmaceutically-acceptable salt or derivative thereof, wherein
R¹ is selected from -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ and -R¹¹-R¹⁴-R¹³-R¹⁴, any of which is substituted by 0, 1, 2, 3, 4 or 5 substituents independently selected from R^{c};
R² is selected from -R²¹, -R²¹-R²², -R²¹-R²⁴ and -R²²-R²⁴, any of which is substituted by 0, 1, 2, 3, 4 or 5 substituents independently selected from R^{c};
R¹¹ is independently at each instance a phenyl, pyridine or pyrazine;
R¹² is independently at each instance C₁₋₈alkyl;
R ¹³ is independently at each instance -C(=O)-, -C(=0)0-. -C(=O)N(R^{a})-, -C(=NR^{a})N(R^{a})-, -O-, -OC(=O)N(R^{a})-, -OC₂₋₆alkylN(R²) -, -OC₂₋₆alkyl-, -OC₂₋₆alkylO-, -S-, -S(=O)-. -S(=O)₂-, -S(=O)₂N(R²) -, -S(=O)₂N(R^{a})C(=O)-, -N(R^{a}) -. -N(R^{a})C(=O)-, -N(R^{a})C(=O)O-, -N(R^{a})C(=O)N(R^{a})-, -N(R^{a})C(=NR^{a})N(R^{a}) -, -N(R^{a})S(=O)₂-, -N(R^{a})S(=O)₂N(Rₐ) -, -NR^{a}C₂₋₆alkylN(R^{a})-, or -NR^{a}C₂₋₆alkylO-;
R¹⁴ is phenyl, pyridine, pyrazine, thiophene, pyrrole, pyrrolidine, furan, tetrahyrofuran, piperidine, piperazine, morpholine, cyclopropane, cyclopentane or cyclohexane;
R ²¹ is phenyl, pyridine or pyrazine;
R²² is independently at each instance C₁₋₈alkyl;
R²³ is independently at each instance -C(=O)-, -C(=O)O-, -C(=O)N(R^{a})-, -C(=NR^{a})N(R^{a})-, -O-. -OC(=O)-, -OC(=O)N(R^{a})-, -OC(=O)N(R^{a})S(=O)₂-, -OC₂₋₆alkylN(R^{a}) -, -OC₂₋₆alkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a}) -, -N(R^{a})-,-N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a}) -, -NR^{a}C₂₋₆alkylN(R^{a})- or -NR^{a}C₂₋₆alkylO-;
R²⁴ is phenyl, pyridine, pyrazine, thiophene, pyrrole, pyrrolidine, furan, tetrahyrofuran, piperidine, piperazine, morpholine, cyclopropane, cyclopentane or cyclohexane;
R^{a} is independently at each instance H or R^{b};
R^{b} is independently at each instance C₁₋₈alkyl, CN, phenyl or benzyl; and
R^{c} is independently at each instance methyl, ethyl, propyl, isopropyl, n-butyl, sec-butyl, tert-butyl, pentyl, C₁₋₄haloalkyl, F, Cl, Br, cyano, nitro, -C₂₋₆alkyl OR^{a}, -C₂₋₆alkylC(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC₂₋₆alkylNR^{a}R^{a}, -OC₂₋₆alkylC(=O)NR^{a}R^{a}, -OC₂₋₆alkylOR^{a}, -SR^{a}, -S(=O)R^{b}, -S(=O)₂R^{b}, -S(=O)₂NR^{a}R^{a},-NR^{a}R^{a}, -N(R^{a})C(=O)R^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆akylNR^{a}R^{a} or -NR^{a}C₂₋₆alkylOR^{a}.

11. A compound according to Claim 1, wherein the compound is selected from:
1,1-dimethylethyl 4-(4-((6-(2,6-dimethylphenyl)-5-oxo-5,6-dihydropyfimido-[5',4':5,6]pyrimido[1,2-a]benzimidazol-2-yl)amino)phenyl)-1-piperazinecarboxylate;
2-((3,4-bis(methyloxy)-5-((2-(4-methyl-1-piperazinyl)ethyl)oxy)phenyl)amino)-6-(2,6-dimethylphenyl)pyrimido[5',4':5,6]pyrimido[1,2-a]benzimidazol-5(6H)-one;
2-((3,4-bis(methyloxy)-5-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-one;
2-((3,5-bis(methyloxy)-4-((2-(1-piperidinyl)ethyl)oxy)phenyl)amino)-6-(2,6-dimethylphenyl)pyrimido[5',4':5,6]pyrimido[1,2-a]benzimidazol-5(6H)-one;
2-((3,5-bis(methyloxy)-4-((2-(4-methyl-1-piperazinyl)ethyl)oxy)phenyl)amino)-6-(2,6-dimethylphenyl)pyrimido[5',4':5,6]pyrimido[1,2-a]benzimidazol-5(6H)-one;
2-((3,5-bis(methyloxy)-4-((2-(4-morpholinyl)ethyl)oxy)phenyl)amino)-6-(2,6-dimethylphenyl)pyrimido[5',4':5,6]pyrimido[1,2-a]benzimidazol-5(6H)-one;
2-((3,5-bis(methyloxy)-4-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-one;
2-((3-chloro-4-((2-((1-methylethyl)amino)ethyl)oxy)phenyl)amino)-6-(2,6-dimethylphenyl)pynmido[5',4':5,6]pynmido[l,2-a]benzimidazol-5(6H)-one;
2-((3-chloro-4-((2-(1-piperidinyl)ethyl)oxy)phenyl)amino)-6-(2,6-dimethylphenyl)-pyrimido[5',4':5,6]pyrimido[1,2-a]benzimidazol-5(6H)-one;
2-((3-chloro-4-((2-(4-(1-methylethyl)-1-piperazinyl)ethyl)oxy)phenyl)amino)-6-(2,6-dimethylphenyl)pyrimido[5',4':5,6]pyrimido[1,2-a]benziraidazol-5(6H)-one;
2-((3-chloro-4-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-one;
2-((3-chloro-4-(4-(1-methylethyl)-1-piperazinyl)phenyl)amino)-6-(2,6-dimethylphenyl)pyrimido[5',4':5,6]pyrimido[1,2-a]benzimidazol-5(6H)-one;
2-((3-chloro-4-(4-methyl-1-piperazinyl)phenyl)amino)-6-(2,6-dimethylphenyl)-pyrimido[5',4':5,6]pyrimido[1,2-a]benzimidazol-5(6H)-one;
2-((4-((2-(dimethylamino)ethyl)oxy)phenyl)amino)-6-(2,6-dimethylphenyl)-pyrimido[5',4':5,6]pyrimido[1,2-a]benzimidazol-5(6H)-one;
2-((4-((3-(diethylamino)propyl)oxy)-3-fluorophenyl)amino)-6-(2,6-dimethylphenyl)-pyrimido[5',4':5,6]pyrimido[1,2-a]benzimidazol-5(6H)-one;
2-((4-((3-(dimethylamino)propyl)oxy)phenyl)amino)-6-(2,6-dimethylphenyl)-pyrimido[5',4':5,6]pyrimido[1,2-a]benzimidazol-5(6H)-one;
2-((4-(1,4-diazepan-1-yl)phenyl)amino)-6-(2,6-dimethylphenyl)pyrimido [5.',4':5,6]-pyrimido[1,2-a]benzimidazol-5(6H)-one;
2-((4-(4-((3-chlorophenyl)methyl)-1-piperazinyl)phenyl)amino)-6-(2,6-dimethylphenyl)pyrimido[5',4':5,6]pyrimido[1,2-a]benzimidazol-5(6H)-one;
2-((4-(4-(2-(dimethylamino)ethyl)-1-piperazinyl)phenyl)amino)-6-(2,6-dimethylphenyl)pyrimido[5';4':5,6]pyrimido[1,2-a]benzimidazol-5(6H)-one;
2-((4-(4-(3-(dimethylamino)propyl)-1-piperazinyl)phenyl)amino)-6-(2,6-dimethylphenyl)pyrimido[5',4':5,6]pyrimido[1,2-a]benzimidazol-5(6H)-one;
2-((4-(4-(cyclohexylmethyl)-1-piperazinyl)phenyl)amino)-6-(2,6-dimethylphenyl)-pyrimido[5',4':5,6]pyrimido[1,2-a]benzimidazol-5(6H)-one;
2-((4-(4-methyl-1-piperazinyl)phenyl)amino)-6-((1R)-1-phenylethyl)pyrimido-[5',4':5,6]pyrimido[1,2-a]benzimidazol-5(6H)-one;
2-((4-(4-methyl-1-piperazinyl)phenyl)amino)-6-((1S)-1-phenylethyl)pyrimido-[5',4':5,6]pyrimido[1,2-a]benzimidazol-5(6H)-one;
2-((4-(4-methyl-1-piperazinyl)phenyl)amino)-6-(2,4,6-trichlorophenyl)pyrimido-[5',4':5,6]pyrimido[1,2-a]benzimidazol-5(6H)-one;
2-((4-(4-methyl-1-piperazinyl)phenyl)amino)-6-(pentafluorophenyl)pyrimido-[5',4':5,6]pyrimido[1,2-a]benzimidazol-5(6H)-one;
2-(4-(4-((6-(2,6-dimethylphenyl)-5-oxo-5,6-dihydropyrimido[5',4':5,6]pyrimido[1,2-a]benzimidazol-2-yl)amino)phenyl)-1-piperazinyl)acetamide;
3-((4-(4-((6-(2,6-dimethylphenyl)-5-oxo-5,6-dihydropyrimido[5',4':5,6]-pyrimido[1,2-a]benzimidazol-2-yl)amino)phenyl)-1-piperazinyl)methyl)-benzonitrile;
6-(2,6-dichlorophenyl)-2-((4-(4-methyl-1-piperazinyl)phenyl)amino)pyrimido-[5',4':5,6]pynmido[1,2-a]benzimidazol-5(6H)-one;
6-(2,6-difluorophenyl)-2-((4-(4-methyl-1-piperazinyl)phenyl)amino)pyrimido-[5',4':5,6]pyrimido[1,2-a]benzimidazol-5(6H)-one;
6-(2,6-dimethylphenyl)-2-((2-methyl-4-((3-(1-piperidinyl)propyl)oxy)phenyl)-amino)pyrimido[5',4':5,6]pyrimido[1,2-a]benzimidazol-5(6H)-one;
6-(2,6-dimethylphenyl)-2-((2-methyl-4-((3-(4-methyl-1-piperazinyl)propyl)oxy)-phenyl)amino)pyrimido[5',4':5,6]pyrimido[1,2-a]benzimidazol-5(6H)-one;
6-(2,6-dimethylphenyl)-2-((2-methyl-4-(4-methyl-1-piperazinyl)phenyl)amino)-pyrimido[5',4':5,6]pyrimido[1,2-a]benzimidazol-5(6H)-one;
6-(2,6-dimethylphenyl)-2-((3-((3-(1-piperidinyl)propyl)oxy)phenyl)amino)pyrimido-[5',4':5,6]pyrimido[1,2-a]benzimidazol-5(6H)-one;
6-(2,6-dimethylphenyl)-2-((3-((3-(4-methyl-1-piperazinyl)propyl)oxy)phenyl)-amino)pyrimido[5',4':5,6]pyrimido[1,2-a]benzimidazol-5(6H)-one;
6-(2,6-dimethylphenyl)-2-((3-(4-methyl-1-piperazinyl)phenyl)amino)pyrimido-[5',4':5,6]pyrimido[1,2-a]benzimidazol-5(6H)-one;
6-(2,6-dimethylphenyl)-2-((3-(methyloxy)-4-(((1-methyl-4-piperidinyl)methyl)oxy)-phenyl)amino)pyrimido[5',4':5,6]pyrimido[1,2-a]benzimidazol-5(6H)-one;
6-(2,6-dimethylphenyl)-2-((3-(methyloxy)-4-(((2R)-2-pyrrolidinylmethyl)oxy)-phenyl)amino)pyrimido[5',4':5,6]pyrimido[1,2-a]benzimidazol-5(6H)-one;
6-(2,6-dimethylphenyl)-2-((3-(methyloxy)-4-((2-(1-methyl-4-piperidinyl)ethyl)oxy)-phenyl)amino)pyrimido[5',4':5,6]pyrimido[1,2-a]benzimidazol-5(6H)-one;
6-(2,6-dimethylphenyl)-2-((3-(methyloxy)-4-((2-(4-methyl-1-piperazinyl)ethyl)oxy)-phenyl)amino)pyrimido[5',4':5,6]pyrimido[1,2-a]benzimidazol-5(6H)-one;
6-(2,6-dimethylphenyl)-2-((3-(methyloxy)-4-((3-(4-methyl-1-piperazinyl)propyl)-oxy)phenyl)amino)pyrimido[5',4':5,6]pyrimido[1,2-a]benzimidazol-5(6H)-one;
6-(2,6-dimethylphenyl)-2-((3-(methyloxy)-4-(1-piperazinyl)phenyl)amino)-pyrimido[5',4':5,6]pyrimido[1,2-a]benzimidazol-5(6H)-one;
6-(2,6-dimethylphenyl)-2-((3-(methyloxy)-4-(4-methyl-1-piperazinyl)phenyl)-amino)pyrimido[5',4':5,6]pyrimido[1,2-a]benzimidazol-5(6H)-one;
6-(2,6-dimethylphenyl)-2-((3,4,5-tris(methyloxy)phenyl)amino)pyrimido[5',4':5,6]-pyrimido[1,2-a]benzimidazol-5(6H)-one;
6-(2,6-dimethylphenyl)-2-((3-fluoro-4-((2-(1-piperidinyl)ethyl)oxy)phenyl)amino)-pyrimido[5',4':5,6]pyrimido[1,2-a]benzimidazol-5(6H)-one;
6-(2,6-dimethylphenyl)-2-((3-fluoro-4-((2-(4-(l-methylethyl)-1-piperazinyl)ethyl)-oxy)phenyl)amino)pyrimido[5',4':5,6]pyrimido[1,2-a]benzimidazol-5(6H)-one;
6-(2,6-dimethylphenyl)-2-((3-fluoro-4-((2-(4-methyl-1-piperazinyl)ethyl)oxy)-phenyl)amino)pyrimido[5',4':5,6]pyrimido[1,2-a]benzimidazol-5(6H)-one;
6-(2,6-dimethylphenyl)-2-((3-fluoro-4-((3-(1-piperidinyl)propyl)oxy)phenyl)amino)-pyrimido[5',4':5,6]pyrimido[1,2-a]benzimidazol-5(6H)-one;
6-(2,6-dimethylphenyl)-2-((3-fluoro-4-((3-(4-(1-methylethyl)-1-piperazinyl)propyl)-oxy)phenyl)amino)pyrimido[5,4':5,6]pyrimido[1,2-a]benzimidazol-5(6H)-one;
6-(2,6-dimethylphenyl)-2-((3-fluoro-4-((3-(4-methyl-1-piperazinyl)propyl)oxy)-phenyl)amino)pyrimido[5',4':5,6]pyrimido[1,2-a]benzimidazol-5(6H)-one;
6-(2,6-dimethylphenyl)-2-((3-fluoro-4-(4-(1-methylethyl)-1-piperazinyl)phenyl)-amino)pyrimido[5',4':5,6]pyrimido[1,2-a]benzimidazol-5(6H)-one;
6-(2,6-dimethylphenyl)-2-((3-fluoro-4-(4-methyl-1-piperazinyl)phenyl)amino)-pyrimido[5',4':5,6]pyrimido[1,2-a]benzimidazol-5(6H)-one;
6-(2,6-dimethylphenyl)-2-((4-((((2S)-1-methyl-2-pyrrolidinyl)methyl)oxy)phenyl)-amino)pyrimido[5',4':5,6]pyrimido[1,2-a]benzimidazol-5(6H)-one;
6-(2,6-dimethylphenyl)-2-((4-(((2R)-2-pyrrolidinylmethyl)oxy)phenyl)amino)-pyrimido[5',4':5,6]pyrimido[1,2-a]benzimidazol-5(6H)-one;
6-(2,6-dimethylphenyl)-2-((4-(((2S)-2-pyrrolidinylmethyl)oxy)phenyl)amino)-pyrimido[5',4':5,6]pyrimido[1,2-a]benzimidazol-5(6H)-one;
6-(2,6-dimethylphenyl)-2-((4-((2-((1-methylethyl)amino)ethyl)oxy)-3-(methyloxy)-phenyl)amino)pyrimido[5',4':5,6]pyrimido[1,2-a]benzimidazol-5(6H)-one;
6-(2,6-dimethylphenyl)-2-((4-((2-((1-methylethyl)amino)ethyl)oxy)phenyl)amino)-pyrimido[5',4';5,6]pyrimido[1,2-a]benzimidazol-5(6H)-one;
6-(2,6-dimethylphenyl)-2-((4-((2-(1-methyl-4-piperidinyl)ethyl)oxy)phenyl)amino)-pyrimido[5',4':5,6]pyrimido[1,2-a]benzimidazol-5(6H)-one;
6-(2,6-dimethylphenyl)-2-((4-((2-(1-piperidinyl)ethyl)oxy)phenyl)amino)-pyrimido[5',4':5,6]pyrimido[1,2-a]benzimidazol-5(6H)-one;
6-(2,6-dimethylphenyl)-2-((4-((2-(4-(1-methylethyl)-1-piperazinyl)ethyl)oxy)-3-(methyloxy)phenyl)amino)pyrimido[5',4':5,6]pyrimido[1,2-a]benzimidazol-5(6H)-one;
6-(2,6-dimethylphenyl)-2-((4-((2-(4-methyl-1-piperazinyl)ethyl)oxy)phenyl)amino)-pyrimido[5',4':5,6]pyrimido[1,2-a]benzimidazol-5(6H)-one;
6-(2,6-dimethylphenyl)-2-((4-((2-(4-morpholinyl)ethyl)oxy)phenyl)amino)-pyrimido [5',4':5,6]pyrimido[1,2-a]benzimidazol-5(6H)-one;
6-(2,6-dimethylphenyl)-2-((4-((2-(methylamino)ethyl)oxy)phenyl)amino)-pyrimido[5',4':5,6]pyrimido[1,2-a]benzimidazol-5(6H)-one;
6-(2,6-dimethylphenyl)-2-((4-((3-(1-piperidinyl)propyl)oxy)phenyl)amino)-pyrimido[5',4':5,6]pyrimido[1,2-a]benzimidazol-5(6H)-one;
6-(2,6-dimethylphenyl)-2-((4-((3-(4-(1-methylethyl)-1-piperazinyl)propyl)oxy)-3-(methyloxy)phenyl)amino)pyrimido[5',4':5,6]pyrimido[1,2-a]benzimidazol-5(6H)-one;
6-(2,6-dimethylphenyl)-2-((4-(1-piperazinyl)phenyl)amino)pyrimido[5',4':5,6]-pyrimido[1,2-a]benzimidazol-5(6H)-one;
6-(2,6-dimethylphenyl)-2-((4-(4-((3-(methyloxy)phenyl)methyl)-1-piperazinyl)-phenyl)amino)pyrimido[5',4':5,6]pyrimido[1,2-a]benzimidazol-5(6H)-one;
6-(2,6-dimethylphenyl)-2-((4-(4-(1-methylethyl)-1-piperazinyl)phenyl)amino)-pyrimido[5',4':5,6]pyrimido[1,2-a]benzimidazol-5(6H)-one;
6-(2,6-dimethylphenyl)-2-((4-(4-(1-methylethyl)-1-piperazinyl)-3-(methyloxy)-phenyl)amino)pyrimido[5',4':5,6]pyrimido[1,2-a]benzimidazol-5(6H)-one;
6-(2,6-dimethylphenyl)-2-((4-(4-(2-(methyloxy)ethyl)-1-piperazinyl)phenyl)amino)-pyrimido[5',4':5,6]pyrimido[1,2-a]benzimidazol-5(6H)-one;
6-(2,6-dimethylphenyl)-2-((4-(4-(2-hydroxyethyl)-1-piperazinyl)phenyl)amino)-pyrimido[5',4':5,6]pyrimido[1,2-a]benzimidazol-5(6H)-one;
6-(2,6-dimethylphenyl)-2-((4-(4-methyl-1,4-diazepan-1-yl)phenyl)amino)-pyrimido[5',4':5,6]pyrimido[1,2-a]benzimidazol-5(6H)-one;
6-(2,6-dimethylphenyl)-2-((4-(4-methyl-1-piperazinyl)phenyl)amino)-pyrimido[5',4':5,6]pyrimido[1,2-a]benzimidazol-5(6H)-one;
6-(2,6-dimethylphenyl)-2-((4-(4-methyl-1-piperazinyl)phenyl)amino)-8,9-dihydroimidazo[1,2-a]pyrimido[5,4-e]pyrimidin-5(6H)-one;
6-(2,6-dimethylphenyl)-2-((4-(4-methyl-1-piperazinyl)phenyl)amino)imidazo[1,2-a]pyrimido[5,4-e]pyrimidin-5(6H)-one;
6-(2-fluorophenyl)-2-((4-(4-methyl-1-piperazinyl)phenyl)amino)pyrimido[5',4':5,6]-pyrimido[1,2-a]benzimidazol-5(6H)-one;
6-(3-(methyloxy)phenyl)-2-((4-(4-methyl-1-piperazinyl)phenyl)amino)pyrimido-[5',4':5,6]pyrimido[1,2-a]benzimidazol-5(6H)-one;
6-(3-fluorophenyl)-2-((4-(4-methyl-1-piperazinyl)phenyl)amino)pyrimido[5',4':5,6]-pyrimido[1,2-a]benzimidazol-5(6H)-one;
6-(3-methylphenyl)-2-((4-(4-methyl-1-piperazinyl)phenyl)amino)-pyrimido[5',4':5,6]pyrimido[1,2-a]benzimidazol-5(6H)-one;
6-(4-(methyloxy)phenyl)-2-((4-(4-methyl-1-piperazinyl)phenyl)amino)pyrimido-[5',4':5,6]pyrimido[1,2-a]benzimidazol-5(6H)-one;
6-(4-fluorophenyl)-2-((4-(4-methyl-1-piperazinyl)phenyl)amino)pyrimido[5',4':5,6]-pyrimido[1,2-a]benzimidazol-5(6H)-one;
6-(4-methylphenyl)-2-((4-(4-methyl-1-piperazinyl)phenyl)amino)-pyrimido[5',4':5,6]pyrimido[1,2-a]benzimidazol-5(6H)-one;
6-(5-chloro-2-(methyloxy)phenyl)-2-((4-(4-methyl-1-piperazinyl)phenyl)amino)-pyrimido[5',4':5,6]pyrimido [1,2-a]benzimidazol-5(6H)-one;
N-(2-(diethylamino)ethyl)-3-((6-(2,6-dimethylphenyl)-5-oxo-5,6-dihydropyrimido-[5',4':5,6]pyrimido[1,2-a]benzimidazol-2-yl)amino)-N-methylbenzamide;
N-(2-(diethylamino)ethyl)-4-((6-(2,6-dimethylphenyl)-5-oxo-5,6-dihydropyrimido-[5',4':5,6]pyrimido[1,2-a]benzimidazol-2-yl)amino)-N-methylbenzamide;
N-(2-(dimethylamino)ethyl)-3-((6-(2,6-dimethylphenyl)-5-oxo-5,6-dihydropyrimido[5',4':5,6]pyrimido[1,2-a]benzimidazol-2-yl)amino)benzamide; and
N-(2-(dimethylamino)ethyl)-4-((6-(2,6-dimethylphenyl)-5-oxo-5,6-dihydropyrimido[5',4':5,6]pyrimido[1,2-a]benzimidazol-2-yl)amino)benzamide.

12. A method of making a compound according to Claim 1, the method comprising the steps of: reacting a pyrimidine ester (A) 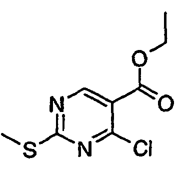 with a benzimidazole (B) 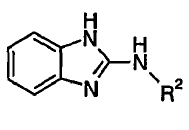 in the presence of a base to form a tetracyclic pyrimidine (C) 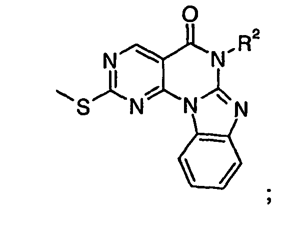 oxidizing the sulfur of the tetracyclic pyrimidine (C) to form a tetracyclic pyrimidine sulfone (D) 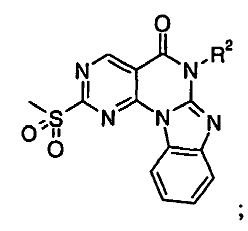 and
reacting the tetracyclic pyrimidine sulfone (D) with R¹NH to make a
compound 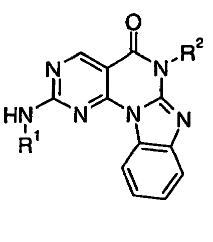 according to Claim 1.

13. A pharmaceutical composition comprising a compound according to any of Claims 1-11 and a pharmaceutically acceptable carrier.

14. Use of a compound according to any of Claims 1-11 for the preparation of a medicament for the treatment of inflammation.

15. Use of a compound according to any of Claims 1-11 for the preparation of a medicament for the inhibition of T cell activation and proliferation in a mammal.

16. Use of a compound according to any of Claims I-II for the preparation of a medicament for the treatment of arthritis, rheumatoid arthritis, psoriatic arthritis, or osteoarthritis in a mammal.

17. Use of a compound according to any of Claims 1-11 for the preparation of a medicament for the treatment of organ transplant, acute transplant or heterograft or homograft rejection, or transplantation tolerance induction in a mammal.

18. Use of a compound according to any of Claims 1-11 for the preparation of a medicament for the treatment of ischemic or reperfusion injury, myocardial infarction, or stroke in a mammal.

19. Use of a compound according to any of Claims 1-11 for the preparation of a medicament for the treatment of multiple sclerosis, inflammatory bowel disease, including ulcerative colitis, Crohn's disease, lupus, contact hypersensitivity, delayed-type hypersensitivity, and gluten-sensitive enteropathy, type 1 diabetes, psoriasis, contact dermatitis, Hashimoto's thyroiditis, Sjogren's syndrome, autoimmune hyperthyroidism, Addison's disease, autoimmune polyglandular disease, autoimmune alopecia, pernicious anemia, vitiligo, autoimmune hypopituatarism. Guillain-Barre syndrome, glomerulonephritis, serum sickness, uticaria, allergic diseases, asthma, hayfever, allergic rhinitis, mycosis fungoides, dermatomyositis, alopecia areata, chronic actinic dermatitis, eczema, Behcet's disease, Pustulosis palmoplanteris, Pyoderma gangrenum, Sezary's syndrome, atopic dermatitis, systemic schlerosis, morphea or atopic dermatitis in a mammal.

20. Use of a compound according to any of Claims 1-11 for the preparation of a medicament for the treatment of colon carcinoma or thymoma in a mammal.

## Patentansprüche

1. Verbindung der Formel I 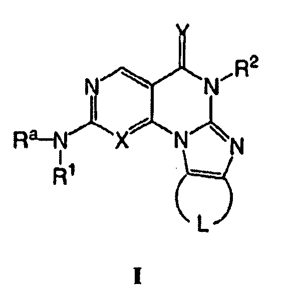 oder ein pharmazeutisch annehmbares Salz oder Derivat davon, wobei
X N oder CH ist;
Y NH, N(CN), O oder S ist;
L eine aus C- und N-Atomen gebildete 4-Atome-Kette ist, wobei die Kette durch 0 oder 1 R³-Gruppen substituiert ist und die Kette zusätzlich durch 0, 1, 2 oder 3 Substituenten substituiert ist, die unabhängig voneinander ausgewählt sind aus R^{c};
R¹ ausgewählt ist aus -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴ -R¹²-R¹³-R¹⁴ -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ und -R¹¹-R¹⁴-R¹³-R¹⁴, von denen jedes durch 0, 1, 2, 3, 4 oder 5 Substituenten substituiert ist, die unabhängig voneinander ausgewählt sind aus R^{c};
R² ausgewählt ist aus -R²¹, -R²¹-R²², -R²¹-R²⁴, -R²²-R²⁴, -R²¹-R²²-R²⁴, -R²¹-R²³ -R²⁴ , -R²²-R²³-R²⁴, -R²¹-R²³-R²²-R²⁴ und -R²¹-R²²-R²³-R²⁴, von denen jedes durch 0, 1, 2, 3, 4 oder 5 Substituenten substituiert ist, die unabhängig voneinander ausgewählt sind aus R^{c};
R³ ausgewählt ist aus -R³², -R³⁴, -R³²-R³⁴, -R³³-R³⁴, -R³³-R³²-R³⁴ und -R³²-R³³-R³⁴, von denen jedes durch 0, 1, 2, 3, 4 oder 5 Substituenten substituiert ist, die unabhängig voneinander ausgewählt sind aus R^{c};
R¹¹ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, 0 und S, enthält, sofern die Kombination von 0- und S-Atomen nicht größer als 2 ist, wobei die Kohlenstoffatome des Rings durch 0, 1 oder 2 Oxogruppen substituiert sind;
R¹² unabhängig in jedem Fall C₁₋₈-Alkyl ist;
R¹³ unabhängig in jedem Fall -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -OC₂₋₆-AlkylN(R^{a})-, -OC₂₋₆-Alkyl-, -OC₂₋₆-AlkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆-AlkylN(R^{a})- oder -NR^{a}C₂₋₆-AlkylO- ist;
R¹⁴ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R ²¹ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R²² unabhängig in jedem Fall C₁₋₈-Alkyl ist;
R²³ unabhängig in jedem Fall -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -OC₂₋₆-AlkylN(R^{a})-, -OC₂₋₆-AlkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a} C₂₋₆-AlkylN(R^{a})- oder -NR^{a}C₂₋₆-AlkylO- ist;
R²⁴ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R³² unabhängig in jedem Fall C₁₋₈-Alkyl ist;
R³³ unabhängig in jedem Fall -C(=O)-, -C(=O)O-, -C(=O)N(R^{a})-, -C(=NR^{a})N(R^{a})-, -O-,-OC(=O)-, -OC(=O)N(R^{a})-, -OC(=O)N(R^{a})S(=O)₂-, -OC₂₋₆-AlkylN(R^{a})-, -OC₂₋₆-AlkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆-AlkylN(R^{a})- oder -NR^{a}C₂₋₆-AlkylO- ist;
R³⁴ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R^{a} unabhängig in jedem Fall H oder R^{b} ist;
R^{b} unabhängig in jedem Fall C₁₋₈-Alkyl, CN, Phenyl oder Benzyl ist; und
R^{c} unabhängig in jedem Fall C₁₋₈-Alkyl, C₁₋₄-Halogenalkyl, Halogen, Cyano, Nitro, -C₂₋₆-AlkylOR^{a}, -C₂₋₆-AlkylC(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -OC₂₋₆-AlkylNR^{a}R^{a}, -OC₂₋₆-AlkylC(=O)NR^{a}R^{a}, -OC₂₋₆-AlkylOR , -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆-AlkylNR^{a}R^{a} oder -NR^{a}C₂₋₆-AlkylOR^{a} ist.

2. Verbindung nach Anspruch 1, wobei X N ist und Y O ist.

3. Verbindung nach Anspruch 1, wobei L eine aus C- und N-Atomen gebildete 4-Atome-Kette ist, wobei die Kette durch 0 oder 1 R³-Gruppen substituiert ist und die Kette zusätzlich durch 0, 1, 2 oder 3 Substituenten substituiert ist, die unabhängig voneinander ausgewählt sind aus R^{c}.

4. Verbindung nach Anspruch 1, wobei R¹¹ Phenyl, Naphthylen, Pyridin, Pyrazin, Triazin, Chinolin, Isochinolin, Thiophen, Pyrrol, Pyrrolidin, Furan, Tetrahydrofuran, Thiazol, Oxazol, Imidazol, Piperidin, Piperazin oder Morpholin ist.

5. Verbindung nach Anspruch 1, wobei R²¹ Phenyl, Naphthylen, Pyridin, Pyrazin, Triazin, Chinolin, Isochinolin, Thiophen, Pyrrol, Pyrrolidin, Furan, Tetrahydrofuran, Thiazol, Oxazol, Imidazol, Piperidin, Piperazin oder Morpholin ist.

6. Verbindung nach Anspruch 1 der Formel II 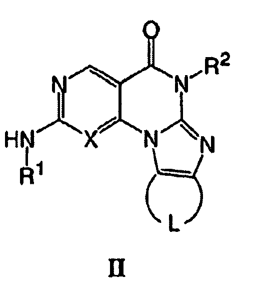 oder ein pharmazeutisch annehmbares Salz davon, wobei
X N oder CH ist;
L eine aus C- und N-Atomen gebildete 3- oder 4-Atome-Kette ist, wobei die Kette durch 0 oder 1 R³-Gruppen substituiert ist und die Kette zusätzlich durch 0, 1, 2 oder 3 Substituenten substituiert ist, die unabhängig voneinander ausgewählt sind aus R^{c};
R¹ ausgewählt ist aus -R¹¹, -R¹¹-R¹², -R¹¹-R¹⁴ -R¹²-R¹⁴, -R¹¹-R¹²-R¹⁴, -R¹¹-R¹³-R¹⁴, -R¹²-R¹³-R¹⁴, -R¹¹-R¹³-R¹²-R¹⁴ und -R¹¹-R¹²-R¹³-R¹⁴, von denen jedes durch 0, 1, 2, 3 oder 4 Substituenten substituiert ist, die unabhängig voneinander ausgewählt sind aus R^{c};
R² ausgewählt ist aus -R²¹, -R²¹-R²², -R²¹-R²⁴, -R²²-R²⁴, -R²¹-R²²-R²⁴, R²¹-R²³-R²⁴, -R²²-R²³-R²⁴, -R²¹-R²³-R²²-R²⁴ und -R²¹-R²²-R²³-R²⁴, von denen jedes durch 0, 1, 2, 3 oder 4 Substituenten substituiert ist, die unabhängig voneinander ausgewählt sind aus R^{c};
R³ ausgewählt ist aus -R³², -R³⁴, -R³²-R³⁴, -R³³-R³⁴, -R³³-R³²-R³⁴, und -R³²-R³³-R³⁴, von denen jedes durch 0, 1, 2, 3 oder 4 Substituenten substituiert ist, die unabhängig voneinander ausgewählt sind aus R^{c};
R¹¹ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R¹² unabhängig in jedem Fall C₁₋₈-Alkyl ist;
R¹³ unabhängig in jedem Fall -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆-AlkylNR^{a}-, -OC₂₋₆-AlkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a} C₂₋₆-AlkylN(R^{a})- oder -NR^{a}C₂₋₆-AlkylO- ist;
R¹⁴ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R²¹ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R²² unabhängig in jedem Fall C₁₋₈-Alkyl ist;
R²³ unabhängig in jedem Fall -C(=O)-, -C(=O)O-, -C(=O)NR^{a}-, -C(=NR^{a})NR^{a}, -O-, -OC(=O)-, -OC(=O)NR^{a}-, -OC(=O)N(R^{a})S(=O)₂-, -OC₂₋₆-AlkylNR^{a}-, -OC₂₋₆-AlkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆-AlkylN(R^{a})- oder -NR^{a}C₂₋₆-AlkylO-ist;
R²⁴ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R³² unabhängig in jedem Fall C₁₋₈-Alkyl ist;
R³³ unabhängig in jedem Fall -C(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -OC₂₋₆-AlkylNR^{a}-, -OC₂₋₆-AlkylO-, -S-, -S(=O)-, -S(=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆-AlkylN(R^{a})- oder -NR^{a}C₂₋₆-AlkylO- ist;
R³⁴ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R^{a} unabhängig in jedem Fall H oder R^{b} ist;
R^{b} unabhängig in jedem Fall C₁₋₈-Alkyl, Phenyl oder Benzyl ist; und
R^{c} unabhängig in jedem Fall C₁₋₈-Alkyl, C₁₋₄-Halogenalkyl, Halogen, Cyano, Nitro -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -OC₂₋₆-AlkylNR^{a}R^{a}, -OC₂₋₆-AlkylOR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b} -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆-AlkylNR^{a}R^{a} oder -NR^{a}C₂₋₆-AlkylOR^{a} ist.

7. Verbindung nach Anspruch 1, wobei
X N ist;
Y O ist;
L eine aus C-Atomen gebildete 4-Atome-Kette ist, wobei die Kette durch 0 oder 1 R³-Gruppen substituiert ist und die Kette zusätzlich durch 0, 1, 2 oder 3 Substituenten substituiert ist, die unabhängig voneinander ausgewählt sind aus R^{c};
R¹ ausgewählt ist aus -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ und -R¹¹-R¹⁴-R¹³-R¹⁴, von denen jedes durch 0, 1, 2, 3, 4 oder 5 Substituenten substituiert ist, die unabhängig voneinander ausgewählt sind aus R°;
R² ausgewählt ist aus -R²¹, -R²¹-R²², -R²¹-R²⁴, -R²²-R²⁴, -R²¹-R²²-R²⁴, -R²¹-R²³-R²⁴ und -R²²-R²³-R²⁴, von denen jedes durch 0, 1, 2, 3, 4 oder 5 Substituenten substituiert ist, die unabhängig voneinander ausgewählt sind aus R^{c};
R³ ausgewählt ist aus -R³², -R³⁴ -R³²-R³⁴, -R³³-R³⁴ -R³³-R³²-R³⁴ und -R³²-R³³-R³⁴, von denen jedes durch 0, 1, 2, 3, 4 oder 5 Substituenten substituiert ist, die unabhängig voneinander ausgewählt sind aus R^{c};
R¹¹ unabhängig in jedem Fall ein Phenyl, Naphthylen, Pyridin, Pyrazin, Triazin, Chinolin, Isochinolin, Thiophen, Pyrrol, Pyrrolidin, Furan, Tetrahydrofuran, Thiazol, Oxazol, Imidazol, Piperidin, Piperazin oder Morpholin ist;
R¹² unabhängig in jedem Fall C₁₋₈-Alkyl ist;
R¹³ unabhängig in jedem Fall-C(=O)-, -C(=O)O-, -C(=O)N(R^{a})-, -C(=NR^{a})N(R^{a})-, -O-, -OC(=O)N(R^{a})-, -OC₂₋₆-AlkylN(R^{a})₂-, -OC₂₋₆-Alkyl-, -OC₂₋₆-AlkylO-, -S-, -S(=O)-, -S(=O)₂-, -S(=O)₂N(R^{a})-, -S(=O)₂N(R^{a})C(=O)-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆-AlkylN(R^{a})- oder -NR^{a}C₂₋₆-AlkylO- ist;
R¹⁴ unabhängig in jedem Fall ein gesättigter oder ungesättigter 5-, 6- oder 7-gliedriger monocyclischer oder 6-, 7-, 8-, 9-, 10- oder 11-gliedriger bicyclischer Ring ist, der 0, 1, 2, 3 oder 4 Atome, ausgewählt aus N, O und S, enthält, sofern die Kombination von O- und S-Atomen nicht größer als 2 ist, wobei die Kohlenstoffatome des Rings durch 0, 1 oder 2 Oxogruppen substituiert sind;
R²¹ unabhängig in jedem Fall ein Phenyl, Naphthylen, Pyridin, Pyrazin, Triazin, Chinolin, Isochinolin, Thiophen, Pyrrol, Pyrrolidin, Furan, Tetrahydrofuran, Thiazol, Oxazol, Imidazol, Piperidin, Piperazin oder Morpholin ist;
R²² unabhängig in jedem Fall C₁₋₈-Alkyl ist;
R²³ unabhängig in jedem Fall -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -OC₂₋₆-AlkylN(R^{a})-, -OC₂₋₆-AlkylO-, -S-, -S(=O)-, -S(=O)₂-, -S(=O)₂N(R^{a})-, -S(=O)₂N(R^{a})C(=O)-, -S(=O)₂N(R^{a})C(=O)O-, -S(=O)₂N(R^{a})C(=O)N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆-AlkylN(R^{a})- oder -NR^{a}C₂₋₆-AlkylO- ist;
R²⁴ unabhängig in jedem Fall ein Phenyl, Naphthylen, Pyridin, Pyrazin, Triazin, Chinolin, Isochinolin, Thiophen, Pyrrol, Pyrrolidin, Furan, Tetrahydrofuran, Thiazol, Oxazol, Imidazol, Piperidin, Piperazin, Morpholin, Pyran, Dioxan, Cyclopropan, Cyclobutan, Cyclopentan oder Cyclohexan ist;
R³² unabhängig in jedem Fall C₁₋₈-Alkyl ist;
R³³ unabhängig in jedem Fall -C(=O)-, -C(=O)O-, -C(=O)N(R^{a})-, -C(=NR^{a})N(R^{a})-, -O-, -OC₂₋₆-AlkylN(R^{a})-, -OC₂₋₆-AlkylO-, -S-, -S(=O)-, -S(=O)₂-, -S(=O)₂N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆-AlkylN(R^{a})- oder -NR^{a}C₂₋₆-AlkylO- ist;
R³⁴ unabhängig in jedem Fall ein Phenyl, Naphthylen, Pyridin, Pyrazin, Triazin, Chinolin, Isochinolin, Thiophen, Pyrrol, Pyrrolidin, Furan, Tetrahydrofuran, Thiazol, Oxazol, Imidazol, Piperidin, Piperazin, Morpholin, Pyran, Dioxan, Cyclopropan, Cyclobutan, Cyclopentan oder Cyclohexan ist;
R^{a} unabhängig in jedem Fall H oder R^{b} ist;
R^{b} unabhängig in jedem Fall C₁₋₈-Alkyl, CN, Phenyl oder Benzyl ist; und
R^{C} unabhängig in jedem Fall C₁₋₈-Alkyl, C₁₋₄-Halogenalkyl, Halogen, Cyano, Nitro, -C₂₋₆-AlkylOR^{a}, -C₂₋₆-AlkylC(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -OC₂₋₆-AlkylNR^{a}R^{a}, -OC₂₋₆-AlkylC(=O)NR^{a}R^{a}, -OC₂₋₆-AlkylOR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆-AlkylNR^{a}R^{a} oder -NR^{a}C₂₋₆-AlkylOR^{a} ist.

8. Verbindung nach Anspruch 7, wobei
L eine aus C-Atomen gebildete unsubstituierte 4-Atome-Kette ist;
R¹¹ ein Phenyl, Naphthylen, Pyridin, Pyrazin, Triazin, Chinolin, Isochinolin oder Thiophen ist;
R¹⁴ ein Phenyl, Naphthylen, Pyridin, Pyrazin, Triazin, Chinolin, Isochinolin, Thiophen, Pyrrol, Pyrrolidin, Furan, Tetrahydrofuran, Thiazol, Oxazol, Imidazol, Piperidin, Piperazin, Morpholin, Pyran, Dioxan, Cyclopropan, Cyclobutan, Cyclopentan oder Cyclohexan ist; und
R²¹ Phenyl, Pyridin, Pyrazin, Triazin oder Thiophen ist.

9. Verbindung nach Anspruch 7, wobei
R^{c} unabhängig in jedem Fall Methyl, Ethyl, Propyl, Isopropyl, n-Butyl, sec-Butyl, tert-Butyl, Pentyl, C₁₋₄-Halogenalkyl, F, Cl, Br, Cyano, Nitro, -C₂₋₆-AlkylOR^{a}, -C₂₋₆-AlkylC(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC₂₋₆-AlkylNR^{a}R^{a}, -OC₂₋₆-AlkylC(=O)NR^{a}R^{a}, -OC₂₋₆-AlkylOR^{a}, -SR^{a}, -S(=O)R^{b}. -S(=O)₂R^{b}, -S(=O)₂NR^{a}R^{a}, -NR^{a}R^{a}, -N(R^{a})C(=O)R^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆-AlkylNR^{a}R^{a} oder -NR^{a}C₂₋₆-AlkylOR^{a} ist.

10. Verbindung nach Anspruch 1 mit der Formel III 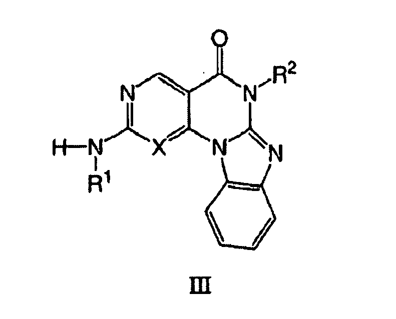 oder ein pharmazeutisch annehmbares Salz oder Derivat davon, wobei
R¹ ausgewählt ist aus -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ und -R¹¹-R¹⁴-R¹³-R¹⁴, von denen jedes durch 0, 1, 2, 3, 4 oder 5 Substituenten substituiert ist, die unabhängig ausgewählt sind aus R^{c};
R² ausgewählt ist aus -R²¹, -R²¹-R²², -R²¹-R²⁴ und -R²²-R²⁴, von denen jedes durch 0, 1, 2, 3, 4 oder 5 Substituenten substituiert ist, die unabhängig ausgewählt sind aus R^{c};
R¹¹ unabhängig in jedem Fall ein Phenyl, Pyridin oder Pyrazin ist;
R¹² unabhängig in jedem Fall C₁₋₈-Alkyl ist;
R¹³ unabhängig in jedem Fall -C(=O)-, -C(=O)O-, -C(=O)N(R^{a})-, -C(=NR^{a})N(R^{a})-, -O-, -OC(=O)N(R^{a})-, -OC₂₋₆-AlkylN(R^{a})-, -OC₂₋₆-Alkyl-, -OC₂₋₆-AlkylO-, -S-, -S(=O)-, -S(=O)₂-, -S(=O)₂N(R^{a})-, -S(=O)₂N(R^{a})C(=O)-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a}) S(=O)₂N(R^{a})-, -NR^{a}C₂₋₆-AlkylN(R^{a})- oder -NR^{a}C₂₋₆-AlkylO- ist;
R¹⁴ Phenyl, Pyridin, Pyrazin, Thiophen, Pyrrol, Pyrrolidin, Furan, Tetrahydrofuran, Piperidin, Piperazin, Morpholin, Cyclopropan, Cyclopentan oder Cyclohexan ist;
R²¹ Phenyl, Pyridin oder Pyrazin ist;
R²² unabhängig in jedem Fall C₁₋₈Alkyl ist;
R²³ unabhängig in jedem Fall -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)2-, -OC₂₋₆-AlkylN(R^{a})-, -OC₂₋₆-AlkylO-, -S-, -S(=O)-, -S(=O)₂-, -S(=O)₂N(R^{a})-, -S(=O)₂N(R^{a})C(=O), -S(=O)₂N(R^{a})C(=O)O-, -S(=O)₂N(R^{a})C(=O)N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}C₂₋₆-AlkylN(R^{a})- oder -NR^{a}C₂₋₆-AlkylO- ist;
R²⁴ Phenyl, Pyridin, Pyrazin, Thiophen, Pyrrol, Pyrrolidin, Furan, Tetrahydrofuran, Piperidin, Piperazin, Morpholin, Cyclopropan, Cyclopentan oder Cyclohexan ist;
R^{a} unabhängig in jedem Fall H oder R^{b} ist;
R^{b} unabhängig in jedem Fall C₁₋₈-Alkyl, CN, Phenyl oder Benzyl ist; und
R^{c} unabhängig in jedem Fall Methyl, Ethyl, Propyl, Isopropyl, n-Butyl, sec-Butyl, tert-Butyl, Pentyl, C₁₋₄-Halogenalkyl, F, Cl, Br, Cyano, Nitro, -C₂₋₆-AlkylOR^{a}, -C₂₋₆-AlkylC(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC₂₋₆-AlkylNR^{a}R^{a}, -OC₂₋₆-AlkylC(=O)NR^{a}R^{a}, -OC₂₋₆AlkylOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆-AlkylNR^{a}R^{a} oder -NR^{a}C₂₋₆-AlkylOR^{a} ist.

11. Verbindung nach Anspruch 1, wobei die Verbindung ausgewählt ist aus:
1, 1-Dimethylethyl-4-(4-((6-(2,6-dimethylphenyl)-5-oxo-5,6-dihydropyrimido-[5',4':5,6]pyrimido[1,2-a]benzimidazol-2-yl)amino)phenyl)-1-piperazincarboxylat;
2-((3,4-Bis(methyloxy)-5-((2-(4-methyl-1-piperazinyl)ethyl)oxy)phenyl)amino)-6-(2,6-dimethylphenyl)pyrimido[5',4':5,6]pyrimido[1,2-a]benzimidazol-5(6H)-on;
2-((3,4-Bis(methyloxy)-5-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-on;
2-((3,5-Bis(methyloxy)-4-((2-(1-piperidinyl)ethyl)oxy)phenyl)amino)-6-(2,6-dimethylphenyl)pyrimido[5',4':5,6]pyrimido[1,2-a]benzimidazol-5(6H)-on;
2-((3,5-Bis(methyloxy)-4-((2-(4-methyl-1-piperazinyl)ethyl)oxy)phenyl)amino)-6-(2,6-dimethylphenyl)pyrimido[5',4':5,6]pyrimido[1,2-a]benzimidazol-5(6H)-on;
2-((3,5-Bis(methyloxy)-4-((2-(4-morpholinyl)ethyl)oxy)phenyl)amino)-6-(2,6-dimethylphenyl)pyrimido[5',4':5,6]pyrimido[1,2-a]benzimidazol-5(6H)-on;
2-((3,5-Bis(methyloxy)-4-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-on;
2-((3-Chlor-4-((2-((1-methylethyl)amino)ethyl)oxy)phenyl)amino)-6-(2,6-dimethylphenyl)pyrimido[5',4':5,6]pyrimido[1,2-a]benzimidazol-5(6H)-on;
2-((3-Chlor-4-((2-(1-piperidinyl)ethyl)oxy)phenyl)amino)-6-(2,6-dimethylphenyl)-pyrimido[5',4':5,6]pyrimido[1,2-a]benzimidazol-5(6H)-on;
2-((3-Chlor-4-((2-(4-( 1-methylethyl)-1-piperazinyl)ethyl)oxy)phenyl)amino)-6-(2,6-dimethylphenyl)pyrimido[5',4':5,6]pyrimido[1,2-a]benzimidazol-5(6H)-on;
2-((3-Chlor-4-((3-(4-methyl-1-piperazinyl)propyl)oxy)phenyl)amino)-6-(2,6-dimethylphenyl)pyrimido[5',4':5,6]pyrimido[1,2-a]benzimidazol-5(6H)-on;
2-((3-Chlor-4-(4-(1-methylethyl)-1-piperazinyl)phenyl)amino)-6-(2,6-dimethylphenyl)pyrimido[5',4':5,6]pyrimido[1,2-a]benzimidazol-5(6H)-on;
2-((3-Chlor-4-(4-methyl-1-piperazinyl)phenyl)amino)-6-(2,6-dimethylphenyl)-pyrimido[5',4':5,6]pyrimido[1,2-a]benzimidazol-5(6H)-on;
2-((4-((2-(Dimethylamino)ethyl)oxy)phenyl)amino)-6-(2,6-dimethylphenyl)-pyrimido-[5',4':5,6]pyrimido[1,2-a]benzimidazol-5(6H)-on;
2-((4-((3-(Diethylamino)propyl)oxy)-3-fluorphenyl)amino)-6-(2,6-dimethylphenyl)-pyrimido[5',4':5,6]pyrimido[1,2-a]benzimidazol-5(6H)-on;
2-((4-((3-(Dimethylamino)propyl)oxy)phenyl)amino)-6-(2,6-dimethylphenyl)-pyrimido[5',4':5,6]pyrimido[1,2-a]benzimidazol-5(6H)-on;
2-((4-(1,4-Diazepan-1-yl)phenyl)amino)-6-(2,6-dimethylphenyl)pyrimido[5',4':5,6]-pyrimido[1,2-a]benzimidazol-5(6H)-on;
2-((4-(4-((3-Chlorphenyl)methyl)-1-piperazinyl)phenyl)amino)-6-(2,6-dimethylphenyl)pyrimido[5',4':5,6]pyrimido[1,2-a]benzimidazol-5(6H)-on;
2-((4-(4-(2-(Dimethylamino)ethyl)-1-piperazinyl)phenyl)amino)-6-(2,6-dimethylphenyl)pyrimido[5',4':5,6]pyrimido[1,2-a]benzimidazol-5(6H)-on;
2-((4-(4-(3-(Dimethylamino)propyl)-1-piperazinyl)phenyl)amino)-6-(2,6-dimethylphenyl)pyrimido[5',4':5,6]pyrimido[1,2-a]benzimidazol-5(6H)-on;
2-((4-(4-(Cyclohexylmethyl)-1-piperazinyl)phenyl)amino)-6-(2,6-dimethylphenyl)-pyrimido[5',4':5,6]pyrimido[1,2-a]benzimidazol-5(6H)-on;
2-((4-(4-Methyl-1-piperazinyl)phenyl)amino)-6-((1R)-1 -phenylethyl)pyrimido-[5',4':5,6]pyrimido[1,2-a]benzimidazol-5(6H)-on;
2-((4-(4-Methyl-1-piperazinyl)phenyl)amino)-6-((S)-1-phenylethyl)pyrimido-[5',4':5,6]pyrimido[1,2-a]benzimidazol-5(6H)-on;
2-((4-(4-Methyl-1-piperazinyl)phenyl)amino)-6-(2,4,6-trichlorphenyl)pyrimido-[5',4':5,6]pyrimido[1,2-a]benzimidazol-5(6H)-on;
2-((4-(4-Methyl-1-piperazinyl)phenyl)amino)-6-(pentafluorphenyl)pyrimido-[5',4':5,6]pyrimido[1,2-a]benzimidazol-5(6H)-on;
2-(4-(4-((6-(2,6-Dimethylphenyl)-5-oxo-5,6-dihydropyrimido[5',4':5,6]pyrimido-[1,2-a]benzimidazol-2-yl)amino)phenyl)-1-piperazinyl)acetamid;
3-((4-(4-((6-(2,6-Dimethylphenyl)-5-oxo-5,6-dihydropyrimido[5',4':5,6]-pyrimido-[1,2-a]benzimidazol-2-yl)amino)phenyl)-1-piperazinyl)methyl)-benzonitril;
6-(2,6-Dichlorphenyl)-2-((4-(4-methyl-1-piperazinyl)phenyl)amino)pyrimido-[5',4':5,6]pyrimido[1,2-a]benzimidazol-5(6H)-on;
6-(2,6-Difluorphenyl)-2-((4-(4-methyl-1-piperazinyl)phenyl)amino)pyrimido-[5',4':5,6]pyrimido[1,2-a]benzimidazol-5(6H)-on;
6-(2,6-Dimethylphenyl)-2-((2-methyl-4-((3-(1-piperidinyl)propyl)oxy)phenyl)amino)-pyrimido[5',4':5,6]pyrimido[1,2-a]benzimidazol-5(6H)-on;
6-(2,6-Dimethylphenyl)-2-((2-methyl-4-((3-(4-methyl-1-piperazinyl)propyl)oxy)phenyl)amino)pyrimido[5',4':5,6]pyrimido[1,2-a]benzimidazol-5(6H)-on;
6-(2,6-Dimethylphenyl)-2-((2-methyl-4-(4-methyl-1-piperazinyl)phenyl)amino)-pyrimido[5',4':5,6]pyrimido[1,2-a]benzimidazol-5(6H)-on;
6-(2,6-Dimethylphenyl)-2-((3-((3-(1-piperidinyl)propyl)oxy)phenyl)amino)pyrimido-[5',4':5,6]pyrimido[1,2-a]benzimidazol-5(6H)-on;
6-(2,6-Dimethylphenyl)-2-((3-((3-(4-methyl-1-piperazinyl)propyl)oxy)phenyl)amino)-pyrimido[5',4':5,6]pyrimido[1,2-a]benzimidazol-5(6H)-on;
6-(2,6-Dimethylphenyl)-2-((3-(4-methyl-1-piperazinyl)phenyl)amino)pyrimido-[5',4':5,6]pyrimido[1,2-a]benzimidazol-5(6H)-on;
6-(2,6-Dimethylphenyl)-2-((3-(methyloxy)-4-(((1-methyl-4-piperidinyl)methyl)oxy)-phenyl)amino)pyrimido[5',4':5,6]pyrimido[1,2-a]benzimidazol-5(6H)-on;
6-(2,6-Dimethylphenyl)-2-((3-(methyloxy)-4-(((2R)-2-pyrrolidinylmethyl)oxy)-phenyl)amino)pyrimido[5',4':5,6]pyrimido[1,2-a]benzimidazol-5(6H)-on;
6-(2,6-Dimethylphenyl)-2-((3-(methyloxy)-4-((2-(1-methyl-4-piperidinyl)ethyl)oxy)-phenyl)amino)pyrimido[5',4':5,6]pyrimido[1,2-a]benzimidazol-5(6H)-on;
6-(2,6-Dimethylphenyl)-2-((3-(methyloxy)-4-((2-(4-methyl-1-piperazinyl)ethyl)oxy)-phenyl)amino)pyrimido[5',4':5,6]pyrimido[1,2-a]benzimidazol-5(6H)-on;
6-(2,6-Dimethylphenyl)-2-((3-(methyloxy)-4-((3-(4-methyl-1-piperazinyl)propyl)-oxy)phenyl)amino)pyrimido[5',4':5,6]pyrimido[1,2-a]benzimidazol-5(6H)-on;
6-(2,6-Dimethylphenyl)-2-((3-(methyloxy)-4-(1-piperazinyl)phenyl)amino)-pyrimido-[5',4':5,6]pyrimido[1,2-a]benzimidazol-5(6H)-on;
6-(2,6-Dimethylphenyl)-2-((3-(methyloxy)-4-(4-methyl-1-piperazinyl)phenyl)-amino)-pyrimido[5',4':5,6]pyrimido[1,2-a]benzimidazol-5(6H)-on;
6-(2,6-Dimethylphenyl)-2-((3,4,5-tris(methyloxy)phenyl)amino)pyrimido[5',4':5,6]-pyrimido[1,2-a]benzimidazol-5(6H)-on;
6-(2,6-Dimethylphenyl)-2-((3-fluor-4-((2-(1-piperidinyl)ethyl)oxy)phenyl)amino)-pyrimido[5',4':5,6]pyrimido[1,2-a]benzimidazol-5(6H)-on;
6-(2,6-Dimethylphenyl)-2-((3-fluor-4-((2-(4-(1-methylethyl)-1-piperazinyl)ethyl)-oxy)-phenyl)amino)pyrimido[5',4':5,6]pyrimido[1,2-a]benzimidazol-5(6H)-on;
6-(2,6-Dimethylphenyl)-2-((3-fluor-4-((2-(4-methyl-1-piperazinyl)ethyl)oxy)-phenyl)-amino)pyrimido[5',4':5,6]pyrimido[1,2-a]benzimidazol-5(6H)-on;
6-(2,6-Dimethylphenyl)-2-((3-fluor-4-((3-(1-piperidinyl)propyl)oxy)phenyl)amino)-pyrimido[5',4':5,6]pyrimido[1,2-a]benzimidazol-5(6H)-on;
6-(2,6-Dimethylphenyl)-2-((3-fluor-4-((3-(4-(1-methylethyl)-1-piperazinyl)propyl)-oxy)phenyl)amino)-pyrimido[5',4':5,6]pyrimido[1,2-a]benzimidazol-5(6H)-on;
6-(2,6-Dimethylphenyl)-2-((3-fluor-4-((3-(4-methyl-1-piperazinyl)propyl)oxy)-phenyl)amino)pyrimido[5',4':5,6]pyrimido[1,2-a]benzimidazol-5(6H)-on;
6-(2,6-Dimethylphenyl)-2-((3-fluor-4-(4-(1-methylethyl)-1-piperazinyl)phenyl)-amino)pyrimido[5',4':5,6]pyrimido[1,2-a]benzimidazol-5(6H)-on;
6-(2,6-Dimethylphenyl)-2-((3-fluor-4-(4-methyl-1-piperazinyl)phenyl)amino)-pyrimido[5',4':5,6]pyrimido[1,2-a]benzimidazol-5(6H)-on;
6-(2,6-Dimethylphenyl)-2-((4-((((2S)-1-methyl-2-pyrrolidinyl)methyl)oxy)phenyl)-amino)pyrimido[5',4':5,6]pyrimido[1,2-a]benzimidazol-5(6H)-on;
6-(2,6-Dimethylphenyt)-2-((4-(((2R)-2-pyrrolidinylmethyl)oxy)phenyl)amino)-pyrimido[5',4':5,6]pyrimido[1,2-a]benzimidazol-5(6H)-on;
6-(2,6-Dimethylphenyl)-2-((4-(((2S)-2-pyrrolidinylmethyl)oxy)phenyl)amino)-pyrimido[5',4':5,6]pyrimido[1,2-a]benzimidazol-5(6H)-on;
6-(2,6-Dimethylphenyl)-2-((4-((2-((1-methylethyl)amino)ethyl)oxy)-3-(methyloxy)-phenyl)amino)pyrimido[5',4':5,6]pyrimido[1,2-a]benzimidazol-5(6H)-on;
6-(2,6-Dimethylphenyl)-2-((4-((2-((1-methylethyl)amino)ethyl)oxy)phenyl)amino)-pyrimido[5',4':5,6]pyrimido[1,2-a]benzimidazol-5(6H)-on;
6-(2,6-Dimethylphenyl)-2-((4-((2-(1-methyl-4-piperidinyl)ethyl)oxy)phenyl)amino)-pyrimido[5',4':5,6]pyrimido[1,2-a]benzimidazol-5(6H)-on;
6-(2,6-Dimethylphenyl)-2-((4-((2-(1-piperidinyl)ethyl)oxy)phenyl)amino)-pyrimido-[5',4':5,6]pyrimido[1,2-a]benzimidazol-5(6H)-on;
6-(2,6-Dimethylphenyl)-2-((4-((2-(4-(1-methylethyl)-1-piperazinyl)ethyl)oxy)-3-(methyloxy)phenyl)amino)pyrimido[5',4':5,6]pyrimido[1,2-a]benzimidazol-5(6H)-on;
6-(2,6-Dimethylphenyl)-2-((4-((2-(4-methyl-1-piperazinyl)ethyl)oxy)phenyl)amino)-pyrimido[5',4':5,6]pyrimido[1,2-a]benzimidazol-5(6H)-on;
6-(2,6-Dimethylphenyl)-2-((4-((2-(4-morpholinyl)ethyl)oxy)phenyl)amino)-pyrimido-[5',4':5,6]pyrimido[1,2-a]benzimidazol-5(6H)-on;
6-(2,6-Dimethylphenyl)-2-((4-((2-(methylamino)ethyl)oxy)phenyl)amino)-pyrimido-[5',4':5,6]pyrimido[1,2-a]benzimidazol-5(6H)-on;
6-(2,6-Dimethylphenyl)-2-((4-((3-(1-piperidinyl)propyl)oxy)phenyl)amino)-pyrimido-[5',4':5,6]pyrimido[1,2-a]benzimidazol-5(6H)-on;
6-(2,6-Dimethylphenyl)-2-((4-((3-(4-(1-methylethyl)-1-piperazinyl)propyl)oxy)-3-(methyloxy)phenyl)amino)pyrimido[5',4':5,6]pyrimido[1,2-a]benzimidazol-5(6H)-on;
6-(2,6-Dimethylphenyl)-2-((4-(1-piperazinyl)phenyl)amino)pyrimido[5',4':5,6]-pyrimido[1,2-a]benzimidazol-5(6H)-on;
6-(2,6-Dimethylphenyl)-2-((4-(4-((3-(methyloxy)phenyl)methyl)-1-piperazinyl)-phenyl)amino)pyrimido[5',4':5,6]pyrimido[1,2-a]benzimidazol-5(6H)-on;
6-(2,6-Dimethylphenyl)-2-((4-(4-(1-methylethyl)-1-piperazinyl)phenyl)amino)-pyrimido[5',4':5,6]pyrimido[1,2-a]benzimidazol-5(6H)-on;
6-(2,6-Dimethylphenyl)-2-((4-(4-(1-methylethyl)-1-piperazinyl)-3-(methyloxy)-phenyl)amino)pyrimido[5',4':5,6]pyrimido[1,2-a]benzimidazol-5(6H)-on;
6-(2,6-Dimethylphenyl)-2-((4-(4-(2-(methyloxy)ethyl)-1-piperazinyl)phenyl)amino)-pyrimido[5',4':5,6]pyrimido[1,2-a]benzimidazol-5(6H)-on;
6-(2,6-Dimethylphenyl)-2-((4-(4-(2-hydroxyethyl)-1-piperazinyl)phenyl)amino)-pyrimido[5',4':5,6]pyrimido[1,2-a]benzimidazol-5(6H)-on;
6-(2,6-Dimethylphenyl)-2-((4-(4-methyl-1,4-diazepan-1-yl)phenyl)amino)-pyrimido-[5',4':5,6]pyrimido[1,2-a]benzimidazol-5(6H)-on;
6-(2,6-Dimethylphenyl)-2-((4-(4-methyl-1-piperazinyl)phenyl)amino)-pyrimido-[5',4':5,6]pyrimido[1,2-a]benzimidazol-5(6H)-on;
6-(2,6-Dimethylphenyl)-2-((4-(4-methyl-1-piperazinyl)phenyl)amino)-8,9-dihydro-imidazo[1,2-a]pyrimido[5,4-e]pyrimidin-5(6H)-on;
6-(2,6-Dimethylphenyl)-2-((4-(4-methyl-1-piperazinyl)phenyl)amino)imidazo-[1,2-a]pyrimido[5,4-e]pyrimidin-5(6H)-on;
6-(2-Fluorphenyl)-2-((4-(4-methyl-1-piperazinyl)phenyl)amino)pyrimido[5',4':5,6]-pyrimido[1,2-a]benzimidazol-5(6H)-on;
6-(3-(Methyloxy)phenyl)-2-((4-(4-methyl-1-piperazinyl)phenyl)amino)pyrimido-[5',4':5,6]pyrimido[1,2-a]benzimidazol-5(6H)-on;
6-(3-Fluorphenyl)-2-((4-(4-methyl-1-piperazinyl)phenyl)amino)pyrimido[5',4':5,6]-pyrimido[1,2-a]benzimidazol-5(6H)-on;
6-(3-Methylphenyl)-2-((4-(4-methyl-1-piperazinyl)phenyl)amino)-pyrimido[5',4':5,6]-pyrimido[1,2-a]benzimidazol-5(6H)-on;
6-(4-(Methyloxy)phenyl)-2-((4-(4-methyl-1-piperazinyl)phenyl)amino)pyrimido-[5',4':5,6]pyrimido[1,2-a]benzimidazol-5(6H)-on;
6-(4-Fluorphenyl)-2-((4-(4-methyl-1-piperazinyl)phenyl)amino)pyrimido[5',4':5,6]-pyrimido[1,2-a]benzimidazol-5(6H)-on;
6-(4-Methyl]phenyl)-2-((4-(4-methyl-1-piperazinyl)phenyl)amino)-pyrimido[5',4':5,6]-pyrimido[1,2-a]benzimidazol-5(6H)-on;
6-(5-Chlor-2-(methyloxy)phenyl)-2-((4-(4-methyl-1-piperazinyl)phenyl)amino)-pyrimido[5',4':5,6]pyrimido[1,2-a]benzimidazol-5(6H)-on;
N-(2-(Diethylamino)ethyl)-3-((6-(2,6-dimethylphenyl)-5-oxo-5,6-dihydropyrimido-[5',4':5,6]pyrimido[1,2-a]benzimidazol-2-yl)amino)-N-methylbenzamid;
N-(2-(Diethylamino)ethyl)-4-((6-(2,6-dimethylphenyl)-5-oxo-5,6-dihydropyrimido-[5',4':5,6]pyrimido[1,2-a]benzimidazol-2-yl)amino)-N-methylbenzamid;
N-(2-(Dimethylamino)ethyl)-3-((6-(2,6-dimethylphenyl)-5-oxo-5,6-dihydropyrimido-[5',4':5,6]pyrimido[1,2-a]benzimidazol-2-yl)amino)benzamid; und
N-(2-(Dimethylamino)ethyl)-4-((6-(2,6-dimethylphenyl)-5-oxo-5,6-dihydropyrimido-[5',4':5,6]pyrimido[1,2-a]benzimidazol-2-yl)amino)benzamid.

12. Verfahren zum Herstellen einer Verbindung nach Anspruch 1, wobei das Verfahren die folgenden Schritte umfasst: Umsetzen eines Pyrimidinesters (A) 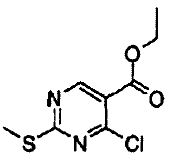 mit einem Benzimidazol (B) 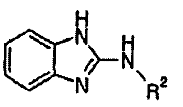 in Gegenwart einer Base, um ein tetracyclisches Pyrimidin (C) 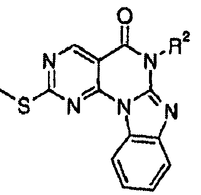 zu bilden; Oxidieren des Schwefels des tetracyclischen Pyrimidins (C), um ein tetracyclisches Pyrimidinsulfon (D) 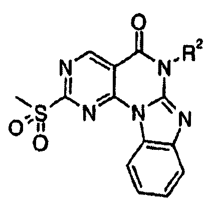 zu bilden; und Umsetzen des tetracyclischen Pyrimidinsulfons (D) mit R¹NH, um eine Verbindung 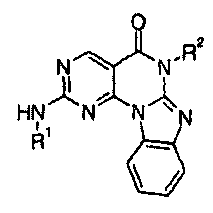 nach Anspruch 1 herzustellen.

13. Pharmazeutische Zusammensetzung, umfassend eine Verbindung nach einem der Ansprüche 1-11 und einen pharmazeutisch annehmbaren Träger.

14. Verwendung einer Verbindung nach einem der Ansprüche 1-11 für die Herstellung eines Medikaments für die Behandlung einer Entzündung.

15. Verwendung einer Verbindung nach einem der Ansprüche 1-11 für die Herstellung eines Medikaments für die Hemmung einer T-Zell-Aktivierung und -Proliferation in einem Säuger.

16. Verwendung einer Verbindung nach einem der Ansprüche 1-11 für die Herstellung eines Medikaments für die Behandlung von Arthritis, rheumatoider Arthritis, psoriatischer Arthritis oder Osteoarthritis bei einem Säuger.

17. Verwendung einer Verbindung nach einem der Ansprüche 1-11 für die Herstellung eines Medikaments für die Behandlung einer Organtransplantat-, akuten Transplantat- oder Heterotransplantat- oder Homotransplantatabstoßung oder Induktion von Transplantationstoleranz bei einem Säuger.

18. Verwendung einer Verbindung nach einem der Ansprüche 1-11 für die Herstellung eines Medikaments für die Behandlung eines ischämischen oder Reperfusionsschadens, Myokardinfarkts oder Schlaganfalls bei einem Säuger.

19. Verwendung einer Verbindung nach einem der Ansprüche 1-11 für die Herstellung eines Medikaments für die Behandlung von Multipler Sklerose, entzündlicher Darmkrankheit, einschließlich Colitis ulcerosa, Morbus Crohn, Lupus, Kontaktüberempfindlichkeit bzw. Kontaktallergie, Spättyp der Überempfindlichkeitsreaktion bzw. Spättyp-Allergie und glutenempfindlicher Enteropathie, Typ I-Diabetes, Psoriasis, Kontaktdermatitis, Hashimoto-Thyreoiditis, Sjögren-Syndrom, Autoimmunthyreoiditis, Addison-Krankheit, autoimmuner polyglandulärer Krankheit bzw. Insuffizienz, autoimmuner Alopezie, perniziöser Anämie, Vitiligo, Autoimmunhypopituitarismus, Guillain-Barré-Syndrom, Glomerulonephritis, Serum-Krankheit, Urtikaria, allergischen Krankheiten, Asthma, Heufieber, allergischer Rhinitis, Mycosis fungoides, Dermatomyositis, Alopecia areata, chronischer aktinischer Dermatitis, Ekzem, Behçet-Krankheit, Psoriasis pustulosa palmaris et plantaris, Pyoderma-Gangrän, Sézary-Syndrom, atopischer Dermatitis, systemischer Sklerose, Morphea oder atopischer Dermatitis bei einem Säuger.

20. Verwendung einer Verbindung nach einem der Ansprüche 1-11 für die Herstellung eines Medikaments für die Behandlung von Dickdarmkrebs oder Thymustumor bei einem Säuger.

## Revendications

1. Composé de formule I 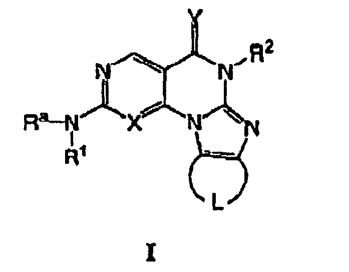 ou un sel ou un dérivé pharmaceutiquement acceptable de celui-ci, dans laquelle
X représente N ou CH ;
Y représente NH, N(CN), O ou S;
L représente une chaîne de 4 atomes constituée d'atomes C et N, où la chaîne est substituée par 0 ou 1 groupe R³ et la chaîne est en outre substituée par 0, 1, 2 ou 3 substituants choisis indépendamment parmi R^{c};
R¹ est choisi parmi, -R¹¹, -R¹¹-R¹², -R¹¹-R¹⁴, -R¹²-R¹⁴, -R¹¹-R¹²-R¹⁴, -R¹¹-R¹³-R¹², -R¹¹-R¹³-R¹⁴, -R¹²-R¹³-R¹⁴, -R¹¹-R¹³-R¹²-R¹⁴, -R¹¹-R¹²-R¹³-R¹⁴, -R¹¹-R¹⁴-R¹²-R¹³, -R¹¹-R¹⁴-R¹³-R¹², -R¹¹-R¹⁴-R¹²-R¹⁴ et -R¹¹-R¹⁴-R¹³-R¹⁴, dont l'un quelconque est substitué par 0, 1, 2, 3, 4 ou 5 substituants choisis indépendamment parmi R^{c} ;
R² est choisi parmi -R²¹, -R²¹-R²², -R²²-R²⁴, -R²²-R²⁴, -R²¹-R²²-R²⁴, -R²¹-R²³-R²⁴, -R²²-R²³-R²⁴, -R²¹-R²³-R²²-R²⁴ et -R²¹-R²²-R²³-R²⁴, dont l'un quelconque est substitué par 0, 1, 2, 3, 4 ou 5 substituants choisis indépendamment parmi R^{c} ;
R³ est choisi parmi -R³², -R³⁴, -R³²-R³⁴, -R³³-R³⁴, -R³³-R³²-R³⁴ et -R³²-R³³-R³⁴, dont l'un quelconque est substitué par 0, 1, 2, 3, 4 ou 5 substituants choisis indépendamment parmi R^{c} ;
R¹¹ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, 0 et S, tant que la combinaison des atomes O et S n'est pas supérieure à 2, où les atomes de carbone du cycle sont substitués par 0, 1 ou 2 groupes oxo ;
R¹² représente indépendamment à chaque fois un groupe alkyle en C₁ à C₈;
R¹³ représente indépendamment à chaque fois -C(=O)-, -C (=O)O-, -C(=O)N(R^{a})-, -C(=NR^{a})N(R^{a})-, -O-, -OC (=O) -, - OC(=O)N(R^{a})-, -OC(=O)N(R^{a})S(=O)₂-, -O-alkyl en C₂ à C₆-N(R^{a})-, -O-alkyl en C₂ à C₆-, -O-alkyl en C₂ à C₆-O-, -S-, -S(=O)-, -S (=O)₂-, -S(=O)₂N(R^{a})-, -S(=O)₂N(R^{a})C(=O)--S(=O)₂N(R^{a})C(=O)O-, -S(=O)₂N(R^{a})C(=O)N(R^{a})-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S (=O)₂N (R^{a}) -, -NR^{a}-alkyl en C₂ à C₆-N(R^{a})- ou -NR^{a}-alkyl en C₂ à C₆-O- ;
R¹⁴ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, O et S, tant que la combinaison des atomes O et S n'est pas supérieure à 2, où les atomes de carbone du cycle sont substitués par 0, 1 ou 2 groupes oxo ;
R²¹ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, 0 et S, tant que la combinaison des atomes 0 et S n'est pas supérieure à 2, où les atomes de carbone du cycle sont substitués par 0, 1 ou 2 groupes oxo ;
R²² représente indépendamment à chaque fois un groupe alkyle en C₁ à C₈ ;
R²³ représente indépendamment à chaque fois -C(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -O-alkyl en C₂ à C₆-N(R^{a})-, -O-alkyl en C₂ à C₆-O-, -S-, -S(=O)-, -S(=O)₂-, -S(=O)₂N(R^{a})-, -S(=O)₂N(R^{a})C(=O)-, -S(=O)₂N-(R^{a})C(=O)O-, -S(=O)₂N(R^{a})C(=O)N(R^{a})-, -N(R^{a})-, -N(R^{a})C-(=O)-, -N(R^{a})C(=O)O-, -N(R^{a})C(=O)N(R^{a})-, -N(R^{a})C(=NR^{a})-N(R^{a})-, -N(R^{a})S(=O)₂-, -N(R^{a})S(=O)₂N(R^{a})-, -NR^{a}-alkyl en C₂ à C₆-N(R^{a})- ou -NR^{a}-alkyl en C₂ à C₆-O-;
R²⁴ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, 0 et S, tant que la combinaison des atomes 0 et S n'est pas supérieure à 2, où les atomes de carbone du cycle sont substitués par 0, 1 ou 2 groupes oxo; R³² représente indépendamment à chaque fois un groupe alkyle en C₁ à C₈;
R³³ représente indépendamment à chaque fois -C(=O)-, -C(=O)O-, -C(=O)N(R^{a})-, -C(=NR^{a})N(R^{a})-, -O-, -OC (=O) -, -OC(=O)N(R^{a})-, -OC(=O)N(R^{a})S(=O)₂-, -O-alkyl en C₂ à C₆-N(R^{a})-, -O-alkyl en C₂ à C₆-O-, -S-, -S(=O)-, -S (=O)₂- -S (=O) ₂N (R^{a}) -, -S (=O) ₂N (R^{a}) C (=O) -, -S(=O)₂N-(R^{a})C(=O)O-, -S (=O) ₂N (R^{a}) C (=O) NR^{a}-, -N(R^{a})-, -N(R^{a})C(=O)-, -N (R^{a}) C (=O) O-, -N(R^{a})C(=O)N(R^{a})-, -N(R^{a})C(=NR^{a})N(R^{a})-, -N (R^{a}) S (=O) ₂-, -N(R^{a})S(=O)₂N (R^{a})-, -NR^{a}-alkyl en C₂ à C₆-N(R^{a})- ou NR^{a}-alkyl en C₂ à C₆-O- ;
R³⁴ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, O et S, tant que la combinaison des atomes 0 et S n'est pas supérieure à 2, où les atomes de carbone du cycle sont substitués par 0, 1 ou 2 groupes oxo; R^{a} représente indépendamment à chaque fois H ou R^{b};
R^{b} représente indépendamment à chaque fois un groupe alkyle en C₁ à C₈, CN, phényle ou benzyle; et
R^{c} représente indépendamment à chaque fois un groupe alkyle en C₁ à C₈, halogénoalkyle en C₁ à C₄, halogéno, cyano, nitro, -alkyl en C₂ à C₆-OR^{a}, -alkyl en C₂ à C₆-C(=O)NR^{a}R^{a}, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC-(=O)NR^{a}R^{a}, -OC(=O)N(R^{a})S(=O)₂R^{b}, -O-alkyl en C₂ à C₆-NR^{a}R^{a}, -O-alkyl en C₂ à C₆-C(=O)NR^{a}R^{a}, -O-alkyl en C₂ à C₆-OR^{a}, -SR^{a}, -S(=O)R^{b}, -S(=O)₂R^{b}, -S-(=O)₂NR^{a}R^{a}, -S(=O)₂N(R^{a})C(=O)R^{b}, -S(=O)₂N(R^{a})C-(=O)OR^{b}, -S(=O)₂N(R^{a})C(=O)NR^{a}R^{a}, -NR^{a}R^{a}, -N(R^{a})C-(=O)R^{b}, -N(R^{a})C(=O)OR^{b}, -N(R^{a})C(=O)NR^{a}R^{a}, -N(R^{a})C-(=NR^{a})NR^{a}R^{a}, -N(R^{a})S(=O)₂R^{b}, -N(R^{a})S(=O)₂NR^{a}R^{a}, -NR^{a}-alkyl en C₂ à C₆-NR^{a}R^{a} ou -NR^{a}-alkyl en C₂ à C₆-OR^{a}.

2. Composé selon la revendication 1, où X représente N et Y représente O.

3. Composé selon la revendication 1, où L représente une chaîne de 4 atomes constituée d'atomes C et N, où la chaîne est substituée par 0 ou 1 groupe R³ et la chaîne est en outre substituée par 0, 1, 2 ou 3 substituants choisis indépendamment parmi R^{c}.

4. Composé selon la revendication 1, où R¹¹ représente un groupe phényle, naphtylène, pyridine, pyrazine, triazine, quinoléine, isoquinoléine, thiophène, pyrrole, pyrrolidine, furane, tétrahydrofurane, thiazole, oxazole, imidazole, pipéridine, pipérazine ou morpholine.

5. Composé selon la revendication 1, où R²¹ représente un groupe phényle, naphtylène, pyridine, pyrazine, triazine, quinoléine, isoquinoléine, thiophène, pyrrole, pyrrolidine, furane, tétrahydrofurane, thiazole, oxazole, imidazole, pipéridine, pipérazine ou morpholine.

6. Composé selon la revendication 1 de formule II 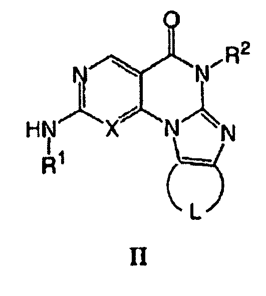 ou un sel pharmaceutiquement acceptable de celui-ci, dans laquelle
X représente N ou CH;
L représente une chaîne de 3 ou 4 atomes constituée d'atomes C et N, où la chaîne est substituée par 0 ou 1 groupe R³ et la chaîne est en outre substituée par 0, 1, 2 ou 3 substituants choisis indépendamment parmi R^{c} ;
R¹ est choisi parmi, -R¹¹, -R¹¹-R¹², -R¹¹-R¹⁴, -R¹²-R¹⁴, -R¹¹-R¹²-R¹⁴, -R¹¹-R¹³-R¹⁴, -R¹²-R¹³-R¹⁴, -R¹¹-R¹³-R¹²-R¹⁴ et -R¹¹-R¹²-R¹³-R¹⁴, dont l'un quelconque est substitué par 0, 1, 2, 3 ou 4 substituants choisis indépendamment parmi R^{c} ;
R² est choisi parmi -R²¹, -R²¹-R²², -R²¹-R²⁴, -R²²-R²⁴, -R²¹-R²²-R²⁴, -R²¹-R²³-R²⁴, -R²²-R²³-R²⁴, -R²¹-R²³-R²²-R²⁴ et -R²¹-R²²-R²³-R²⁴, dont l'un quelconque est substitué par 0, 1, 2, 3 ou 4 substituants choisis indépendamment parmi R^{c} ;
R³ est choisi parmi -R³², -R³⁴, -R³²-R³⁴, -R³³-R³⁴, -R³³-R³²-R³⁴ et -R³²-R³³-R³⁴, dont l'un quelconque est substitué par 0, 1, 2, 3 ou 4 substituants choisis indépendamment parmi R^{c};
R¹¹ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, O et S, tant que la combinaison des atomes 0 et S n'est pas supérieure à 2, où les atomes de carbone du cycle sont substitués par 0, 1 ou 2 groupes oxo ; R¹² représente indépendamment à chaque fois un groupe alkyle en C₁ à C₈ ;
R¹³ représente indépendamment à chaque fois - C(=O)-, -C(=O)O-, -C (=O) NR^{a}-, -C (=NR^{a}) NR^{a}-, -O-, -OC-(=O)-, -OC (=O) NR^{a}-, -OC (=O) N (R^{a}) S (=O)₂-, -O-alkyl en C₂ à C₆-NR^{a}-, -O-alkyl en C₂ à C₆-O-, -S-, -S (=O) -, -S-(=O)₂-, -S (=O)₂NR^{a}-, -S (=O)₂N (R^{a}) C (=O) -, -S(=O)₂N(R^{a})-C(=O)O-, -S(=O)₂N(R^{a}) C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}-alkyl en C₂ à C₆-N (R^{a}) - ou NR^{a}-alkyl en C₂ à C₆-O-;
R¹⁴ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, 0 et S, tant que la combinaison des atomes O et S n'est pas supérieure à 2, où les atomes de carbone du cycle sont substitués par 0, 1 ou 2 groupes oxo; R²¹ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, O et S, tant que la combinaison des atomes 0 et S n'est pas supérieure à 2, où les atomes de carbone du cycle sont substitués par 0, 1 ou 2 groupes oxo;
R²² représente indépendamment à chaque fois un groupe alkyle en C₁ à C₈;
R²³ représente indépendamment à chaque fois -C-(=O)-, -C(=O)O-, -C(=O)NRa-, -C(=NR^{a})NR^{a}-, -O-, -OC(=O)-, -OC(=O)NR^{a}-, -OC(=O)N(Ra)S(=O)2-, -O-alkyl en C₂ à C₆-NR^{a}-, -O-alkyl en C₂ à C₆-O-, -S-, -S(=O)-, -S(=O)₂-, -S(=O)₂NR^{a}-, -S(=O)₂N(R^{a})C(=O)-, -S(=O)₂N(R^{a})C(=O)O-, -S-(=O)₂N(R^{a})C(=O)NR^{a}-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N (R^{a}) S (=O)₂N (R^{a}) -, -NR^{a}-alkyl en C₂ à C₆-N(R^{a})- ou NR^{a}-alkyl en C₂ à C₆-O- ;
R²⁴ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, 0 et S, tant que la combinaison des atomes 0 et S n'est pas supérieure à 2, où les atomes de carbone du cycle sont substitués par 0, 1 ou 2 groupes oxo ; R³² représente indépendamment à chaque fois un groupe alkyle en C₁ à C₈ ;
R³³ représente indépendamment à chaque fois -C-(=O)-, -C(=O)O-, -C(=O)NR^{a}-, -C(=NR^{a})NR^{a}-, -O-, -OC(=O)-, -OC(=O)NR^{a}-, -OC(=O)N(R^{a})S(=O)₂-, -O-alkyl en C₂ à C₆-NR^{a}-, -O-alkyl en C₂ à C₆-O-, -S-, -S(=O)-, -S (=O)₂-, -S(=O)₂NR^{a}-, -S(=O)₂N(R^{a})C(=O)-, -S(=O)₂N(R^{a})C(=O)O-, -S(=O)₂N(R^{a})C(=O)NR^{a}-, -N(R^{a})-, -N(R^{a})C(=O)-, -N(R^{a})C-(=O)O-, -N(R^{a})C(=O)N(R^{a})-, -N(R^{a})C(=NR^{a})N(R^{a})-, -N(R^{a})S-(=O)₂-, -N(R^{a})S(=O)₂N(R^{a})-, -NR^{a}-alkyl en C₂ à C₆-N(R^{a})- ou NR^{a}-alkyl en C₂ à C₆-O- ;
R³⁴ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, 0 et S, tant que la combinaison des atomes 0 et S n'est pas supérieure à 2, où les atomes de carbone du cycle sont substitués par 0, 1 ou 2 groupes oxo ;
R^{a} représente indépendamment à chaque fois H ou R^{b} ;
R^{b} représente indépendamment à chaque fois un groupe alkyle en C₁ à C₈, phényle ou benzyle ; et
R^{c} représente indépendamment à chaque fois un groupe alkyle en C₁ à C₈, halogénoalkyle en C₁ à C₄, halogéno, cyano, nitro, -C(=O)R^{b}, -C(=O)OR^{b}, -C-(=O)NR^{a}R^{a}, -C (=NR^{a}) NR^{a}R^{a}, -OR^{a}, -OC(=O)R^{b}, -OC(=O)NR^{a}R^{a}, -OC(=O)N(R^{a})S(=O)₂R^{b}, -O-alkyl en C₂ à C₆-NR^{a}R^{a}, -O-alkyl en C₂ à C₆-OR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})-C(=O)NR^{a}R^{a}, -NR^{a}R^{a}, -N(R^{a})C(=O)R^{b}, -N(R^{a})C(=O)OR^{b}, -N-(R^{a})C(=O)NR^{a}R^{a}, -N(R^{a})C(=NR^{a})NR^{a}R^{a}, -N(R^{a})S(=O)₂R^{b}, -N-(R^{a}) S (=O) ₂NR^{a}R^{a}, -NR^{a}-alkyl en C₂ à C₆-NR^{a}R^{a} ou -NR^{a}-alkyl en C₂ à C₆-OR^{a}.

7. Composé selon la revendication 1 où
X représente N ;
Y représente 0 ;
L représente une chaîne de 4 atomes formée d'atomes C, où la chaîne est substituée par 0 ou 1 groupe R³ et la chaîne est en outre substituée par 0, 1, 2 ou 3 substituants choisis indépendamment parmi R^{c};
R¹ est choisi parmi -R¹¹, -R¹¹-R¹² -R¹¹-R¹⁴, R¹²-R¹⁴, -R¹¹-R¹²-R¹⁴, -R¹¹-R¹³-R¹², -R¹¹-R¹³-R¹⁴, -R¹²-R¹³-R¹⁴ -R¹¹-R¹³-R¹²-R¹⁴, -R¹¹-R¹²-R¹³-R¹⁴ -R¹¹-R¹⁴-R¹²-R¹³, -R¹¹-R¹⁴-R¹³-R¹², -R¹¹-R¹⁴-R¹²-R¹⁴ et -R¹¹-R¹⁴-R¹³-R¹⁴, dont l'un quelconque est substitué par 0, 1, 2, 3, 4 ou 5 substituants choisis indépendamment parmi R^{c} ;
R² est choisi parmi -R²¹, -R²¹-R²², -R²¹-R²⁴, -R²²-R²⁴, -R²¹-R²²-R²⁴, R²¹-R²³-R²⁴ et -R²²-R²³-R²⁴, dont l'un quelconque est substitué par 0, 1, 2, 3, 4 ou 5 substituants choisis indépendamment parmi R^{c} ;
R³ est choisi parmi -R³², R³⁴, -R³²-R³⁴, R³³-R³⁴, -R³³-R³²-R³⁴ et -R³²-R³³-R³⁴, dont l'un quelconque est substitué par 0, 1, 2, 3, 4 ou 5 substituants choisis indépendamment parmi R^{c} ;
R¹¹ représente un groupe phényle, naphtylène, pyridine, pyrazine, triazine, quinoléine, isoquinoléine, thiophène, pyrrole, pyrrolidine, furane, tétrahydrofurane, thiazole, oxazole, imidazole, pipéridine, pipérazine ou morpholine ; R¹² représente indépendamment à chaque fois un groupe alkyle en C₁ à C₈ ;
R¹³ représente indépendamment à chaque fois - C(=O)-, -C(=O)O-, -C(=O)N(R^{a})-, -C(=NR^{a})N(R^{a})-, -O-, -OC(=O)N(R^{a})-, -O-alkyl en C₂ à C₆-N(R^{a})₂-, -O-alkyl en C₂ à C₆-, -O-alkyl en C₂ à C₆-O-, -S-, -S(=O)-, -S(=O)₂-, -S(=O)₂N(R^{a})-, -S(=O)₂N(R^{a})C(=O)-, -N(R^{a})-, -N(R^{a})C(=O)-, -N(R^{a})C(=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}-alkyl en C₂ à C₆-N (R^{a}) - ou NR^{a}-alkyl en C₂ à C₆-O- ;
R¹⁴ représente indépendamment à chaque fois un cycle monocyclique de 5, 6 ou 7 chaînons ou bicyclique de 6, 7, 8, 9, 10 ou 11 chaînons, saturé ou insaturé contenant 0, 1, 2, 3 ou 4 atomes choisis parmi N, 0 et S, tant que la combinaison des atomes 0 et S n'est pas supérieure à 2, où les atomes de carbone du cycle sont substitués par 0, 1 ou 2 groupes oxo ;
R²¹ représente indépendamment à chaque fois un groupe phényle, naphtylène, pyridine, pyrazine, triazine, quinoléine, isoquinoléine, thiophène, pyrrole, pyrrolidine, furane, tétrahydrofurane, thiazole, oxazole, imidazole, pipéridine, pipérazine ou morpholine ;
R²² représente indépendamment à chaque fois un groupe alkyle en C₁ à C₈;
R²³ représente indépendamment à chaque fois -C-(=O)-, -C(=O)O-, -C(=O)N(R^{a})-, -C(=NR^{a})N(R^{a})-, -O-, -OC-(=O)-, -OC(=O)N(R^{a})-, -OC (=O) N (R^{a}) S (=O)₂-, -O-alkyl en C₂ à C₆-N(R^{a})-, -O-alkyl en C₂ à C₆-O-, -S-, -S (=O)-, *-*S(=O)₂-, -S(=O)₂N(R^{a})-, -S(=O)₂N(R^{a})C(=O)-, -S (=O) ₂N-(R^{a})C(=O)O-, -S(=O)₂N(R^{a})C(=O)N(R^{a})*-,* -N(R^{a})-, -N (R^{a}) C-(=O)-, N(R^{a})C(=O)O-, -N(R^{a})C(=O)N(R^{a})-, -N (R^{a}) C-(=NR^{a})N(R^{a})-, -N(R^{a})S(=O)₂-, -N(R^{a})S(=O)₂N(R^{a})-, -NR^{a}- alkyl en C₂ à C₆-N (R^{a}) - ou NR^{a}-alkyl en C₂ à C₆-O-;
R²⁴ représente indépendamment à chaque fois un groupe phényle, naphtylène, pyridine, pyrazine, triazine, quinoléine, isoquinoléine, thiophène, pyrrole, pyrrolidine, furane, tétrahydrofurane, thiazole, oxazole, imidazole, pipéridine, pipérazine, morpholine, pyrane, dioxane, cyclopropane, cyclobutane, cyclopentane ou cyclohexane ;
R³² représente indépendamment à chaque fois un groupe alkyle en C₁ à C₈ ;
R³³ représente indépendamment à chaque fois -C(=O)-, -C(=O)O-, -C(=O)N(R^{a})-, -C(=NR^{a})N(R^{a})-, -O-, -O-alkyl en C₂ à C₆-N(R^{a})-, -0-alkyl en C₂ à C₆-O-, -S-, -S(=O)-, -S(=O)₂-, -S(=O)₂N(R^{a})-, -N(R^{a})-, -N(R^{a})C(=O)-, -N(R^{a})C=O)O-, -N(R^{a})C(=O)N(R^{a})-, -N(R^{a})C(=NR^{a})N(R^{a})-, -N(R^{a})S(=O)₂-, -N(R^{a})S(=O)₂N(R^{a})-, -NR^{a}-alkyl en C₂ à C₆-N(R^{a})- ou -NR^{a}-alkyl en C₂ à C₆-O- ;
R³⁴ représente indépendamment à chaque fois un groupe phényle, naphtylène, pyridine, pyrazine, triazine, quinoléine, isoquinoléine, thiophène, pyrrole, pyrrolidine, furane, tétrahydrofurane, thiazole, oxazole, imidazole, pipéridine, pipérazine, morpholine, pyrane, dioxane, cyclopropane, cyclobutane, cyclopentane ou cyclohexane ;
R^{a} représente indépendamment à chaque fois H ou R^{b};
R^{b} représente indépendamment à chaque fois un groupe alkyle en C₁ à C₈, CN, phényle ou benzyle ; et
R^{c} représente indépendamment à chaque fois un groupe alkyle en C₁ à C₈, halogénoalkyle en C₁ à C₄, halogéno, cyano, nitro, -alkyl en C₂ à C₆-OR^{a}, -alkyl en C₂ à C₆-C (=O) NR^{a}R^{a}, -C(=O)R^{b}, -C(=O)OR^{b}, -C(=O)NR^{a}R^{a}, -C(=NR^{a})NR^{a}R^{a}, -OR^{a}, -OC(=O)R^{b}, -OC(=O)NR^{a}R^{a}, -OC(=O)N(R^{a})S(=O)₂R^{b}, -O-alkyl en C₂ à C₆-NR^{a}R^{a}, -O-alkyl en C₂ à C₆-C(=O)NR^{a}R^{a}, -O-alkyl en C₂ à C₆-OR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})-C(=O)OR^{b}, -S(=O)₂N(R^{a})C(=O)NR^{a}R^{a}, -NR^{a}R^{a}, -N(R^{a})C-(=O)R^{b}, -N(R^{a})C(=O)OR^{b}, -N(R^{a})C(=O)NR^{a}R^{a}, -N(R^{a})C-(=NR^{a})NR^{a}R^{a}, -N(R^{a})S(=O)₂R^{b}, -N(R^{a})S(=O)₂NR^{a}R^{a}, -NR^{a}-alkyl en C₂ à C₆-NR^{a}R^{a} ou -NR^{a}-alkyl en C₂ à C₆-OR^{a}.

8. Composé selon la revendication 7 où
L représente une chaîne de 4 atomes non substituée formée d'atomes C ;
R¹¹ représente un groupe phényle, naphtylène, pyridine, pyrazine, triazine, quinoléine, isoquinoléine ou thiophène;
R¹⁴ représente un groupe phényle, naphtylène, pyridine, pyrazine, triazine, quinoléine, isoquinoléine, thiophène, pyrrole, pyrrolidine, furane, tétrahydrofurane, thiazole, oxazole, imidazole, pipéridine, pipérazine, morpholine, pyrane, dioxane, cyclopropane, cyclobutane, cyclopentane ou cyclohexane ; et
R²¹ représente un groupe phényle, pyridine, pyrazine, triazine ou thiophène.

9. Composé selon la revendication 7 où
R^{c} représente indépendamment à chaque fois un groupe méthyle, éthyle, propyle, isopropyle, n-butyle, sec-butyle, tert-butyle, pentyle, halogénoalkyle en C₁ à C₄, F, Cl, Br, cyano, nitro, -alkyl en C₂ à C₆-OR^{a}, -alkyl en C₂ à C₆-C(=O)NR^{a}R^{a}, -C(=O)R^{b}, -C(=O)OR^{b}, -C(=O)NR^{a}R^{a}, -C-(=NR^{a})NR^{a}R^{a}, -OR^{a}, -O-alkyl en C₂ à C₆-NR^{a}R^{a}, -O-alkyl en C₂ à C₆-C(=O)NR^{a}R^{a}, -0-alkyl en C₂ à C₆-OR^{a}, -SR^{a}, -S(=O)R^{b}, -S(=O)₂R^{b}, -S(=O)₂NR^{a}R^{a}, -N-R^{a}R^{a}, -N(R^{a})C(=O)R^{b}, N(R^{a})C(=O)NR^{a}R^{a}, -N(R^{a})C-(=NR^{a})NR^{a}R^{a}, -N(R^{a})S(=O)₂R^{b}, -N(R^{a})S(=O)₂NR^{a}R^{a}, -NR^{a}-alkyl en C₂ à C₆-NR^{a}R^{a} ou -NR^{a}-alkyl en C₂ à C₆-OR^{a}.

10. Composé selon la revendication 1 ayant une formule III 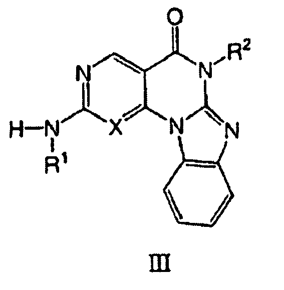 ou un sel ou un dérivé pharmaceutiquement acceptable de celui-ci, dans laquelle
R¹ est choisi parmi, -R¹¹, -R¹¹-R¹², -R¹¹-R¹⁴, -R¹² R¹⁴, -R¹¹-R¹²-R¹⁴, -R¹¹-R¹³-R¹², -R¹¹-R¹³-R¹⁴ -R¹²-R¹³-R¹⁴, -R¹¹-R¹³-R¹³-R¹⁴, -R¹¹-R¹²-R¹³-R¹⁴ -R¹¹-R¹⁴-R¹²-R¹³, -R¹¹-R¹⁴-R¹³-R¹², -R¹¹-R¹⁴-R¹²-R¹⁴ et -R¹¹-R¹⁴-R¹³-R¹⁴, dont l'un quelconque est substitué par 0, 1, 2, 3, 4 ou 5 substituants choisis indépendamment parmi R^{c};
R² est choisi parmi -R²¹, -R²¹-R²², -R²¹-R²⁴ et -R²²-R²⁴, dont l'un quelconque est substitué par 0, 1, 2, 3, 4 ou 5 substituants choisis indépendamment parmi R^{C} ;
R¹¹ représente indépendamment à chaque fois un groupe phényle, pyridine ou pyrazine ;
R¹² représente indépendamment à chaque fois un groupe alkyle en C₁ à C₈ ;
R¹³ représente indépendamment à chaque fois -C-(=O)-, -C(=O)O-, -C(=O)N(R^{a})-, -C(=NR^{a})N(R^{a})-, -O-, -OC(=O)N(R^{a})-, -O-alkyl en C₂ à C₆-N(R^{a})-, - O-alkyl en C₂ à C₆-, -O-alkyl en C₂ à C₆-O-, -S-, -S(=O)-, -S(=O)₂-, -S(=O)₂N(R^{a})-, -S(=O)₂N(R^{a})-C(=O)-, -N(R^{a})-, -N(R^{a})C(=O)-, -N(R^{a})C(=O)O-, -N-(R^{a})C(=O)N(R^{a})-, -N (R^{a}) C (=NR^{a}) N (R^{a}) **-,** -N (R^{a}) S-(=O)₂-, -N(R^{a})S(=O)₂N(R^{a})-, -NR^{a}-alkyl en C₂ à C₆-N ( R^{a} ) - ou NR^{a}-alkyl en C₂ à C₆-O- ;
R¹⁴ représente un groupe phényle, pyridine, pyrazine, thiophène, pyrrole, pyrrolidine, furane, tétrahydrofurane, pipéridine, pipérazine, morpholine, cyclopropane, cyclopentane ou cyclohexane ;
R²¹ représente un groupe phényle, pyridine ou pyrazine ;
R²² représente indépendamment à chaque fois un groupe alkyle en C₁ à C₈ ;
R²³ représente indépendamment à chaque fois -C-(=O)-, -C(=O)O-, -C(=O)N(R^{a})-, -C(=NR^{a})N(R^{a})-, -O-, -OC(=O)-, -OC(=O)N(R^{a})-, -OC(=O)N(R^{a})S(=O)₂-, -O-alkyl en C₂ à C₆-N(R^{a})-, -O-alkyl en C₂ à C₆-O-, -S-, -S(=O)-, -S(=O)₂-, -S(=O)₂N(R^{a})*-,* -S-(=O)₂N(R^{a})C(=O)-, -S(=O)₂N(R^{a})C(=O)O-, -S(=O)₂N-(R^{a})C(=O)N(R^{a})-, -N(R^{a})-, -N(R^{a})C(=O)-, -N (R^{a}) C-(=O)O⁻ -N(R^{a})C(=O)N(R^{a})-, -N(R^{a})C(=NR^{a})N(R^{a})*-*, -N(R^{a})S(=O)₂-, -N(R^{a})S(=O)₂N(R^{a})-, -NR^{a}-alkyl en C₂ à C₆-N (R^{a}) - ou NR^{a}-alkyl en C₂ à C₆-O- ;
R²⁴ représente un groupe phényle, pyridine, pyrazine, thiophène, pyrrole, pyrrolidine, furane, tétrahydrofurane, pipéridine, pipérazine, morpholine, cyclopropane, cyclopentane ou cyclohexane ;
R^{a} représente indépendamment à chaque fois H ou R^{b} ;
R^{b} représente indépendamment à chaque fois un groupe alkyle en C₁ à C₈, CN, phényle ou benzyle ; et
R^{c} représente indépendamment à chaque fois un groupe méthyle, éthyle, propyle, isopropyle, n-butyle, sec-butyle, tert-butyle, pentyle, halogénoalkyle en C₁ à C₄, F, Cl, Br, cyano, nitro, -alkyl en C₂ à C₆-OR^{a}, -alkyl en C₂ à C₆-C(=O)NR^{a}R^{a}, -C(=O)R^{b}, -C(=O)OR^{b}, -C(=O)NR^{a}R^{a}, -C-(=NR^{a})NR^{a}R^{a}, -OR^{a}, -O-alkyl en C₂ à C₆-NR^{a}R^{a}, -O-alkyl en C₂ à C₆-C(=O)NR^{a}R^{a}, -O-alkyl en C₂ à C₆-OR^{a}, -SR^{a}, -S(=O)R^{b}, -S(=O)₂R^{b}, -S(=O)₂NR^{a}R^{a}, -N-R^{a}R^{a}, -N(R^{a})C(=O)R^{b}, N(R^{a})C(=O)NR^{a}R^{a}, -N(R^{a})C-(=NR^{a})NR^{a}R^{a}, -N(R^{a})S(=O)₂R^{b}, -N(R^{a})S(=O)₂NR^{a}R^{a}, -NR^{a}-alkyl en C₂ à C₆-NR^{a}R^{a} ou -NR^{a}-alkyl en C₂ à C₆-OR^{a}.

11. Composé selon la revendication 1, où le composé est choisi parmi :
le 4-(4-((6-(2,6-diméthylphényl)-5-oxo-5,6-dihydropyrimido[5',4':5,6]pyrimido[1,2-a]-benzimidazol-2-yl)amino)phényl)-1-pipérazinecarboxylate de 1, 1-diméthyléthyle ;
la 2-((3,4-bis(méthyloxy)-5-((2-(4-méthyl-1-pipérazinyl)éthyl)oxy)phényl)amino)-6-(2,6-diméthyl-phényl)pyrimido[5',4':5,6]pyrimido[1, 2-a]benzimidazol-5(6H)-one;
la 2-((3,4-bis(méthyloxy)-5-((3-(4-méthyl-1-pipérazinyl)propyl)oxy)phényl)amino)-6-(2,6-diméthylphényl)pyrimido[5',4':5,6]pyrimido[1,2-a]benzimidazol-5(6H)-one ;
la 2-((3,5-bis(méthyloxy)-4-((2-(1-pipéridinyl)-éthyl)oxy)phényl)amino)-6-(2,6-diméthylphényl)-pyrimido-[5',4':5,6]pyrimido[1,2-a]benzimidazol-5(6H)-one ;
la 2-((3,5-bis(méthyloxy)-4-((2-(4-méthyl-1-pipérazinyl)éthyl)oxy)phényl)amino)-6-(2,6-diméthyl-phényl)pyrimido[5',4':5,6]pyrimido[1, 2-a]benzimidazol-5(6H)-one ;
la 2-((3,5-bis(méthyloxy)-4-((2-(4-morpholinyl)-éthyl)oxy)phényl)amino)-6-(2,6-diméthylphényl)-pyrimido-[5',4':5,6]pyrimido[1,2-a]benzimidazol-5 (6H) -one ;
la 2-((3,5-bis(méthyloxy)-4-((3-(4-méthyl-1-pipérazinyl)propyl)oxy)phényl)amino)-6-(2,6-diméthyl-phényl)pyrimido[5',4':5,6]pyrimido-[1,2-a]benzimidazol-5(6H)-one ;
la 2-((3-chloro-4-((2-((1-méthyléthyl)amino)-éthyl)oxy)phényl)amino)-6-(2,6-diméthylphényl)-pyrimido-[5',4':5,6]pyrimido[1,2-a]benzimidazol-5(6H)-one ;
la 2-((3-chloro-4-((2-(1-pipéridinyl)éthyl)oxy)-phényl)amino)-6-(2,6-diméthylphényl)pyrimido-[5',4':5,6]pyrimido[1,2-a]benzimidazol-5(6H)-one ;
la 2-((3-chloro-4-((2-(4-(1-méthyléthyl)-1-pipérazinyl)éthyl)oxy)phényl)amino)-6-(2,6-diméthylphényl)pyrimido-[5',4':5,6]pyrimido[1,2-a]benzimidazol-5(6H)-one ;
la 2-((3-chloro-4-((3-(4-méthyl-1-pipérazinyl)-propyl)oxy)phényl)amino)-6-(2,6-diméthylphényl)-pyrimido[5',4':5,6]pyrimido[1,2-a]benzimidazol-5(6H)-one ;
la 2-((3-chloro-4-(4-(1-méthyléthyl)-1-pipérazinyl)phényl)amino)-6-(2,6-diméthylphényl)-pyrimido[5',4':5,6]pyrimido[1,2-a]benzimidazol-5(6H)-one ;
la 2-((3-chloro-4-(4-méthyl-1-pipérazinyl)-phényl)-amino)-6-(2,6-diméthylphényl)pyrimido-[5',4':5,6]-pyrimido[1,2-a]benzimidazol-5(6H)-one ;
la 2-((4-((2-(diméthylamino)éthyl)oxy)phényl)-amino)-6-(2,6-diméthylphényl)pyrimido[5',4': 5,6]-pyrimido[1,2-a]benzimidazol-5(6H)-one;
la 2-((4-((3-(diéthylamino)propyl)oxy)-3-fluorophényl)amino)-6-(2,6-diméthylphényl)pyrimido-[5',4':5,6]pyrimido[1,2-a]benzimidazol-5(6H)-one ;
la 2-((4-((3-(diméthylamino)propyl)oxy)phényl)-amino)-6-(2,6-diméthylphényl)pyrimido[5',4': 5,6]-pyrimido[1,2-a]benzimidazol-5(6H)-one ;
la 2-((4-(1,4-diazépan-1-yl)phényl)amino)-6-(2, 6-diméthylphényl)pyrimido[5',4':5,6]pyrimido-[1,2-a]-benzimidazol-5(6H)-one ;
la 2-((4-(4-((3-chlorophényl)méthyl)-1-pipérazinyl)phényl)amino)-6-(2,6-diméthylphényl)-pyrimido[5',4':5,6]pyrimido[1,2-a]benzimidazol-5(6H)-one ;
la 2-((4-(4-(2-(diméthylamino)éthyl)-1-pipérazinyl)phényl)amino)-6-(2,6-diméthylphényl)-pyrimido[5',4';5,6]pyrimido[1,2-a]benzimidazol-5(6H)-one ;
la 2-((4-(4-(3-(diméthylamino)propyl)-1-pipérazinyl)phényl)amino)-6-(2,6-diméthylphényl)-pyrimido[5',4':5,6]pyrimido[1,2-a]benzimidazol-5(6H)-one ;
la 2-((4-(4-(cyclohexylméthyl)-1-pipérazinyl)-phényl)amino)-6-(2,6-diméthylphényl)pyrimido-[5', 4':5,6]pyrimido[1,2-a]benzimidazol-5(6H)-one ;
la 2-((4-(4-méthyl-1-pipérazinyl)phényl)amino)-6-((1R)-1-phényléthyl)pyrimido[5',4':5,6]-pyrimido[1,2-a]-benzimidazol-5(6H)-one;
la 2-((4-(4-méthyl-1-pipérazinyl)phényl)amino)-6-((1S)-1-phényléthyl)pyrimido[5',4':5,6]-pyrimido[1,2-a]-benzimidazol-5(6H)-one ;
la 2-((4-(4-méthyl-1-pipérazinyl)phényl)amino)-6-(2,4,6-trichlorophényl)pyrimido[5',4':5,6]-pyrimido-[1,2-a]benzimidazol-5(6H)-one;
la 2-((4-(4-méthyl-1-pipérazinyl)phényl)amino)-6-(pentafluorophényl)pyrimido[5',4':5,6]-pyrimido[1,2-a]-benzimidazol-5(6H)-one;
le 2-(4-(4-((6-(2,6-diméthylphényl)-5-oxo-5,6-dihydropyrimido[5',4':5,6]pyrimido-[1,2-a]-benzimidazol-2-yl)amino)phényl)-1-pipérazinyl)-acétamide ;
le 3-((4-(4-((6-(2,6-diméthylphényl)-5-oxo-5,6-dihydropyrimido[5',4':5,6]pyrimido-[1,2-a]benzimidazol-2-yl)amino)phényl)-1-pipérazinyl)méthyl)benzonitrile ;
la 6-(2,6-dichlorophényl)-2-((4-(4-méthyl-1-pipérazinyl)phényl)amino)pyrimido[5',4':5,6]pyri mido-[1,2-a]-benzimidazol-5(6H)-one ;
la 6-(2,6-difluorophényl)-2-((4-(4-méthyl-1-pipérazinyl)phényl)amino)pyrimido[5',4':5,6]pyri mido-[1,2-a]-benzimidazol-5(6H)-one ;
la 6-(2,6-diméthylphényl)-2-((2-méthyl-4-((3-(1-pipéridinyl)propyl)oxy)phényl)amino)pyrimido-[5', 4':5,6]pyrimido-[1,2-a]benzimidazol-5(6H)-one ;
la 6-(2,6-diméthylphényl)-2-((2-méthyl-4-((3-(4-méthyl-1-pipérazinyl)propyl)oxy)phényl)amino)-pyrimido-[5',4':5,6]pyrimido-[1,2-a]benzimidazol-5(6H)-one ;
la 6-(2,6-diméthylphényl)-2-((2-méthyl-4-(4-méthyl-1-pipérazinyl)phényl)amino)pyrimido[5',4': 5,6]-pyrimido-[1,2-a]benzimidazol-5(6H)-one;
la 6-(2,6-diméthylphényl)-2-((3-((3-(1-pipéridinyl)propyl)oxy)phényl)amino)pyrimido-[5',4':5, 6]pyrimido-[1,2-a]benzimidazol-5(6H)-one ;
la 6-(2,6-diméthylphényl)-2-((3-((3-(4-méthyl-l-pipérazinyl)propyl)oxy)phényl)amino)pyrimido-[5',4':5,6]pyrimido[1,2-a]benzimidazol-5(6H)-one ;
la 6-(2,6-diméthylphényl)-2-((3-(4-méthyl-1-pipérazinyl)phényl)amino)pyrimido[5',4':5,6]pyri mido-[1,2-a]benzimidazol-5(6H)-one ;
la 6-(2,6-diméthylphényl)-2-((3-(méthyloxy)-4-(((1-méthyl-4-pipéridinyl)méthyl)oxy)phényl)-amino)-pyrimido[5',4':5,6]pyrimido[1,2-a]benzimidazol-5(6H)-one ;
la 6-(2,6-diméthylphényl)-2-((3-(méthyloxy)-4-(((2R)-2-pyrrolidinylméthyl)oxy)phényl)amino)-pyrimido-[5',4':5,6]pyrimido[1,2-a]benzimidazol-5(6H)-one ;
la 6-(2,6-diméthylphényl)-2-((3-(méthyloxy)-4-((2-(1-méthyl-4-pipéridinyl)éthyl)oxy)phényl)-amino)-pyrimido[5',4':5,6]pyrimido[1,2-a]benzimidazol-5(6H)-one ;
la 6-(2,6-diméthylphényl)-2-((3-(méthyloxy)-4-((2-(4-méthyl-1-pipérazinyl)éthyl)oxy)phényl)-amino)-pyrimido[5',4':5,6]pyrimido[1,2-a]benzimidazol-5(6H)-one ;
la 6-(2,6-diméthylphényl)-2-((3-(méthyloxy)-4-((3-(4-méthyl-1-pipérazinyl)propyl)oxy)phényl)-amino)-pyrimido[5',4':5,6]pyrimido[1,2-a]benzimidazol-5(6H)-one ;
la 6 - (2,6-diméthylphényl) -2- ((3-(méthyloxy)-4-(1-pipérazinyl)phényl)amino)pyrimido[5',4':5,6]-pyrimido- [1,2-a]benzimidazol-5(6H)-one;
la 6- (2,6-diméthylphényl) -2- ((3-(méthyloxy)-4-(4-méthyl-1-pipérazinyl)phényl)amino)pyrimido[5', 4':5,6]-pyrimido[1,2-a]benzimidazol-5(6H)-one;
la 6-(2,6-diméthylphényl)-2-((3,4,5-tris-(méthyloxy)phényl)amino)pyrimido[5',4':5,6]pyrimido-[1,2-a]benzimidazol-5(6H)-one;
la 6-(2,6-diméthylphényl)-2-((3-fluoro-4-((2-(1-pipéridinyl)éthyl)oxy)phényl)amino)pyrimido[5',4 ':5,6]-pyrimido[1,2-a]benzimidazol-5(6H)-one ;
la 6-(2,6-diméthylphényl)-2-((3-fluoro-4-((2-(4-(1-méthyléthyl)-1-pipérazinyl)éthyl)oxy)phényl)-amino)-pyrimido[5',4':5,6]pyrimido[1,2-a]-benzimidazol-5(6H)-one ;
la 6-(2,6-diméthylphényl)-2-((3-fluoro-4-((2-(4-méthyl-1-pipérazinyl)éthyl)oxy)phényl)amino)-pyrimido-[5',4':5,6]pyrimido[1,2-a]benzimidazol-5 (6H) -one ;
la 6-(2,6-diméthylphényl)-2-((3-fluoro-4-((3-(1-pipéridinyl)propyl)oxy)phényl)amino)pyrimido-[5', 4':5,6]pyrimido[1,2-a]benzimidazol-5(6H)-one;
la 6-(2,6-diméthylphényl)-2-((3-fluoro-4-((3-(4-(1-méthyléthyl)-1-pipérazinyl)propyl)oxy)-phényl)-amino)-pyrimido[5',4':5,6]pyrimido[1,2-a]benzimidazol-5(6H)-one ;
la 6-(2,6-diméthylphényl)-2-((3-fluoro-4-((3-(4-méthyl-1-pipérazinyl)propyl)oxy)phényl)amino)-pyrimido-[5',4':5,6]pyrimido[1,2-a]benzimidazol-5(6H)-one ;
la 6-(2,6-diméthylphényl)-2-((3-fluoro-4-(4-(1-méthyléthyl)-1-pipérazinyl)phényl)amino)pyrimido-[5',4':5,6]pyrimido[1,2-a]benzimidazol-5 (6H) -one ;
la 6-(2,6-diméthylphényl)-2-((3-fluoro-4-(4-méthyl-1-pipérazinyl)phényl)amino)pyrimido[5', 4':5,6]-pyrimido[1,2-a] benzimidazol-5(6H)-one;
la 6- (2,6-diméthylphényl) -2- ((4-((((2S)-1-méthyl-2-pyrrolidinyl) méthyl)oxy)phényl)amino)-pyrimido-[5',4':5,6]pyrimido[1,2-a]benzimidazol-5 (6H) -one;
la 6-(2,6-diméthylphényl) -2- ((4-(((2R)-2-pyrrolidinylméthyl)oxy)phényl)amino)pyrimido-[5',4': 5,6]pyrimido[1,2-a] benzimidazol-5(6H)-one;
la 6-(2,6-diméthylphényl)-2-((9-(((2S)-2-pyrrolidinylméthyl)oxy)phényl)amino)pyrimido-[5',4': 5,6]pyrimido[1,2-a]benzimidazol-5(6H)-one ;
la 6-(2,6-diméthylphényl)-2-((4-((2-((1-méthyléthyl)amino)éthyl)oxy)-3-(méthyloxy)phényl)-amino)-pyrimido[5',4':5,6]pyrimido[1,2-a]benzimidazol-5(6H)-one ;
la 6-(2,6-diméthylphényl)-2-((4-((2-((1-méthyléthyl)amino)éthyl)oxy)phényl)amino)pyrimido[5',4' :5,6]-pyrimido[1,2-a]benzimidazol-5(6H)-one ;
la 6-(2,6-diméthylphényl)-2-((4-((2-(1-méthyl-4-pipéridinyl)éthyl)oxy)phényl)amino)pyrimido[5',4' :5,6]-pyrimido[1,2-a]benzimidazol-5(6H)-one ;
la 6- ( 2, 6-diméthylphényl)-2- ((4-((2-(1-pipéridinyl)éthyl)oxy)phényl)amino)pyrimido[5',4':5,6] -pyrimido[1,2-a]benzimidazol-5(6H)-one ;
la 6-(2,6-diméthylphényl)-2-((4-((2-(4-(1-méthyléthyl)-1-pipérazinyl)éthyl)oxy)-3-(méthyloxy)-phényl)-amino)pyrimido[5',4':5,6]pyrimido[1, 2-a]benzimidazol-5(6H)-one;
la 6-(2,6-diméthylphényl)-2-((4-((2-(4-méthyl-1-pipérazinyl)éthyl)oxy)phényl)amino)pyrimido[5',4 ':5,6]-pyrimido[1,2-a]benzimidazol-5(6H)-one;
la 6-(2,6-diméthylphényl)-2-((4-((2-(4-morpholinyl)éthyl)oxy)phényl)amino)pyrimido[5',4':5,6] -pyrimido[1,2-a]benzimidazol-5(6H)-one ;
la 6-(2,6-diméthylphényl)-2-((4-((2-(méthylamino)-éthyl)oxy)phényl)amino)pyrimido[5', 4':5,6]pyrimido-[1,2-a]benzimidazol-5(6H)-one;
la 6-(2,6-diméthylphényl)-2-((4-((3-(1-pipéridinyl)propyl)oxy)phényl)amino)pyrimido-[5',4': 5,6]pyrimido[1,2-a]benzimidazol-5(6H)-one ;
la 6-(2,6-diméthylphényl)-2-((4-((3-(4-(1-méthyléthyl)-1-pipérazinyl)propyl)oxy)-3-(méthyloxy)-phényl)-amino)pyrimido[5',4':5,6]pyrimido-[1,2-a]benzimidazol-5(6H)-one;
la 6-(2,6-diméthylphényl)-2-((4-(1-pipérazinyl)-phényl)amino)pyrimido[5',4':5,6]pyrimido[1,2-a]-benzimidazol-5(6H)-one ;
la 6-(2,6-diméthylphényl)-2-((4-(4-((3-(méthyloxy)-phényl)méthyl)-1-pipérazinyl)phényl)amino)-pyrimido[5',4':5,6]pyrimido[1,2-a]benzimidazol-5(6H)-one ;
la 6-(2,6-diméthylphényl)-2-((4-(4-(1-méthyléthyl)-1-pipérazinyl)phényl)amino)pyrimido-[5',4':5,6]-pyrimido[1,2-a]benzimidazol-5(6H)-one ;
la 6-(2,6-diméthylphényl)-2-((4-(4-(1-méthyléthyl)-1-pipérazinyl)-3-(méthyloxy)phényl)-amino)-pyrimido[5',4':5,6]pyrimido[1,2-a]benzimidazol-5(6H)-one;
la 6-(2,6-diméthylphényl)-2-((4-(4-(2-(méthyloxy)-éthyl)-1-pipérazinyl)phényl)amino)pyrimido[5', 4':5,6]-pyrimido[1,2-a]benzimidazol-5(6H)-one;
la 6-(2,6-diméthylphényl)-2-((4-(4-(2-hydroxyéthyl)-1-pipérazinyl)phényl)amino)pyrimido[5', 4':5,6]-pyrimido[1,2-a]benzimidazol-5(6H)-one;
la 6-(2,6-diméthylphényl)-2-((4-(4-méthyl-1,4-diazépan-1-yl)phényl)amino)pyrimido[5',4': 5,6]-pyrimido-[1,2-a]benzimidazol-5(6H)-one;
la 6-(2,6-diméthylphényl)-2-((4-(4-méthyl-1-pipérazinyl)phényl)amino)pyrimido[5',4';5,6]pyri mido-[1,2-a]benzimidazol-5(6H)-one;
la 6-(2,6-diméthylphényl)-2-((4-(4-méthyl-1-pipérazinyl)phényl)amino)-8,9-dihydroimidazo[1,2-a]-pyrimido[5,4-e]pyrimidin-5(6H)-one;
la 6-(2,6-diméthylphényl)-2-((4-(4-méthyl-1-pipérazinyl)phényl)amino)imidazo[1,2-a]pyrimido[ 5,4-e]-pyrimidin-5(6H)-one ;
la 6-(2-fluorophényl)-2-((4-(4-méthyl-1-pipérazinyl)phényl)amino)pyrimido[5',4':5,6]pyrimido-[1,2-a]benzimidazol-5(6H)-one ;
la 6-(3-(méthyloxy)phenyl)-2-((4-(4-méthyl-1-pipérazinyl)phényl)amino)pyrimido[5',4':5,6]pyrimido - [1,2-a]benzimidazol-5(6H)-one;
la 6-(3-fluorophényl)-2-((4-(4-méthyl-1-pipérazinyl)phényl)amino)pyrimido[5',4':5,6]pyrimido-[1,2-a]benzimidazol-5(6H)-one ;
la 6-(3-méthylphényl)-2-((4-(4-méthyl-1-pipérazinyl)phényl)amino)pyrimido[5',4':5,6]pyrimido-[1,2-a]benzimidazol-5(6H)-one;
la 6-(4-(méthyloxy)phényl)-2-((4-(4-méthyl-1-pipérazinyl)phényl)amino)pyrimido[5',4':5,6]pyri mido-[1,2-a]benzimidazol-5(6H)-one ;
la 6-(4-fluorophényl)-2-((4-(4-méthyl-1-pipérazinyl)phényl)amino)pyrimido[5',4':5,6]pyrimido-[1,2-a]benzimidazol-5(6H)-one ;
la 6-(4-méthylphényl)-2-((4-(4-méthyl-1-pipérazinyl)phényl)amino)pyrimido[5',4':5,6]pyrimido-[1,2-a]benzimidazol-5(6H)-one ;
la 6-(5-chloro-2-(méthyloxy)phényl)-2-((4-(4-méthyl-1-pipérazinyl)phényl)amino)pyrimido[5',4': 5,6]-pyrimido[1,2-a]benzimidazol-5(6H)-one ;
le N-(2-(diéthylamino)éthyl)-3-((6-(2,6-diméthylphényl)-5-oxo-5,6-dihydropyrimido[5',4':5,6] pyrimido-[1,2-a]benzimidazol-2-yl)amino)-N-méthylbenzamide ;
le N-(2-(diéthylamino)éthyl)-4-((6-(2,6-diméthylphényl)-5-oxo-5,6-dihydropyrimido[5',4':5,6]-pyrimido-[1,2-a]benzimidazol-2-yl)amino)-N-méthylbenzamide ;
le N-(2-(diméthylamino)éthyl)-3-((6-(2,6-diméthylphényl)-5-oxo-5,6-dihydropyrimido[5',4':5,6]-pyrimido-[1,2-a]benzimidazol-2-yl)amino)benzamide ; et
le N-(2-(diméthylamino)éthyl)-4-((6-(2,6-diméthylphényl)-5-oxo-5,6-dihydropyrimido[5',4':5,6]-pyrimido-[1,2-a]benzimidazol-2-yl)amino)benzamide.

12. Méthode de fabrication d'un composé selon la revendication 1, la méthode comprenant les étapes consistant à :
faire réagir un ester de pyrimidine (A) 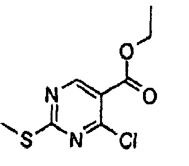 avec un benzimidazole (B) 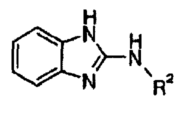 en présence d'une base pour former une pyrimidine tétracyclique (C) 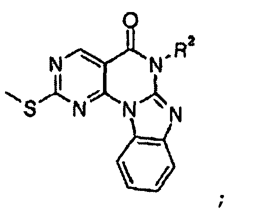 oxyder le soufre de la pyrimidine tétracyclique (C) pour former une sulfone de pyrimidine tétracyclique (D) et 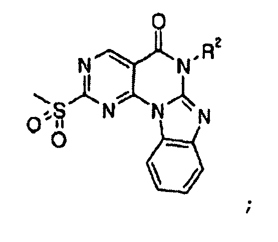 faire réagir la sulfone de pyrimidine tétracyclique (D) avec R¹NH pour fabriquer un composé 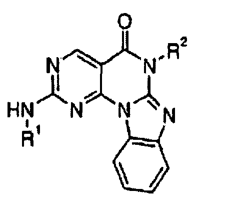 selon la revendication 1.

13. Composition pharmaceutique comprenant un composé selon l'une quelconque des revendications 1 à 11 et un support pharmaceutiquement acceptable.

14. Utilisation d'un composé selon l'une quelconque des revendications 1 à 11 pour la préparation d'un médicament destiné au traitement de l'inflammation.

15. Utilisation d'un composé selon l'une quelconque des revendications 1 à 11 pour la préparation d'un médicament destiné à l'inhibition de l'activation et de la prolifération des cellules T chez un mammifère.

16. Utilisation d'un composé selon l'une quelconque des revendications 1 à 11 pour la préparation d'un médicament destiné au traitement de l'arthrite, la polyarthrite rhumatoïde, l'arthrite psoriasique ou l'arthrose chez un mammifère.

17. Utilisation d'un composé selon l'une quelconque des revendications 1 à 11 pour la préparation d'un médicament destiné au traitement d'un rejet de transplantation, de transplantation aiguë ou d'hétérogreffe ou d'homogreffe d'organe ou à l'induction d'une tolérance de transplantation chez un mammifère.

18. Utilisation d'un composé selon l'une quelconque des revendications 1 à 11 pour la préparation d'un médicament destiné au traitement d'une lésion ischémique ou de reperfusion, un infarctus du myocarde ou un accident vasculaire cérébral chez un mammifère.

19. Utilisation d'un composé selon l'une quelconque des revendications 1 à 11 pour la préparation d'un médicament destiné au traitement de la sclérose en plaques, une maladie inflammatoire de l'intestin, y compris la rectocolite hémorragique, la maladie de Crohn, le lupus, l'hypersensibilité de contact, l'hypersensibilité de type retardée, l'entéropathie sensible au gluten, le diabète de type I, le psoriasis, la dermatite de contact, la thyroïdite de Hashimoto, le syndrome de Sjögren, l'hyperthyroïdie auto-immune, la maladie d'Addison, la maladie polyglandulaire auto-immune, l'alopécie auto-immune, l'anémie pernicieuse, le vitiligo, l'hypopituitarisme auto-immun, le syndrome de Guillain-Barré, la glomérulonéphrite, la maladie du sérum, l'urticaire, des maladies allergiques, l'asthme, le rhume des foins, la rhinite allergique, la mycosis fongoide, la dermatomyosite, la pelade, la dermatite actinique chronique, l'eczéma, la maladie de Behcet, la pustulose palmoplantaire, la pyodermie gangréneuse, le syndrome de Sézary, la dermatite atopique, la sclérose systémique, la morphée ou la dermatite atopique chez un mammifère.

20. Utilisation d'un composé selon l'une quelconque des revendications 1 à 11 pour la préparation d'un médicament destiné au traitement du carcinome du côlon ou du thymome chez un mammifère.
